# EUROPEAN PATENT APPLICATION

(11) **EP 4 843 984 A1**
(43) Date of publication of application: **25.11.2026**
(21) Application number: 25741530.7
(22) Date of filing: 15.01.2025
(51) Int. Cl.: C07D 403/12, C07C 237/04, A61K 31/7088, A61K 31/713, A61K 31/165, A61P 35/00

(54) **TARGETING COMPOUND AND USE THEREOF**

(30) Priority: 15.01.2024 CN 202410057328; 29.08.2024 CN 202411207330
(71) Applicant: Hubei Bio-Pharmaceutical Industrial Technological Institute Inc., Wuhan, Hubei 430075 (CN)
(72) Inventor: ZHANG, Xuejun, Wuhan, Hubei 430075 (CN); JIA, Yimin, Wuhan, Hubei 430075 (CN); JIANG, Wen, Wuhan, Hubei 430075 (CN); YANG, Yang, Wuhan, Hubei 430075 (CN); ZHAO, Xin, Wuhan, Hubei 430075 (CN); LI, Cong, Wuhan, Hubei 430075 (CN); KE, Chaoqi, Wuhan, Hubei 430075 (CN); YE, Dabing, Wuhan, Hubei 430075 (CN)
(74) Representative: HGF
(86) International application number: PCT/CN2025/072524
(87) International publication number: WO 2025/152969

(57) **Abstract**

Provided is a targeting compound capable of specifically binding to integrin αvβ6. The compound is as shown in formula (I). The targeting compound is useful in transferring and delivering active molecules to a cell or tissue expressing integrin αvβ6.

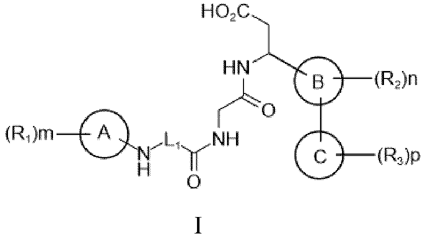

## Description

### FIELD

The present disclosure belongs to the field of medicine. Particularly, the present disclosure relates to a targeting compound and use thereof.

### BACKGROUND

Integrin αvβ6 is a heterodimer composed of αv and β6 integrin subunits, each of which consists of a large extracellular domain, a flexible leg region, a transmembrane segment, and a short cytoplasmic tail. Integrin αvβ6 serves as a receptor for latency-associated peptide (LAP) of transforming growth factor-β (TGF-β) and extracellular matrix (ECM) proteins including fibronectin, vitronectin, and tenascin. Integrin αvβ6 is frequently upregulated in diseases such as cancer and fibrosis, with an estimated one-third of all solid cancers showing upregulation, and its expression is associated with disease progression and poor prognosis. Multiple studies have demonstrated that the severity of many cancers, including lung cancer, breast cancer, colon cancer, gastric cancer, and ovarian cancer, is correlated with the expression of αvβ6. Integrin αvβ6 is an attractive tumor marker and potential therapeutic target, especially given its role in the expression of matrix metalloproteinases (MMPs) and the activation of TGF-β1.

Compounds targeting integrin αvβ6 are suitable for conjugation with active molecules in order to transport and deliver the active molecules in vivo to cells expressing integrin αvβ6. For particular transported molecules, such as therapeutic oligonucleotide-based compounds (e.g., antisense oligonucleotides or RNAi agents), targeting ligands targeting integrin αvβ6 may be conjugated to the oligonucleotide-based compound to deliver the therapeutic agents to cells and/or tissues expressing integrin αvβ6, and to facilitate entry of the therapeutic agents into cells through receptor-mediated endocytosis, pinocytosis, or other mechanisms. Accordingly, there exists a need in the art to develop targeting ligands that target integrin αvβ6.

The receptor for advanced glycation end products (RAGE) is a novel pattern recognition receptor, which is widely involved in the pathological processes of many diseases, such as Alzheimer's disease, pneumonia, tumors, and diabetes. In particular, this receptor has been confirmed in preclinical animal models to be associated with both type II and non-type II inflammatory responses. Currently, ARO-RAGE, a small nucleic acid compound targeting RAGE, is in phase I clinical study for the treatment of inflammatory lung diseases such as asthma and chronic obstructive pulmonary disease. According to genomic analysis, this receptor is highly specifically expressed on the surface of alveolar cells and is less expressed in other tissues and organs. Therefore, targeting ligands that target integrin αvβ6 and compounds capable of silencing RAGE protein expression are suitable for evaluating the delivery capability of the targeting ligands.

### SUMMARY

An object of the present disclosure is to provide a targeting compound, which is a targeting ligand capable of targeting integrin αvβ6. The targeting compound has affinity for integrin αvβ6 and can specifically bind to integrin αvβ6. The targeting compound can be conjugated to an active molecule to promote transport and delivery of the active molecule (such as an antisense oligonucleotide) to cells or tissues expressing integrin αvβ6, such that the active molecule exerts its function in these cells or tissues.

The targeting compound of the present disclosure is a compound represented by Formula I, or a tautomer or stereoisomer thereof. The targeting compound includes a linker, via which the active molecule is further linked. A scaffold may further be present between the targeting compound and the active molecule, and the scaffold has a monodentate, bidentate, tridentate, or tetradentate structure. The active molecule is linked to one, two, three or four targeting compounds via the scaffold.

In a first aspect of the present disclosure, there is provided a compound represented by Formula I, or a tautomer or stereoisomer thereof: 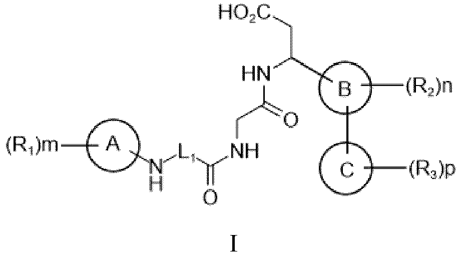 wherein:
ring A is 5-membered heteroaromatic ring;
ring B is 6- to 10-membered aromatic ring or 5- to 6-membered heteroaromatic ring;
ring C is 6- to 10-membered aromatic ring, 5- to 10-membered heteroaromatic ring, or 5-to 10-membered heterocycloalkyl;
L₁ is C₁ to C₆ alkylene or halogenated C₁ to C₆ alkylene;
R₁ is selected from H, halogen, -NH₂, -CN, -OH, -SF₅, -COOH, C₁₋₆ alkyl, halogenated C₁₋₆ alkyl, C₁₋₆ alkoxy, -CONH₂, -CONR₁₁R₁₂, -C(O)-C₁₋₆ alkyl, -S(O)₂-C₁₋₆ alkyl, -COOR₁₃, - NH-C₁₋₆ alkyl, and -NR₁₄R₁₅;
R₂ and R₃ are each independently selected from H, halogen, -NH₂, -CN, -OH, -SF₅, - COOH, C₁₋₆ alkyl, C₁₋₆ alkoxy, -CONH₂, -CONR₁₁R₁₂, -C(O)-C₁₋₆ alkyl, -S(O)₂-C₁₋₆ alkyl, - COOR₁₃, -NH-C₁₋₆ alkyl, and -NR₁₄R₁₅,
   wherein R₁₁, R₁₂, R₁₃, R₁₄, and R₁₅ are each independently C₁₋₆ alkyl;
R₁, R₂, and R₃ are optionally substituted with one, two, or three identical or different substituents selected from halogen, -NH₂, -CN, -OH, -SF₅, -COOH, C₁₋₆ alkyl, and C₁₋₆ alkoxy;
ring B or ring C is optionally attached to a linker T, wherein the linker T is used for direct or indirect attachment to an active molecule;
when a plurality of R₁ substituents are present, the plurality of R₁ substituents are identical or different;
when a plurality of R₂ substituents are present, the plurality of R₂ substituents are identical or different;
when a plurality of substituents R₃ substituents are present, the plurality of R₃ substituents are identical or different;
m is 1, 2, or 3;
n is 1 or 2; and
p is 1 or 2.

It should be noted that the above "R₁, R₂, and R₃ are optionally substituted with substituents" means that hydrogen atoms in hydrogen-containing groups (e.g., C₁₋₆ alkyl) present in R₁, R₂, and R₃ are optionally substituted with substituents.

In an optional embodiment of the present disclosure, ring B is a 6- to 10-membered aromatic ring.

In an optional embodiment of the present disclosure, ring B is 6- to 8-membered aromatic ring.

In a preferred embodiment, the structural fragment 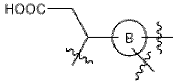 has a structure of

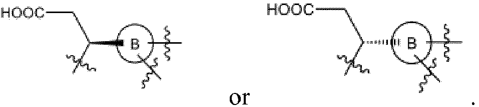

In a preferred embodiment, the group 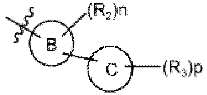 is 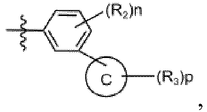 where R₂, R₃, ring C, n, and p are as defined in the first aspect.

In a preferred embodiment, R₂ is H.

In a preferred embodiment, ring C is 5- to 10-membered heteroaromatic ring.

In a preferred embodiment, ring C is 5- to 8-membered heteroaromatic ring.

In a preferred embodiment, ring C is 5- or 6-membered heteroaromatic ring, wherein heteroatom is selected from N, O, and S, and wherein a plurality of heteroatoms are present, the plurality of heteroatoms are identical or different.

In a preferred embodiment, ring C is 5-membered N-containing heteroaromatic ring.

In a preferred embodiment, ring C is selected from furan, thiophene, pyrrole, thiazole, imidazole, pyrazole, oxazole, triazole, tetrazole, pyridine, pyridazine, pyrimidine, and pyrazine.

In a preferred embodiment, ring C is pyrazole.

In a preferred embodiment, ring C is

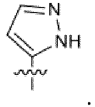

In a preferred embodiment, R₃ is halogen, -NH₂, -CN, -OH, C₁₋₆ alkyl, C₁₋₆ alkoxy, or halogenated C₁₋₆ alkyl.

In a preferred embodiment, R₃ is methyl.

In a preferred embodiment, L₁ is C₁ to C₆ alkylene.

In a preferred embodiment, L₁ is -CH₂CH₂CH₂-.

In a preferred embodiment, the compound represented by Formula I has a structure of: 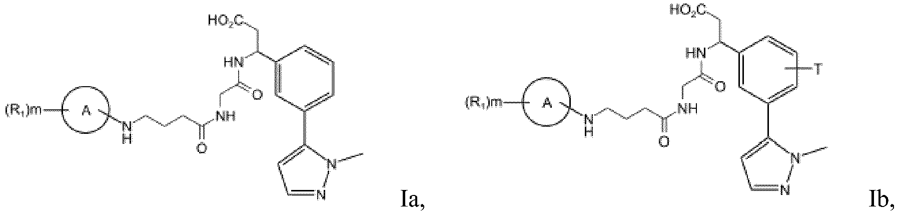 or 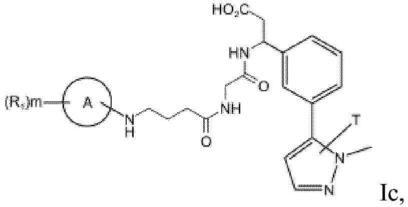 where ring A, R₁, and m are as defined in the first aspect.

In a preferred embodiment, the compound represented by Formula I, when attached to the linker T, has a structure of: 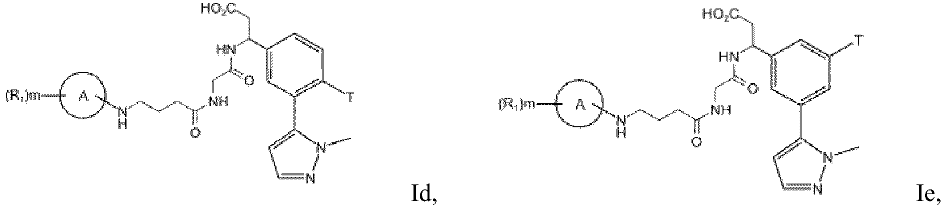 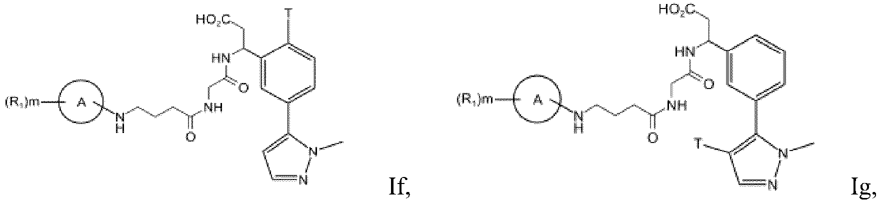 or 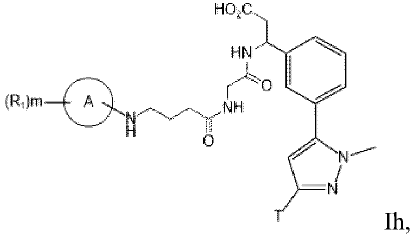 where ring A, R₁ and m are as defined in the first aspect.

In a preferred embodiment, ring A is 5-membered heteroaryl; R₁ is as defined in the first aspect; and m is 0, 1, or 2.

In a preferred embodiment, the structural fragment 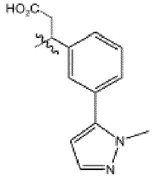 is

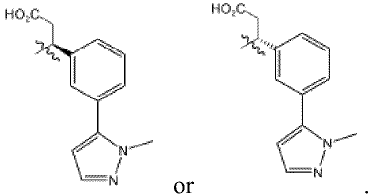

In a preferred embodiment, ring A is 5-membered heteroaromatic ring, wherein heteroatom is selected from N, O, and S, and wherein when a plurality of heteroatoms are present, the plurality of heteroatoms are identical or different.

In a preferred embodiment, ring A is 5-membered N-containing heteroaromatic ring.

In a preferred embodiment, ring A is selected from furan, thiophene, pyrrole, thiazole, imidazole, pyrazole, oxazole, triazole, and tetrazole.

In a preferred embodiment, ring A is imidazole or triazole.

In a preferred embodiment, ring A is

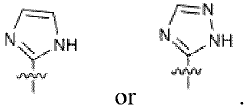

In a preferred embodiment, 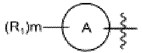 is 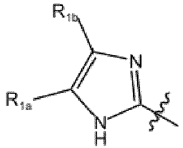 or 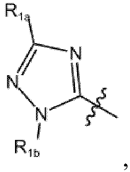 where R₁ₐ and R_{1b} are each independently R₁.

In a preferred embodiment, R₁ is halogen, -NH₂, -CN, -OH, -SF₅, -COOH, C₁₋₆ alkyl, -CONH₂, -C(O)-C₁₋₆ alkyl, or -S(O)₂-C₁₋₆ alkyl, wherein the C₁₋₆ alkyl, the -C(O)-C₁₋₆ alkyl, or the -S(O)₂-C₁₋₆ alkyl are optionally substituted with one, two, or three identical or different substituents selected from halogen, -NH₂, -CN, -OH, -SF₅, C₁₋₃ alkyl, and C₁₋₃ alkoxy.

In a preferred embodiment, R₁ is H, -F, -Cl, -CN, -SF₅, -COOH, -CF₃, -CHF₂, - COCH₃, -CONH₂, or -S(O)₂CH₃.

In a preferred embodiment, R₁ is H, -F, -Cl, or -CN.

In a preferred embodiment, 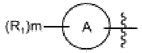 is selected from

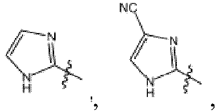

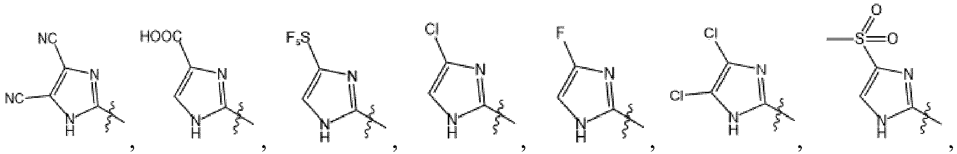

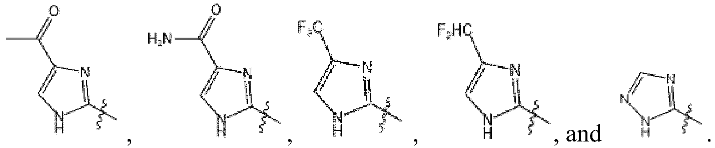

In a preferred embodiment, the compound represented by Formula I has a linker T.

In a preferred embodiment, the linker T includes a polyethylene glycol unit.

In a preferred embodiment, the linker T includes 2 to 20 polyethylene glycol units.

In a preferred embodiment, the linker T is 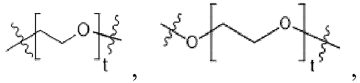 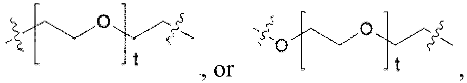 where t ranges from 1 to 10.

In a preferred embodiment, the linker T is 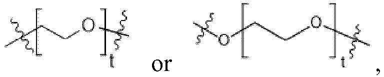 where t ranges from 1 to 10.

In a preferred embodiment, the linker T is 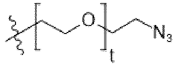 or 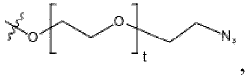 where t ranges from 1 to 10.

In a preferred embodiment, t as defined in any one of the above is 1, 2, 3, 4, 5, or 6.

In a preferred embodiment, the linker T is 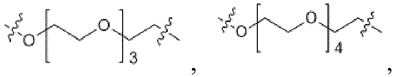 or

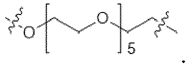

In a preferred embodiment, the linker T is

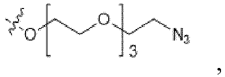

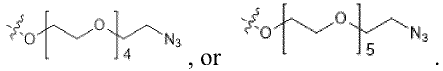

In a preferred embodiment, the compound represented by Formula I has a structure selected from one or more of: 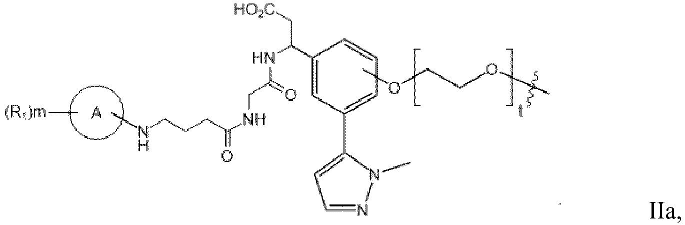 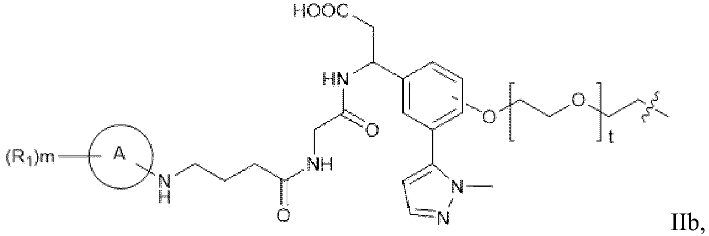 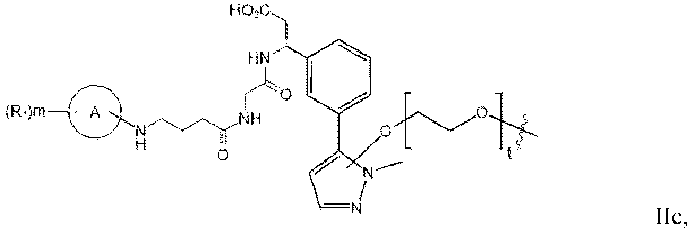 or

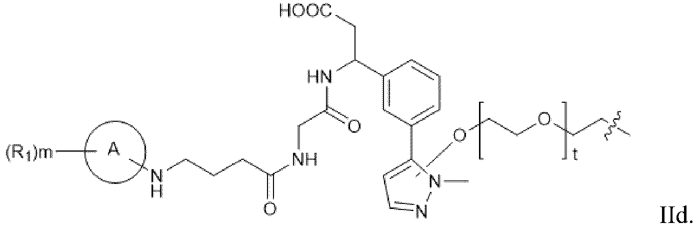

In a preferred embodiment, the compound represented by Formula I has a structure selected from one or more of: 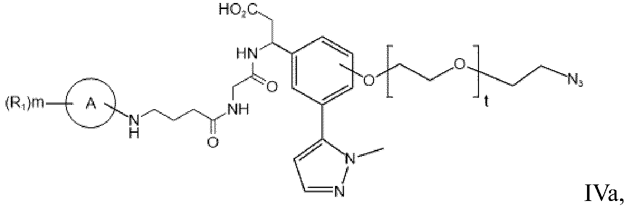 or

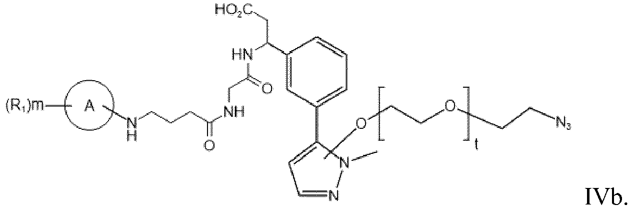

In a preferred embodiment, t ranges from 1 to 10.

In a preferred embodiment, t is 1, 2, 3, 4, 5, or 6.

In a preferred embodiment, the compound has a structure selected from one or more of: 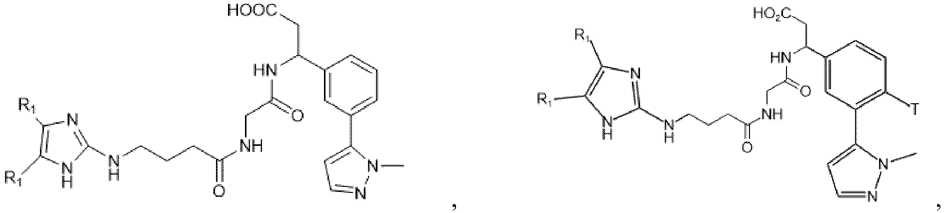 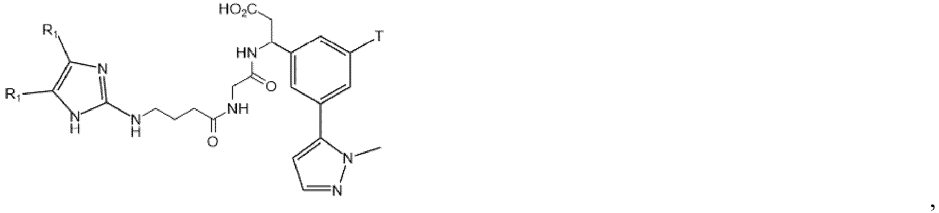 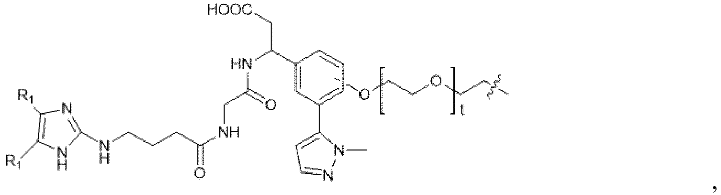 or 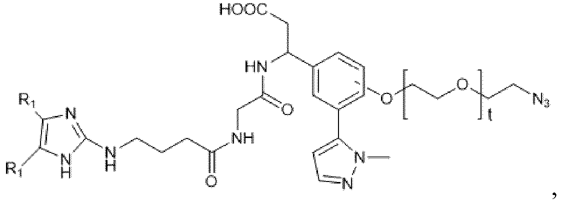 where 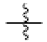 represents an attachment point, and each R₁ is independently as defined in the first aspect.

In a preferred embodiment, each R₁ is independently H, -F, -Cl, or -CN.

In a preferred embodiment, the compound represented by Formula I, or the tautomer or stereoisomer thereof has a structure of:

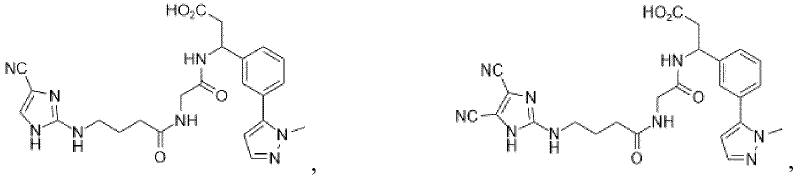

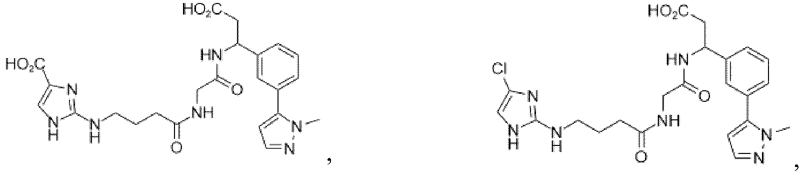

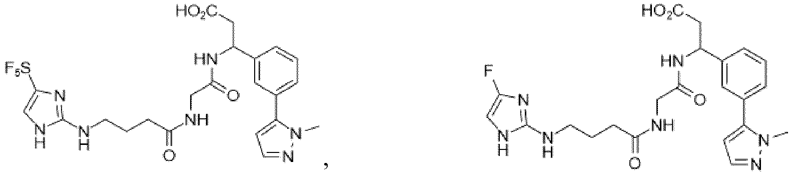

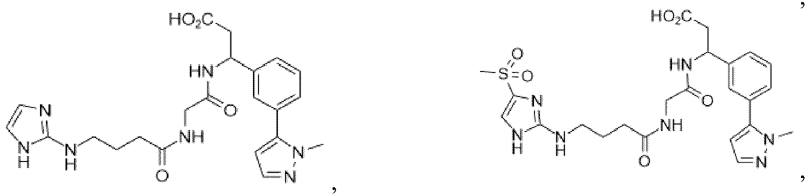

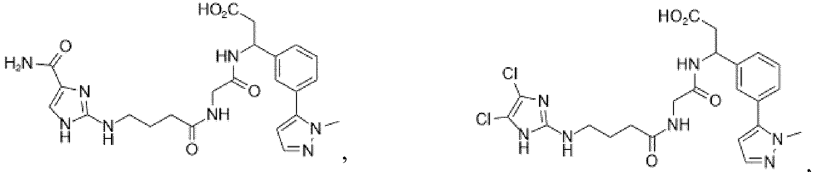

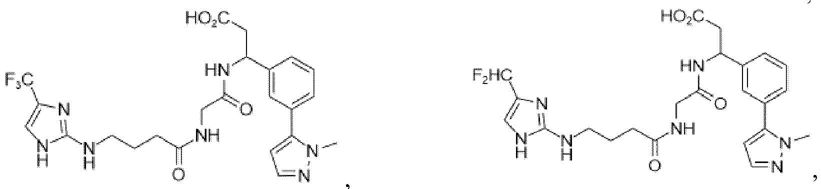

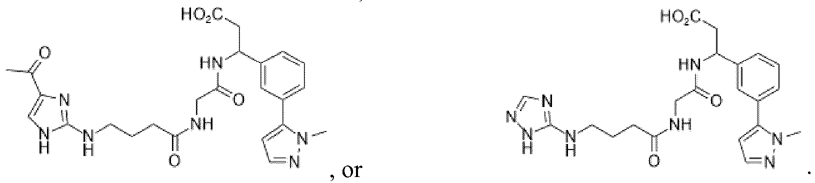

In a preferred embodiment, the compound represented by Formula I, or the tautomer or stereoisomer thereof has a structure of:

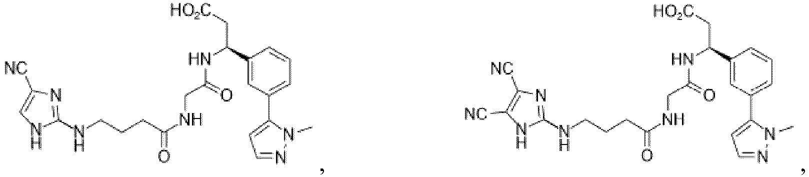

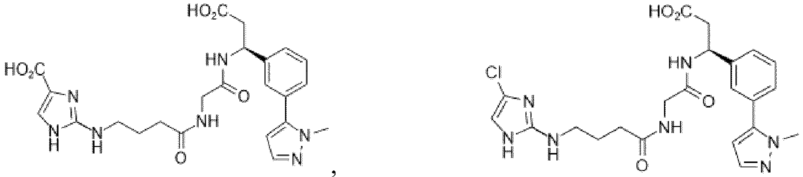

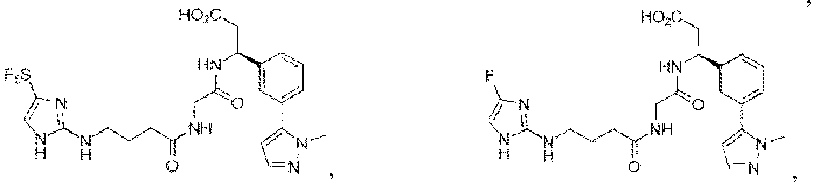

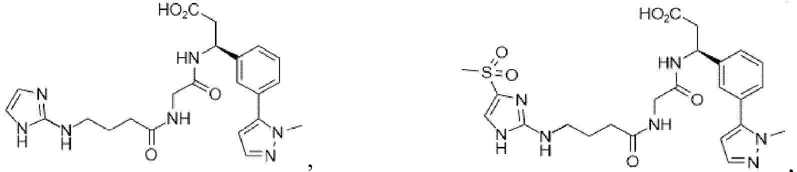

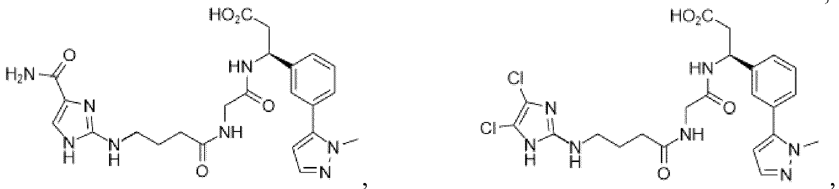

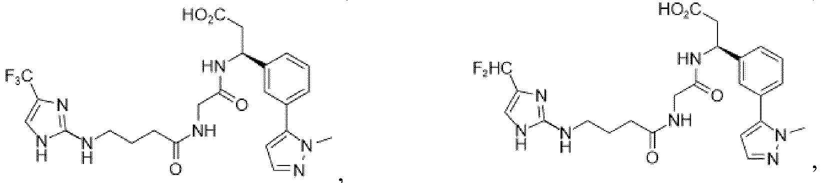

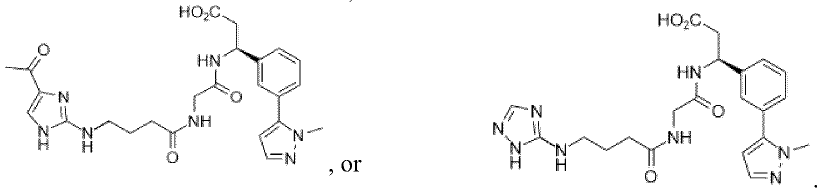

In a preferred embodiment, the compound represented by Formula I (attached to the linker T) has a structure selected from one or more of: 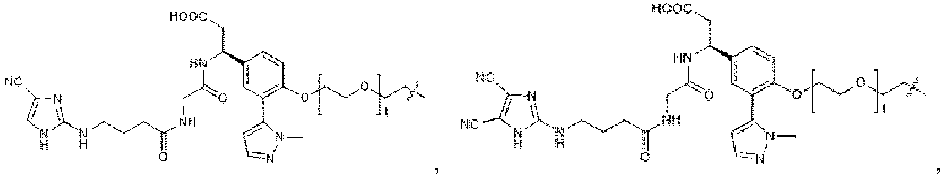 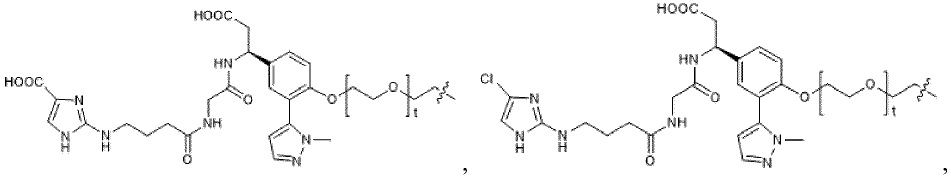 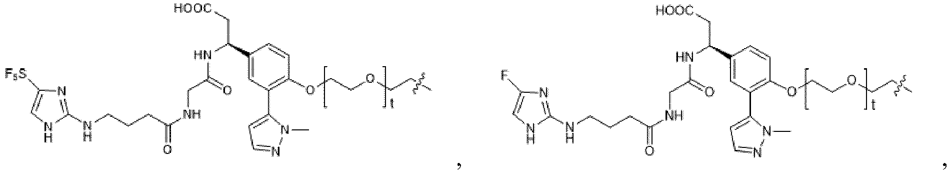 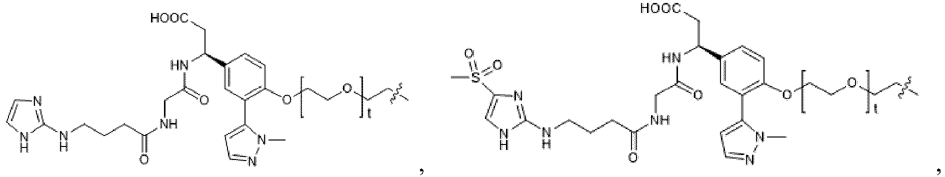 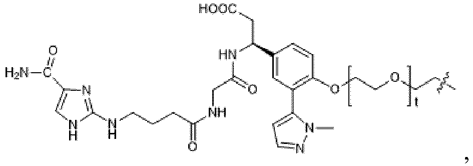 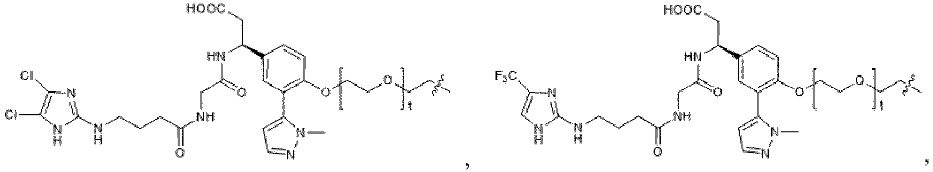 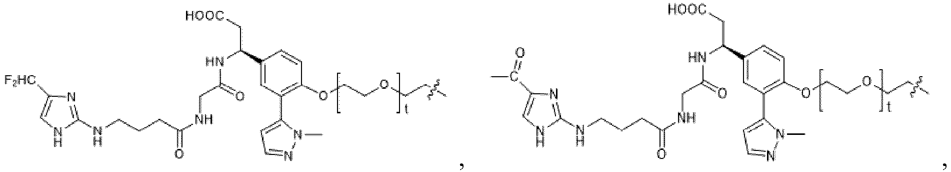 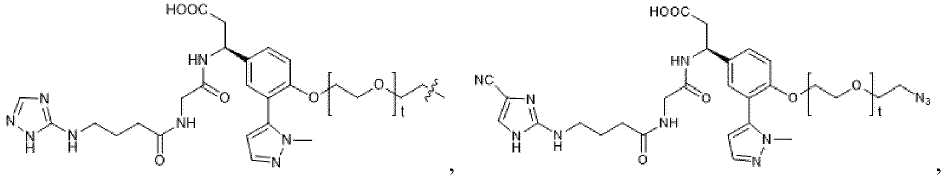 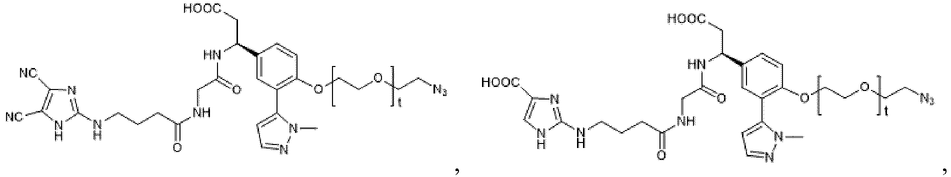 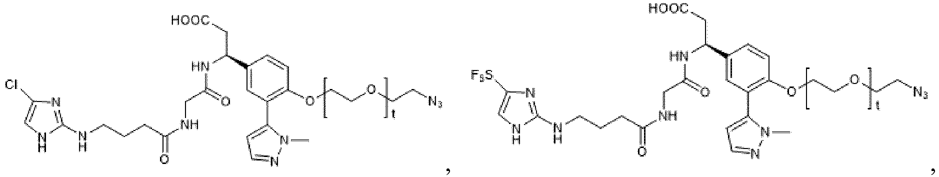 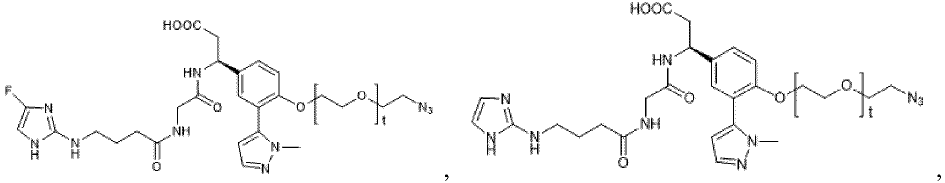 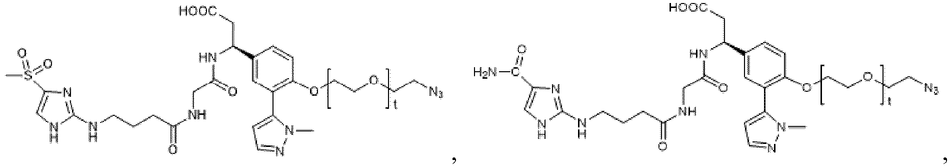 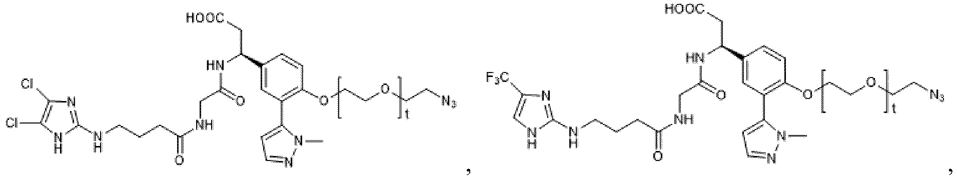 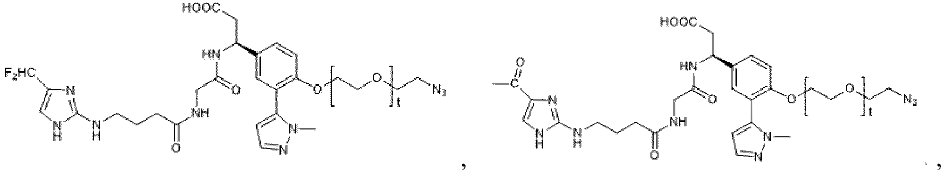 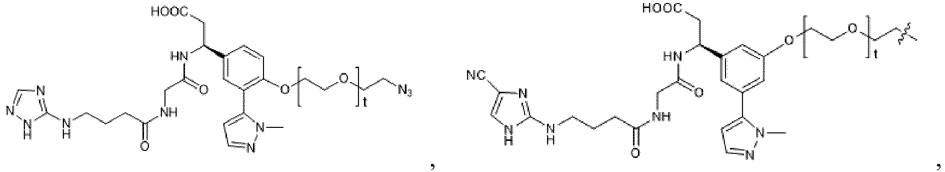 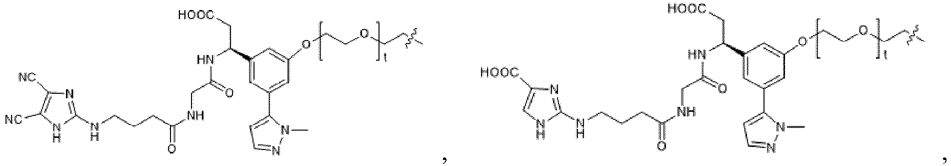 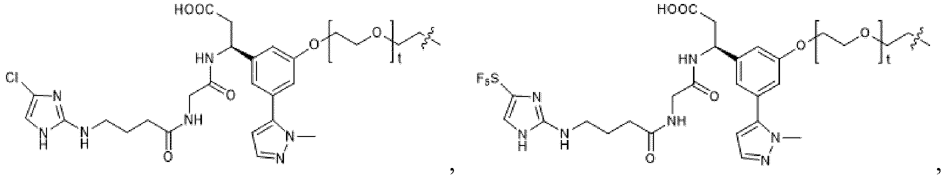 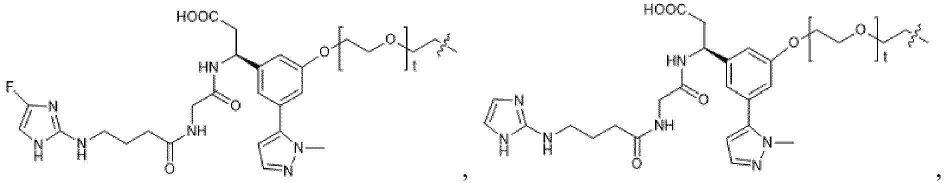 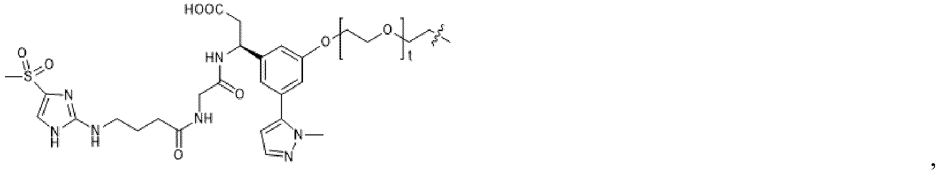 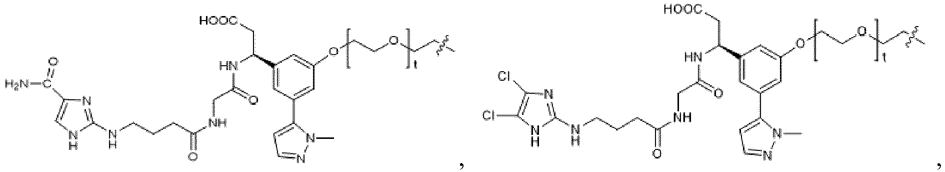 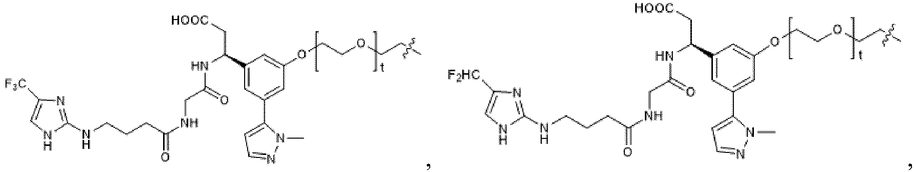 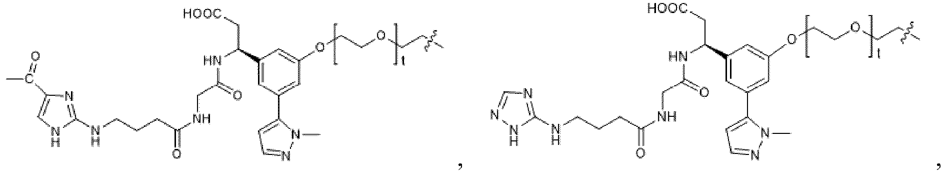 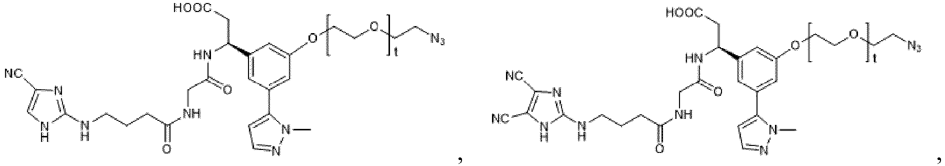 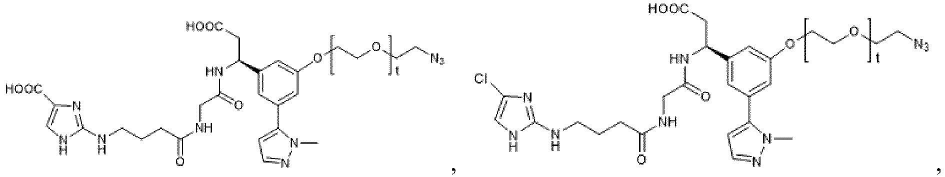 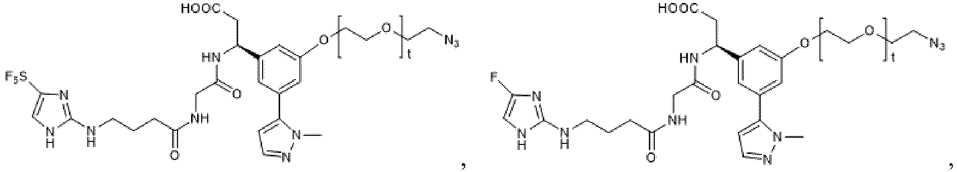 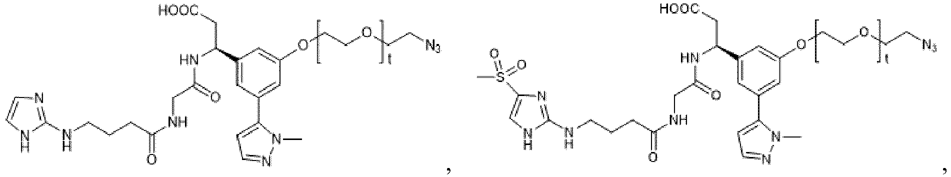 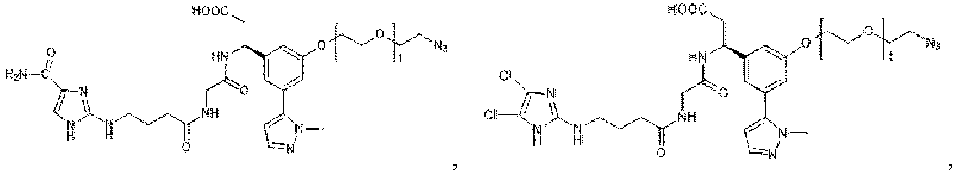 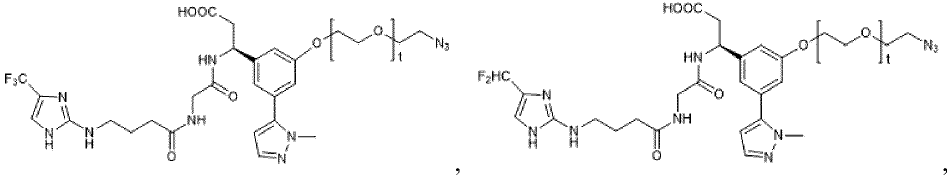 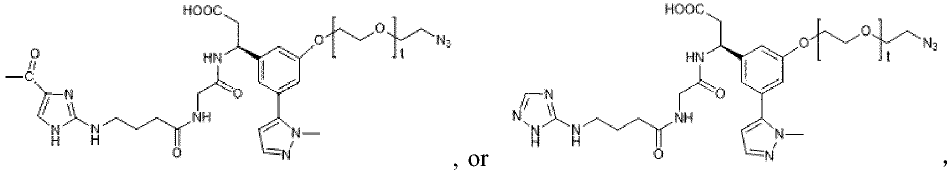 where t is 3, 4, or 5; and 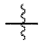 represents an attachment point.

In a preferred embodiment, the compound represented by Formula I (attached to the linker T) has a structure selected from one or more of: 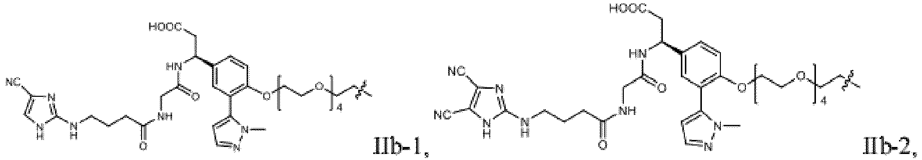 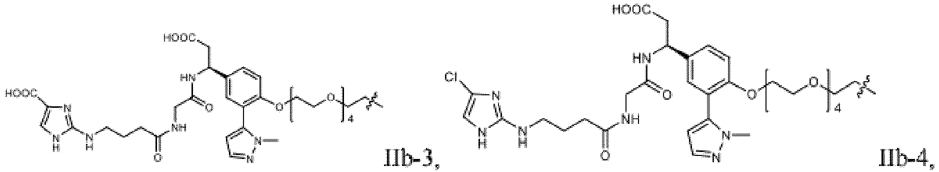 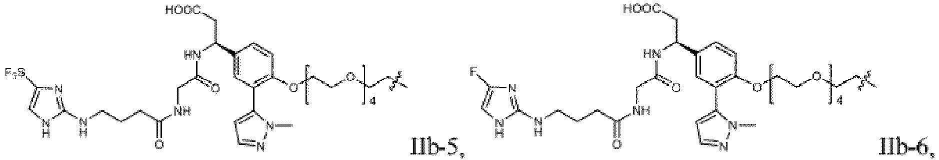 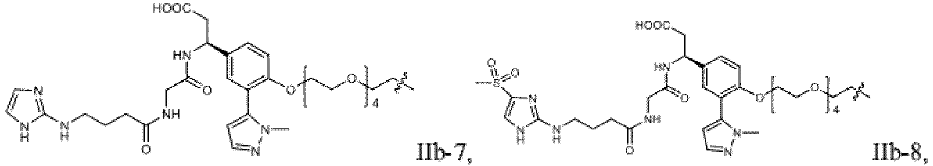 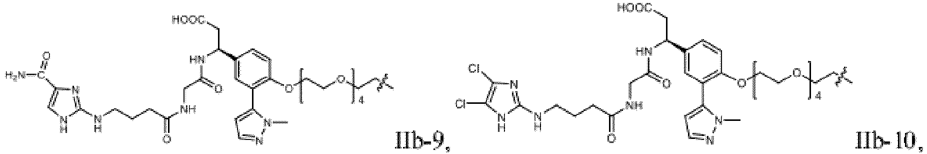 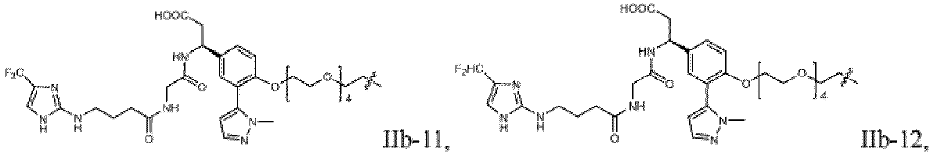 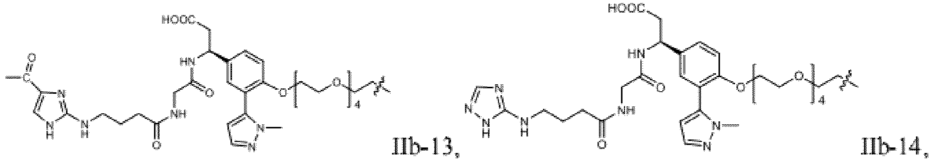 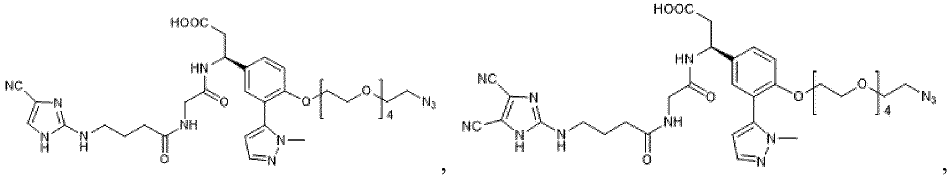 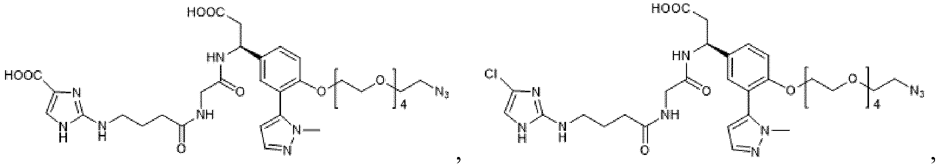 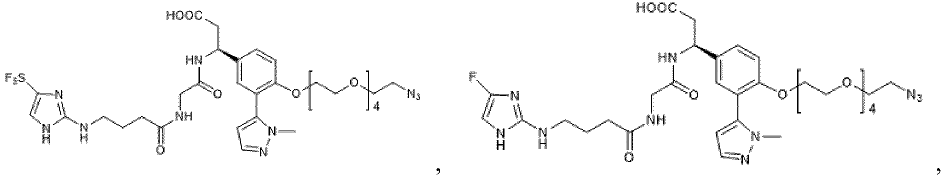 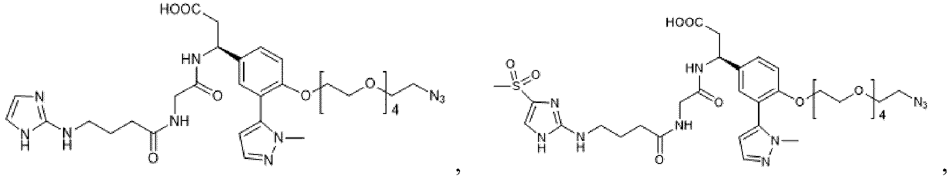 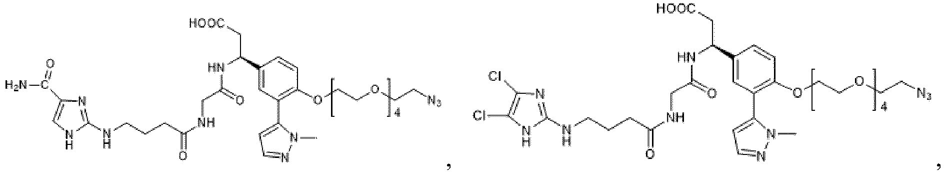 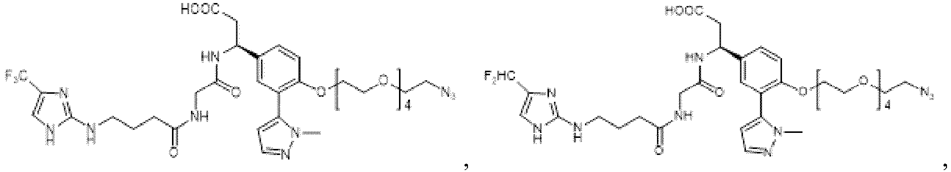 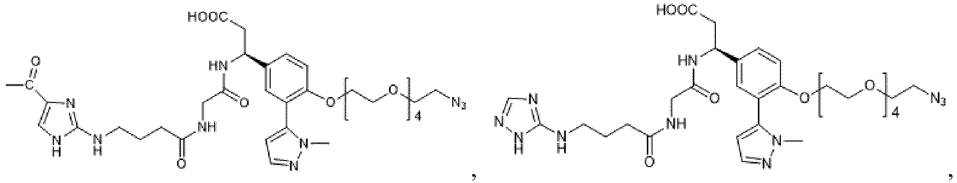 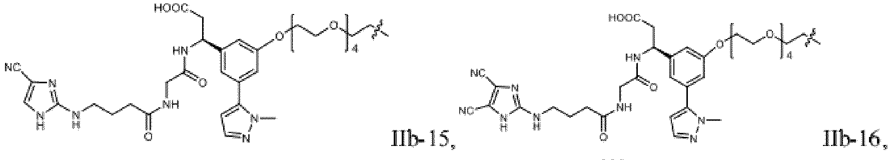 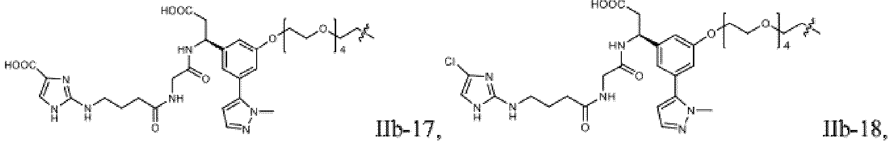 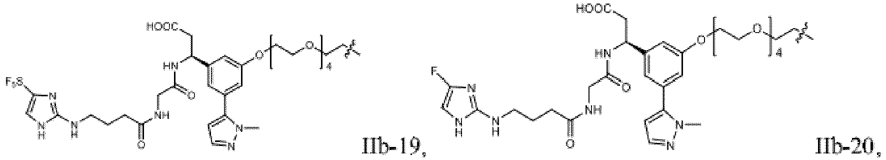 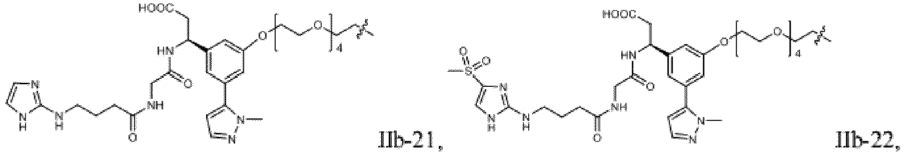 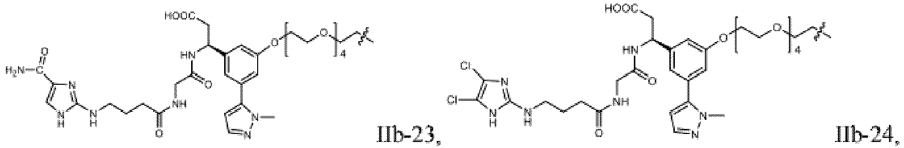 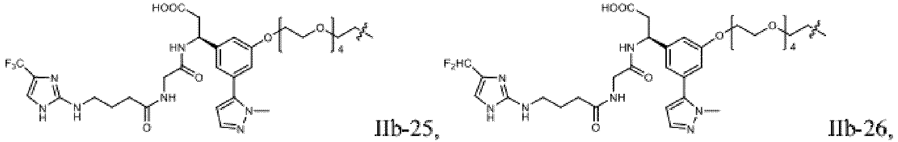 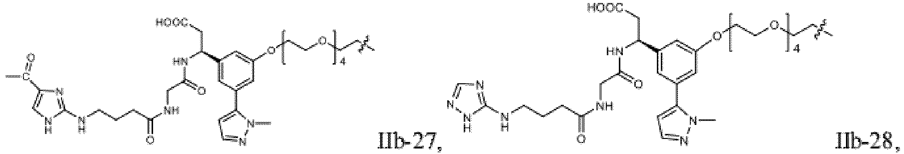 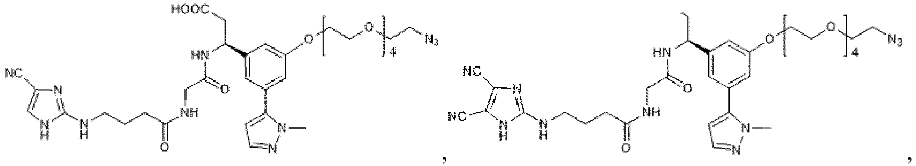 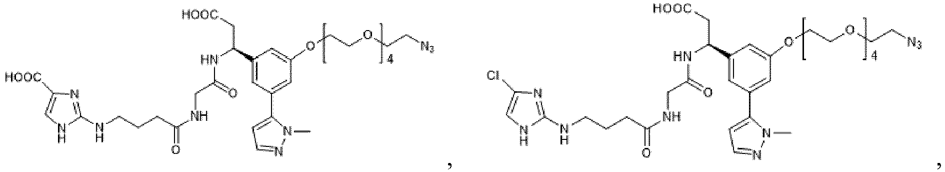 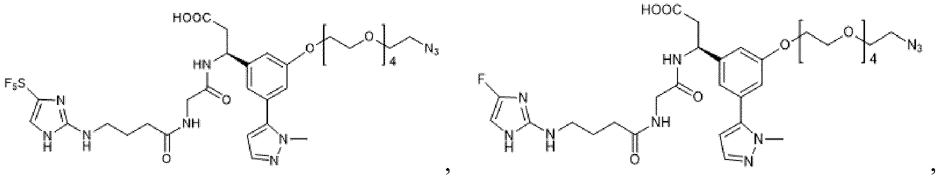 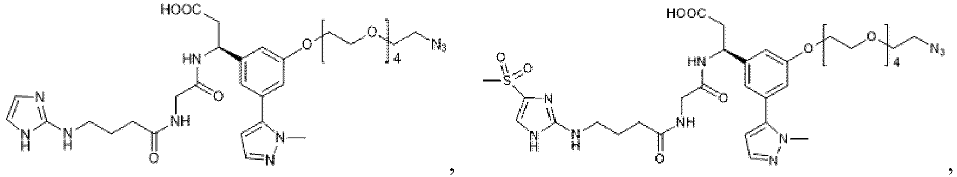 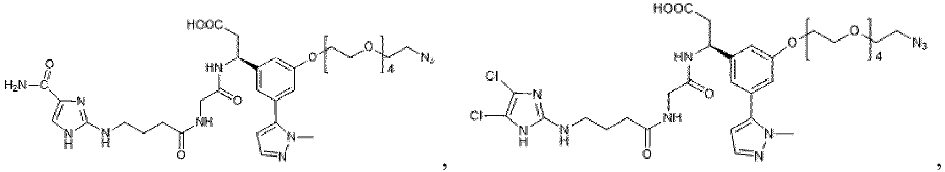 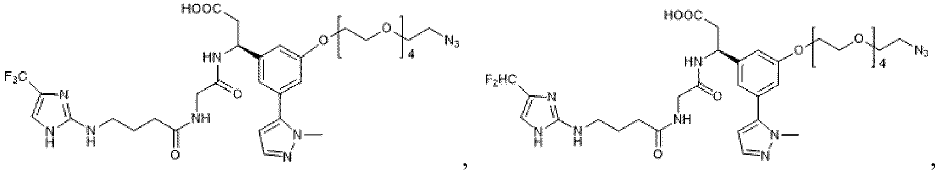 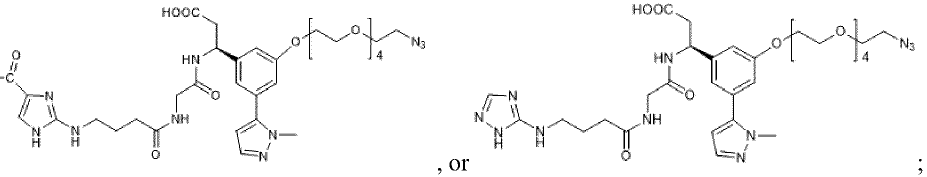 where 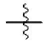 represents an attachment point.

In a second aspect of the present disclosure, there is provided a conjugate. The conjugate includes the compound represented by Formula I, or the tautomer or stereoisomer according to the first aspect, and an active molecule G. The compound represented by Formula I includes a linker T, and the active molecule G is attached to the linker T of the compound represented by Formula I.

It should be noted that when the compound represented by Formula I is linked to other molecules (e.g., in the conjugate according to the second aspect), the compound represented by Formula I shall refer to the compound represented by Formula I including the linker T. When the linker T of the compound represented by Formula I contains N₃, the compound represented by Formula I is a precursor for the preparation of the conjugate. When the linker T of the compound represented by Formula I does not contain N₃ but contains 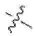 , 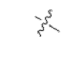 in the compound represented by Formula I is linked to the active molecule G.

In a preferred embodiment, the conjugate further includes a scaffold. The active molecule G is attached to the linker T of the compound represented by Formula I via the scaffold.

As used herein, the term "scaffold" refers to a structure that links one molecule or a portion of a molecule to another molecule or another portion of a molecule. For example, in the present disclosure, the active molecule G may be conjugated to one or more compounds represented by Formula I via the scaffold. The scaffold includes at least one attachment point for each ligand (the compound represented by Formula I, or the tautomer or stereoisomer thereof) and at least one attachment point for each active molecule G.

In a preferred embodiment, the conjugate has one or more characteristics selected from:
aa) the active molecule G is an active pharmaceutical ingredient or a prodrug thereof;
bb) the active molecule G is a small molecule, an antibody, an antibody fragment, an immunoglobulin, a monoclonal antibody, a label or marker, a lipid, a natural or modified nucleic acid, a natural or modified oligonucleotide, a natural or modified polynucleotide, a peptide, a nucleic acid aptamer, a polymer, a polyamine, a protein, a toxin, a vitamin, polyethylene glycol, a hapten, digoxin, biotin, a radioactive atom or molecule, or a fluorophore;
cc) the active molecule G is a natural or modified oligonucleotide;
dd) the active molecule G is ASO, siRNA, or miRNA;
ee) the scaffold has a monodentate, bidentate, tridentate, or tetradentate structure; or
ff) the active molecule G is attached to one, two, three, or four compounds represented by Formula I via the scaffold.

For the oligonucleotide, as used herein, the term "natural or modified oligonucleotide" refers to an oligonucleotide composed of natural nucleic acids or an oligonucleotide including modified nucleic acids. The term "native or modified polynucleotide" refers to a polynucleotide composed of natural nucleic acids or a polynucleotide including modified nucleic acids.

In a preferred embodiment, the active molecule G is attached to ring B and/or ring C of the compound represented by Formula I. In a preferred embodiment, the active molecule G is attached to one, two, three, or four compounds represented by Formula I via the scaffold.

In a preferred embodiment, the conjugate is 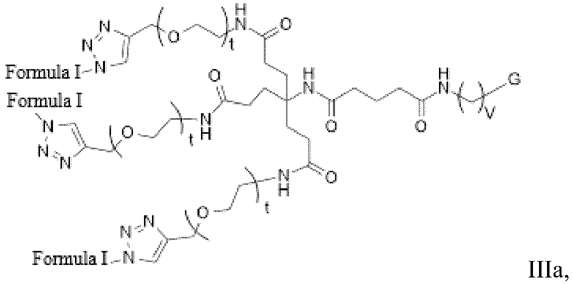 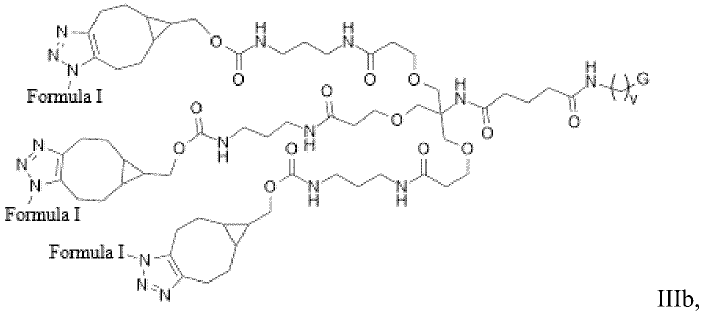 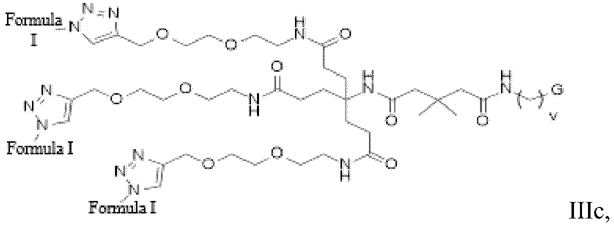 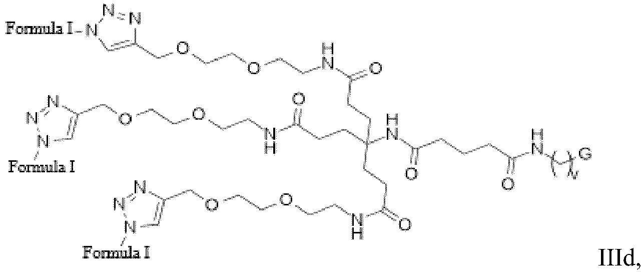 or 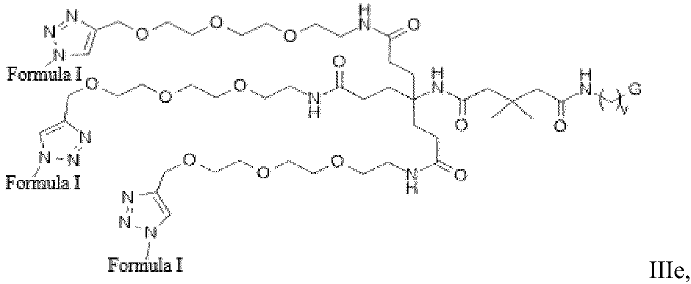 where:
Formula I is the compound represented by Formula I, and G is the active molecule G; and
v ranges from 1 to 10.

In a preferred embodiment, t is 1, 2, 3, 4, 5, or 6.

In a preferred embodiment, t is 4.

In a preferred embodiment, v is 4, 5, 6, 7, or 8.

In a preferred embodiment, the active molecule G is a natural or modified oligonucleotide.

In a preferred embodiment, the active molecule G is ASO, siRNA, or miRNA.

In a preferred embodiment, the structure of the conjugate is as follows:

| Structure of Conjugate | | | Structure of Conjugate | | | Structure of Conjugate | | | Structure of Conjugate | | | Structure of Conjugate | | |
|---|---|---|---|---|---|---|---|---|---|---|---|---|---|---|
| Conju gate | Formula I | v | Conju gate | Formula I | v | Conjug ate | Formula I | v | Conju gate | Formula I | v | Conj ugate | Formula I | v |
| IIIa | IIb-1 | 6 | IIIb | IIb-1 | 6 | IIIc | IIb-1 | 6 | IIId | IIb-1 | 6 | IIIe | IIb-1 | 6 |
| | IIb-2 | 6 | | IIb-2 | 6 | | IIb-2 | 6 | | IIb-2 | 6 | | IIb-2 | 6 |
| | IIb-3 | 6 | | IIb-3 | 6 | | IIb-3 | 6 | | IIb-3 | 6 | | IIb-3 | 6 |
| | IIb-4 | 6 | | IIb-4 | 6 | | IIb-4 | 6 | | IIb-4 | 6 | | IIb-4 | 6 |
| | IIb-5 | 6 | | IIb-5 | 6 | | IIb-5 | 6 | | IIb-5 | 6 | | IIb-5 | 6 |
| | IIb-6 | 6 | | IIb-6 | 6 | | IIb-6 | 6 | | IIb-6 | 6 | | IIb-6 | 6 |
| | IIb-7 | 6 | | IIb-7 | 6 | | IIb-7 | 6 | | IIb-7 | 6 | | IIb-7 | 6 |
| | IIb-8 | 6 | | IIb-8 | 6 | | IIb-8 | 6 | | IIb-8 | 6 | | IIb-8 | 6 |
| | IIb-9 | 6 | | IIb-9 | 6 | | IIb-9 | 6 | | IIb-9 | 6 | | IIb-9 | 6 |
| | IIb-10 | 6 | | IIb-10 | 6 | | IIb-10 | 6 | | IIb-10 | 6 | | IIb-10 | 6 |
| | IIb-11 | 6 | | IIb-11 | 6 | | IIb-11 | 6 | | IIb-11 | 6 | | IIb-11 | 6 |
| | IIb-12 | 6 | | IIb-12 | 6 | | IIb-12 | 6 | | IIb-12 | 6 | | IIb-12 | 6 |
| | IIb-13 | 6 | | IIb-13 | 6 | | IIb-13 | 6 | | IIb-13 | 6 | | IIb-13 | 6 |
| | IIb-14 | 6 | | IIb-14 | 6 | | IIb-14 | 6 | | IIb-14 | 6 | | IIb-14 | 6 |
| | IIb-15 | 6 | | IIb-15 | 6 | | IIb-15 | 6 | | IIb-15 | 6 | | IIb-15 | 6 |
| | IIb-16 | 6 | | IIb-16 | 6 | | IIb-16 | 6 | | IIb-16 | 6 | | IIb-16 | 6 |
| | IIb-17 | 6 | | IIb-17 | 6 | | IIb-17 | 6 | | IIb-17 | 6 | | IIb-17 | 6 |
| | IIb-18 | 6 | | IIb-18 | 6 | | IIb-18 | 6 | | IIb-18 | 6 | | IIb-18 | 6 |
| | IIb-19 | 6 | | IIb-19 | 6 | | IIb-19 | 6 | | IIb-19 | 6 | | IIb-19 | 6 |
| | IIb-20 | 6 | | IIb-20 | 6 | | IIb-20 | 6 | | IIb-20 | 6 | | IIb-20 | 6 |
| | IIb-21 | 6 | | IIb-21 | 6 | | IIb-21 | 6 | | IIb-21 | 6 | | IIb-21 | 6 |
| | IIb-22 | 6 | | IIb-22 | 6 | | IIb-22 | 6 | | IIb-22 | 6 | | IIb-22 | 6 |
| | IIb-23 | 6 | | IIb-23 | 6 | | IIb-23 | 6 | | IIb-23 | 6 | | IIb-23 | 6 |
| | IIb-24 | 6 | | IIb-24 | 6 | | IIb-24 | 6 | | IIb-24 | 6 | | IIb-24 | 6 |
| | IIb-25 | 6 | | IIb-25 | 6 | | IIb-25 | 6 | | IIb-25 | 6 | | IIb-25 | 6 |
| | IIb-26 | 6 | | IIb-26 | 6 | | IIb-26 | 6 | | IIb-26 | 6 | | IIb-26 | 6 |
| | IIb-27 | 6 | | IIb-27 | 6 | | IIb-27 | 6 | | IIb-27 | 6 | | IIb-27 | 6 |
| | IIb-28 | 6 | | IIb-28 | 6 | | IIb-28 | 6 | | IIb-28 | 6 | | IIb-28 | 6 |

The specific structures of IIb-1 to IIb-28 are as set forth in the structures described above in the first aspect of the present disclosure.

In a preferred embodiment, the conjugate includes the following structures: 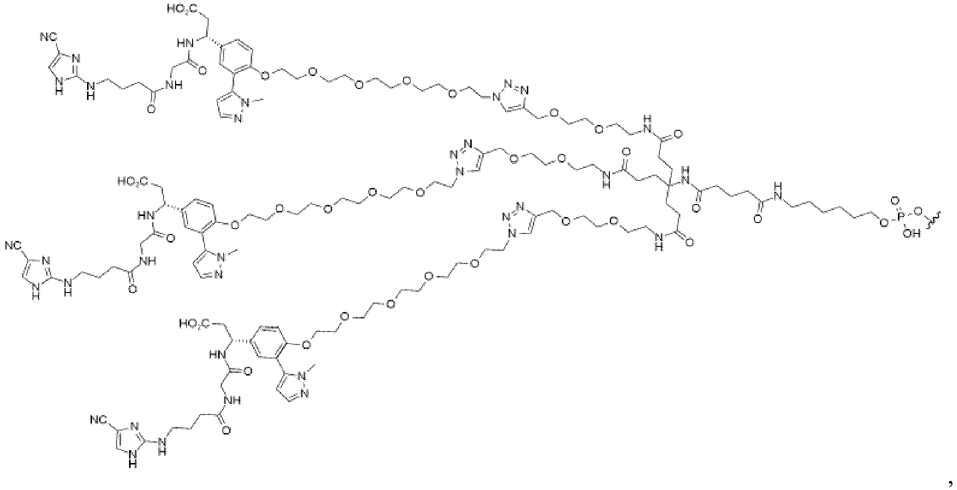 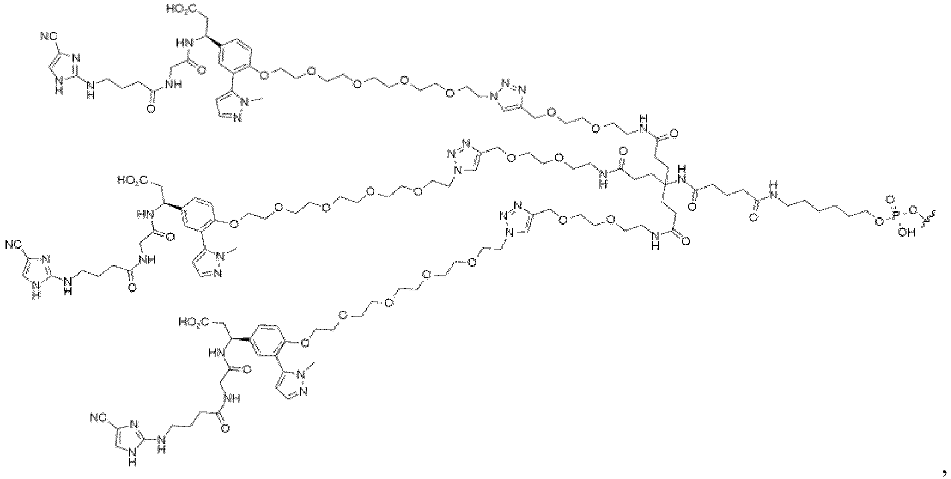 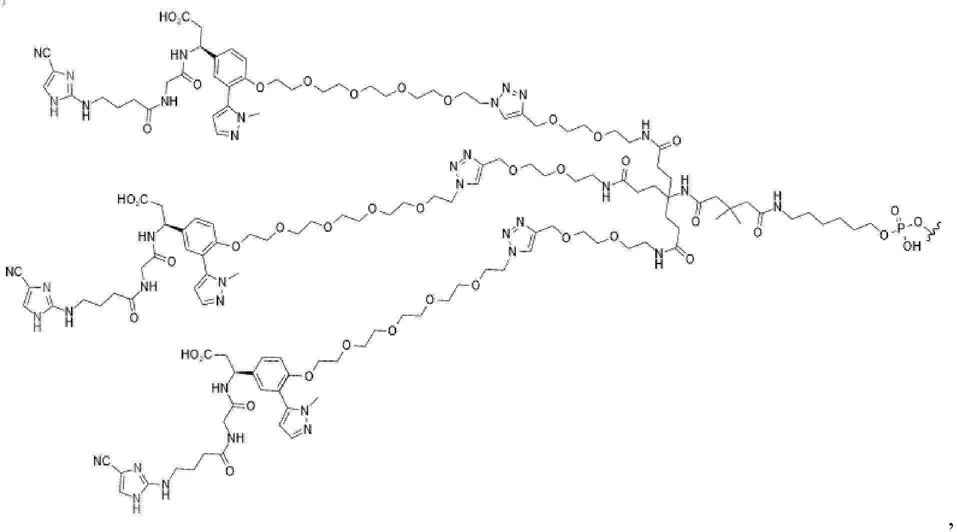 or 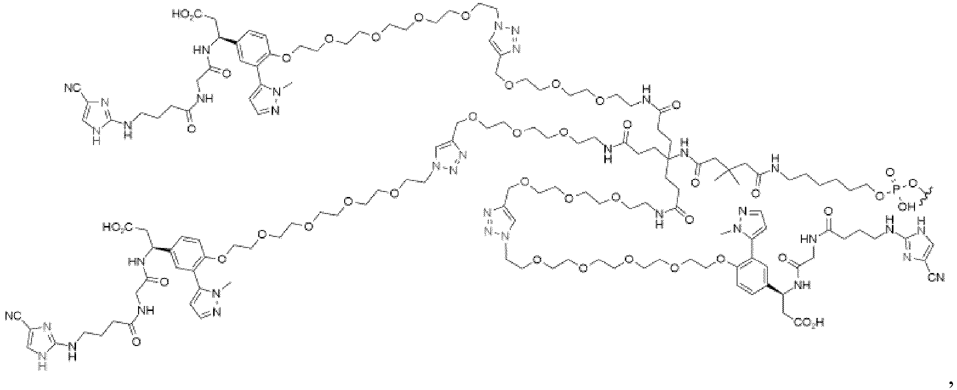 where 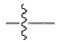 represents an attachment point to an oligonucleotide.

In a third aspect of the present disclosure, there is provided a composition. The composition includes the compound represented by Formula I, or the tautomer or stereoisomer thereof according to the first aspect, or includes the conjugate according to the second aspect.

In a preferred embodiment, the composition further includes a pharmaceutically acceptable excipient.

In a fourth aspect of the present disclosure, there is provided use of the compound represented by Formula I, or the tautomer or stereoisomer thereof according to the first aspect, the conjugate according to the second aspect, or the composition according to the third aspect in the manufacture of a medicament.

In a preferred embodiment, the medicament is for use in:
i) the delivery of an active molecule G to a cell; or
ii) the inhibition of expression of a target gene in the cell; or
iii) the treatment of a pulmonary disease.

In a preferred embodiment, the cell is a cell expressing integrin αvβ6.

In a preferred embodiment, the cell is a type I or a type II alveolar epithelial cell, a goblet cell, a secretory epithelial cell, a ciliated epithelial cell, a corneal or conjunctival epithelial cell, a dermal epithelial cell, a bile duct epithelial cell, an intestinal epithelial cell, a ductal epithelial cell, a glandular epithelial cell, or an epithelial tumor (carcinoma).

In a fifth aspect of the present disclosure, there is provided the compound represented by Formula I, or the tautomer or stereoisomer thereof according to the first aspect, the conjugate according to the second aspect, or the composition according to the third aspect, for use in:
i) delivering an active molecule G to a cell; and/or
ii) inhibiting expression of a target gene in a cell; and/or
iii) treating a pulmonary disease; and/or
iv) preparing a medicament for inhibiting expression of a target gene in a cell; and/or
v) preparing a medicament for treating a pulmonary disease.

In a preferred embodiment, the cell is a cell expressing integrin αvβ6.

In a preferred embodiment, the cell is a type I or type II alveolar epithelial cell, a goblet cell, a secretory epithelial cell, a ciliated epithelial cell, a corneal or conjunctival epithelial cell, a dermal epithelial cell, a bile duct epithelial cell, an intestinal epithelial cell, a ductal epithelial cell, a glandular epithelial cell, or an epithelial tumor (carcinoma).

In a sixth aspect of the present disclosure, there is provided a method for delivering an active molecule to a cell, inhibiting expression of a target gene in a cell, or treating a pulmonary disease. The method includes: administering to a patient the compound represented by Formula I, or the tautomer or stereoisomer thereof according to the first aspect, the conjugate according to the second aspect, or the composition according to the third aspect.

In a preferred embodiment, the cell is the cell according to the fourth aspect.

In a preferred embodiment, the active molecule is the active molecule G.

In a preferred embodiment, the active molecule G is ASO, siRNA, or miRNA.

In a seventh aspect of the present disclosure, there is provided a compound. The compound includes any one of the following structures: 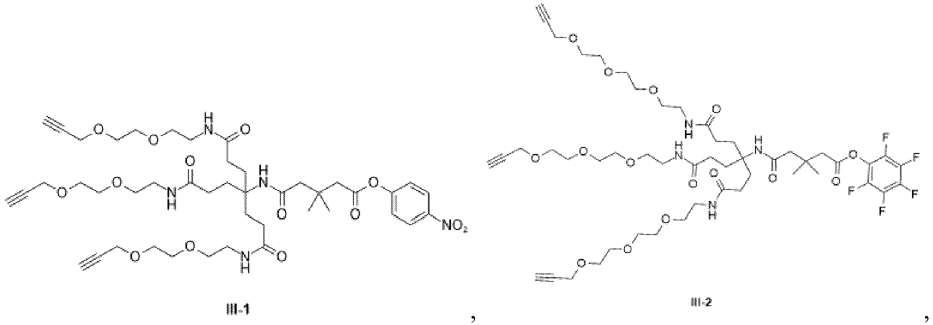 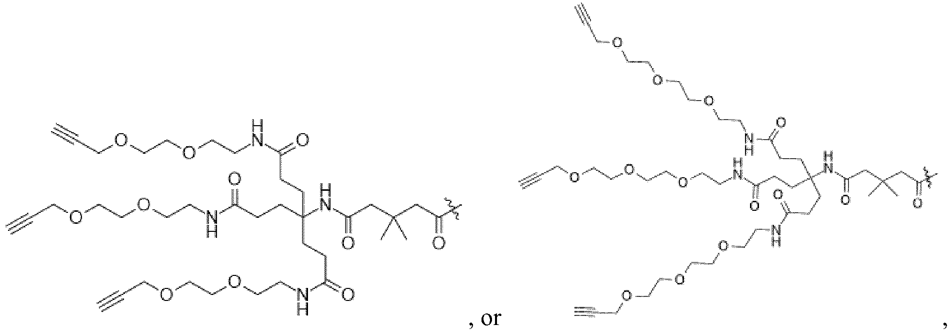 where 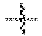 represents an attachment point.

### Linker

As disclosed herein, the targeting compound further includes a linker, via which the active molecule is further linked. In some embodiments, the linker is the linker T as described herein. In some embodiments, the linker may include a polyethylene glycol (PEG) unit structure. The linker may include 2 to 20 polyethylene glycol unit structures.

In some embodiments, the linker is 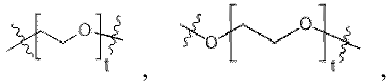 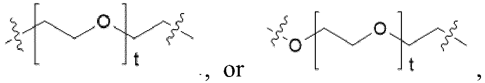 where 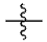 represents an attachment site respectively linked to the targeting compound and the active molecule.

In some embodiments, at the end linked to the active molecule, the linker includes a reactive group, through which the active molecule is directly conjugated, or the active molecule is conjugated after connection with a scaffold. In some embodiments, the reactive group is an azido group or an alkynyl-containing group.

In some embodiments, the linker is 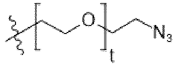 or

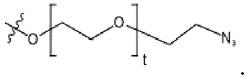

In some embodiments, t ranges from 1 to 10.

In some embodiments, preferably, t as defined in any one of the above is 1, 2, 3, 4, 5, 6, or 7.

The attachment site between the linker and the targeting compound is not particularly limited. In some embodiments, the linker is attached to ring B or ring C of the targeting compound (the compound represented by Formula I of the present disclosure).

In some embodiments, the compound represented by Formula I has a structure of:

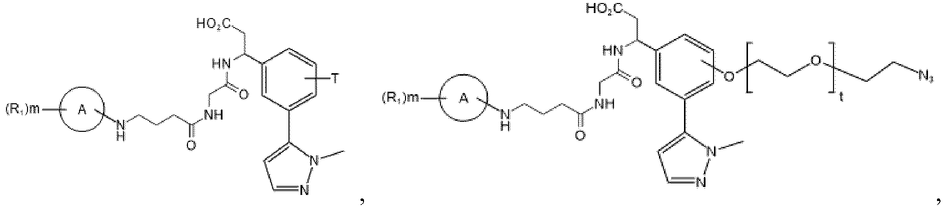

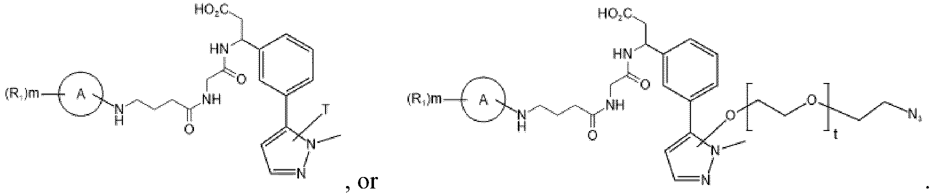

### Targeting Compound and Scaffold

As disclosed herein, in some embodiments, one or more targeting compounds (the compound represented by Formula I of the present disclosure) may be linked to one or more transported active molecules. In some embodiments, only one targeting compound is conjugated to the active molecule (referred to herein as a "monodentate" or "monovalent" ligand). In some embodiments, two targeting compounds are conjugated to the active molecule (referred to herein as a "bidentate" or a "bivalent" ligand). In some embodiments, three targeting compounds are conjugated to the active molecule (referred to herein as a "tridentate" or a "trivalent" ligand). In some embodiments, four targeting compounds are conjugated to the active molecule (referred to herein as a "tetradentate" or a "tetravalent" ligand). In some embodiments, more than four are conjugated to the active molecule.

As disclosed herein, in some embodiments, the scaffold and the active molecule are linked to the targeting compound via the linker. In some embodiments, the scaffold has a monodentate, bidentate, tridentate, or tetradentate structure. In some embodiments, the active molecule is linked to a targeting compound via a monodentate scaffold. In some embodiments, the active molecule is linked to two targeting compounds via a bidentate scaffold. In some embodiments, the active molecule is linked to three targeting compounds via a tridentate scaffold. In some embodiments, the active molecule is linked to four targeting compounds via a tetradentate scaffold.

The scaffolds are generally known in the art. Non-limiting examples of scaffolds that may be used with the targeting compounds disclosed herein include, but are not limited to, polymers and polyamino acids (e.g., diglutamic acid, poly-L-lysine). In some embodiments, the scaffold may include an amine-reactive group, an amide bond, and an alkyne-containing moiety (e.g., alkynyl, cyclooctyne). In some embodiments, the alkyne-containing moiety includes, but is not limited to, alkyne maleimide, and alkyne NHS ester.

In some embodiments, the scaffold may include a cysteine linker or group, dibenzocyclooctyne (DBCO)-PEG₁₋₂₄-NHS, propargyl-PEG₁₋₂₄-NHS, and/or multidentate DBCO and/or propargyl moieties.

In some embodiments, a typical tridentate structure scaffold is, for example:

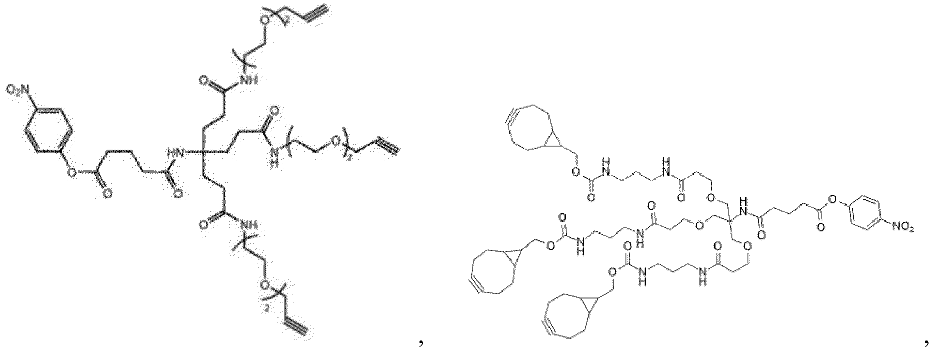

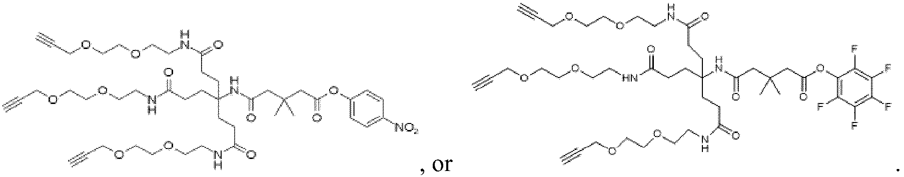

The scaffold includes an amine-reactive group, an amide bond, three PEG₂ units, and alkyne. The amine-reactive group may be conjugated, through amide formation, to a primary amine, such as a terminal amine group (e.g., NH₂-C₆), on the transported active molecule. The alkyne may be conjugated to an azido-modified linker to form a triazole structure, which can then be linked to the targeting compound. In the scaffold, the amine-reactive group is p-nitrophenol (also referred to as 4-nitrophenol) ester.

In some embodiments, the amine-reactive group is not particularly limited. The amine-reactive group may also be, for example,

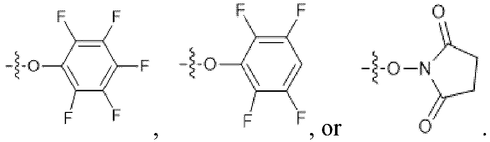

In some embodiments, the scaffold may be synthesized as a phosphoramidite compound, which can allow the tridentate scaffold to be coupled to an oligonucleotide (e.g., the 5' terminus of the sense strand) through phosphoramidite synthesis, as shown in the following structure: 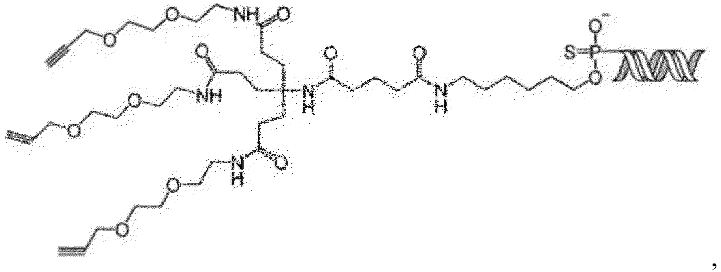 where 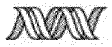 represents an oligonucleotide, such as a siRNA.

### Conjugate

The present disclosure provides a conjugate having a structure of 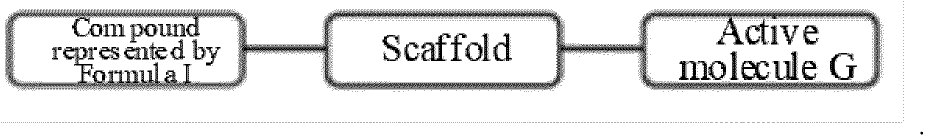 As disclosed herein above, in some embodiments, one or more integrin αvβ6 ligands (e.g., the compound represented by Formula I of the present disclosure) may be linked to one or more transported active molecules.

In some embodiments, the active molecule G may be coupled to the scaffold through phosphoramidite synthesis. The scaffold may have a monodentate, bidentate, tridentate, or tetradentate structure. In some embodiments, the azide group (e.g., 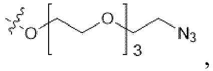 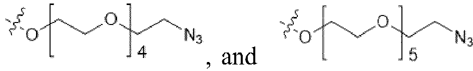 ) on the targeting compound may react (e.g., through a click chemical reaction) with the alkyne (e.g., benzocyclooctyne, propargyl) of the scaffold to form a triazole structure, which is then connected to the targeting compound. An exemplary structure is: 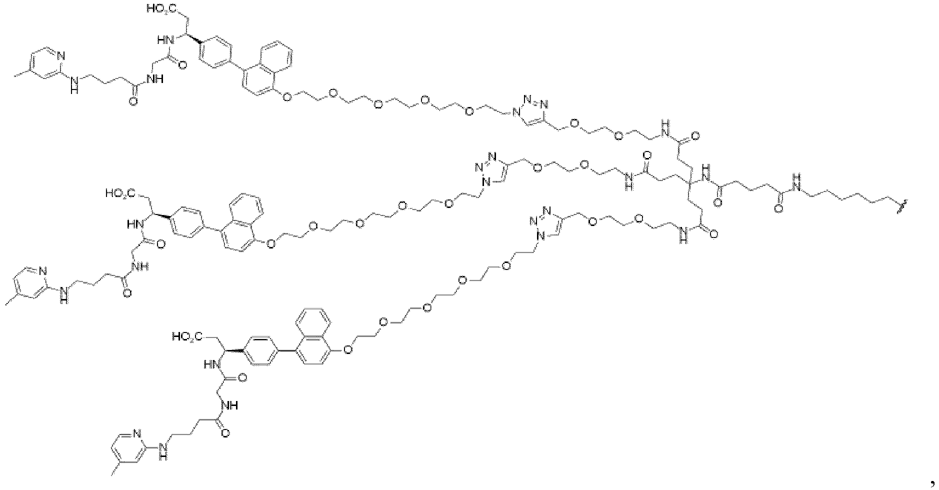 where 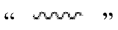 represents an attachment point to an active molecule (e.g., an oligonucleotide).

The active molecule described herein can be linked to the targeting compound to form a conjugate. In some embodiments, the conjugate is the conjugate according to the third aspect.

### Active Molecule

The "active molecule" described herein may be linked to the targeting compound and thereby transported to cells and/or tissues expressing integrin αvβ6. The transported "active molecule" (or referred to as the transported molecule) is any molecule that, when dissociated from the integrin αvβ6 ligands described herein, can exert a desired effect on cells including an integrin αvβ6 receptor. The transported active molecule may be, but is not limited to, a pharmaceutical ingredient, a pharmaceutical product, a prodrug, a substance having known therapeutic benefit, a small molecule, an antibody, an antibody fragment, an immunoglobulin, a monoclonal antibody, a label or marker, a lipid, a natural or modified nucleic acid, an oligonucleotide or a polynucleotide, a peptide, a polymer, a polyamine, a protein, a nucleic acid aptamer, a toxin, a vitamin, PEG, a hapten, digoxin, biotin, a radioactive atom or molecule, or a fluorophore. In some embodiments, one or more transported active molecules (e.g., identical or different transported active molecules) are linked to one or more integrin αvβ6 ligands in order to target the transported active molecules to the cells expressing integrin αvβ6. In some embodiments, the active molecule is the active molecule G according to the present disclosure.

### Oligonucleotide

In the present disclosure, the term "oligonucleotide" refers to a short-chain nucleotide consisting of less than 50 bases (including nucleotides within deoxyribonucleic acid (DNA) or ribonucleic acid (RNA)). The oligonucleotide of the present disclosure may have a single-stranded structure or a double-stranded structure, and the specific type is not limited and falls within the scope of the present disclosure. Exemplarily, in the case of the double-stranded structure, the RNAi agent is composed of a sense strand and an antisense strand, and includes, but is not limited to: short (or small) interfering RNA (siRNA), double-stranded RNA (dsRNA), microRNA (miRNA), short hairpin RNA (shRNA), and dicer substrate; and in the case of the single-stranded structure, the RNAi agent includes, but is not limited to, an antisense oligonucleotide (ASO).

Additional aspects and advantages of the embodiments of the present disclosure will be provided at least in part in the following description, or will become apparent in part from the following description, or can be learned from the practice of the embodiments of the present disclosure.

### Terms and Definitions

Unless otherwise stated, the definitions of groups and terms described in the specification and claims include actual definitions, exemplary definitions, preferred definitions, definitions recorded in tables, and definitions of specific compounds in the examples, etc., which may be arbitrarily combined and integrated with each other. The group definitions and compound structures that are combined and integrated should fall within the scope of the present disclosure.

Unless defined otherwise, all technical and scientific terms herein have the same meaning as commonly understood by one of ordinary skill in the art to which the claimed subject matter belongs. The patents, patent applications, publications cited herein are hereby incorporated by reference in their entireties, unless stated otherwise. When a term has multiple definitions, the definition in this section will prevail.

It should be understood that the foregoing general description and the following detailed description are illustrative and merely for explanation, rather than limiting the subject matter of the present disclosure in any way. In the present disclosure, unless specifically stated otherwise, the use of the singular also includes the plural. It must be noted that, as used in this specification and the claims, the singular forms include plural referents unless the context clearly dictates otherwise. It should also be noted that the use of "or" or "alternatively" means "and/or" unless stated otherwise. Furthermore, use of the term "include" or "comprise", as well as other forms, such as "comprising", "including", and "containing", is not for limitation.

Definitions of standard chemical terms may be found in reference document (including "ADVANCED ORGANIC CHEMISTRY 4THED.", Carey and Sundberg, Vols. A (2000) and B (2001), Plenum Press, New York). Unless otherwise indicated, conventional methods in the related art, for example, mass spectroscopy, NMR, IR and UV/VIS spectroscopy, and pharmacological methods, are employed. Unless specific definitions are set forth, the terms in the related description of analytical chemistry, organic synthetic chemistry, and medicinal and medicinal chemistry are those known in the art. Standard techniques may be used in chemical synthesis, chemical analysis, pharmaceutical preparation, formulation and delivery, and treatment of patients. For example, reactions and purifications can be performed using the manufacturer's instructions of the kit, or in a manner well known in the related art or as described herein. The techniques and procedures described above may generally be performed with conventional methods well known in the art according to the description in a number of general and more specific documents cited and discussed throughout the present description. Throughout the description, groups and substituents thereof can be chosen by one skilled in the field to provide stable moieties and compounds.

Where substituent groups are depicted by conventional chemical formulae, written from left to right, the substituent groups also encompass the chemically equivalent substituents that would result from writing the structural formula from right to left. For example, CH₂O is equivalent to OCH₂. As used herein, 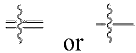 refers to the point of attachment of a group. As used herein, "R₁", "R1", and "R¹" have the same meaning and are interchangeable. For other symbols such as R₂, similar definitions have the same meanings.

The section headings used herein are for organizational purposes only and are not to be construed as limiting the subject matter described. All documents or portions of documents cited in the present disclosure, including but not limited to patents, patent applications, articles, books, manuals, and treatises, are incorporated herein by reference in their entireties.

In addition to the foregoing, the following terms, when used in the specification and claims of the present disclosure, have the meanings indicated below, unless otherwise specifically stated.

As used herein, numerical ranges recited in the specification and claims, when interpreted as "integers", should be understood as including both endpoints of the range and each integer within the range. For example, "an integer ranging from 0 to 5" should be understood as reciting each integer of 0, 1, 2, 3, 4, and 5.

In present disclosure, the term "halogen", either alone or as part of other substituents, refers to fluorine, chlorine, bromine, and iodine.

As used herein, the term "amino", either alone or as part of other substituents, refers to -NH₂.

As used herein, the term "nitro", either alone or as part of other substituents, refers to -NO₂.

As used herein, the term "cyano", either alone or as part of other substituents, refers to -CN.

As used herein, the term "alkyl", either alone or as part of other substituents, means a straight or branched hydrocarbon chain group consisting solely of carbon and hydrogen atoms, containing no unsaturated bonds, and having, for example, 1 to 6 carbon atoms and connected to the rest of the molecule by a single bond. Examples of alkyl include, but are not limited to, methyl, ethyl, n-propyl, isopropyl, n-butyl, isobutyl, tert-butyl, pentyl, isopentyl, neopentyl, and hexyl. Alkyl may be unsubstituted or substituted with one or more suitable substituents. Alkyl may also be an isotopic isomer of the naturally abundant alkyl enriched in an isotope of carbon and/or hydrogen (i.e., deuterium or tritium).

The term "C₁ to C₆ alkyl", either alone or as part of other substituents, should be interpreted as a straight or branched chain saturated monovalent hydrocarbon radical having 1, 2, 3, 4, 5, or 6 carbon atoms. Examples of "C₁ to C₆ alkyl" are, for example, methyl, ethyl, propyl, butyl, pentyl, hexyl, isopropyl, isobutyl, sec-butyl, tert-butyl, isopentyl, 2-methylbutyl, 1-methylbutyl, 1-ethylpropyl, 1,2-dimethylpropyl, neopentyl, 1,1-dimethylpropyl, 4-methylpentyl, 3-methylpentyl, 2-methylpentyl, 1-methylpentyl, 2-ethylbutyl, 1-ethylbutyl, 3,3-dimethylbutyl, 2,2-dimethylbutyl, 1,1-dimethylbutyl, 2,3-dimethylbutyl, 1,3-dimethylbutyl, 1,2-dimethylbutyl, or isomers thereof. The term "C₁ to C₅ alkyl" should be interpreted as a straight or branched chain saturated monovalent hydrocarbon radical having 1, 2, 3, or 5 carbon atoms. In particular, the group has 1, 2, or 3 carbon atoms ("C₁ to C₃ alkyl"), for example, methyl, methylene, ethyl, n-propyl, or isopropyl.

The term "C₁ to C₆ alkoxy", either alone or as part of other substituents, should be interpreted to include a straight or branched saturated monovalent hydrocarbon radical having 1, 2, 3, 4, 5, or 6 carbon atoms and an oxygen atom, or present as C₁ to C₆ alkyl-O-C₁ to C₆ alkyl as defined in the present specification, the oxygen atom being attached to any carbon atom of the straight or branched chain of the C₁ to C₆ alkyl, including but not limited to: methoxy (CH₃-O-), ethoxy (C₂H₅-O-), propoxy (C₃H₇-O-), and butoxy (C₄H₉-O-).

The term "cycloalkyl" or "carbocyclyl", either alone or as part of other substituents, refers to a cyclic alkyl. The term "m- to n-membered cycloalkyl" or "Cₘ to Cₙ cycloalkyl" should be interpreted as a saturated, unsaturated, or partially saturated carbocyclic ring having m to n atoms. For example, "3- to 10-membered cycloalkyl" or "C₃ to C₁₀ cycloalkyl" refers to a cyclic alkyl group containing 3 to 10 carbon atoms, which may contain 1 to 3 rings. The cyclic alkyl group includes monocyclic, bicyclic, tricyclic, spirocyclic, or bridged ring structures. Examples of unsubstituted cycloalkyl include, but are not limited to, cyclopropyl, cyclobutyl, cyclopentyl, cyclohexyl, and adamantyl, or a bicyclic hydrocarbon group such as a decalin ring. Cycloalkyl may be substituted with one or more substituents. In some embodiments, cycloalkyl may be a cycloalkyl fused to an aryl or heteroaryl.

The term "heterocycloalkyl" or "heterocyclyl", either alone or as part of other substituents, refers to cycloalkyl in which one or more (in some embodiments, 1 to 3) carbon atoms are replaced with heteroatoms, such as, but not limited to, N, O, S, and P. The term "m-to n-membered heterocycloalkyl" or "Cₘ to Cₙ heterocycloalkyl" should be interpreted as a saturated, unsaturated, or partially saturated ring having m to n atoms. For example, the term "4- to 10-membered heterocycloalkyl" should be interpreted as a saturated, unsaturated, or partially saturated ring having 4 to 10 atoms. In some embodiments, heterocycloalkyl may be a heterocycloalkyl fused to an aryl or heteroaryl. When a prefix such as "3- to 8-membered" is used to denote heterocycloalkyl, the number of carbons is also meant to include heteroatoms. It includes monocyclic, bicyclic, tricyclic, spirocyclic, or bridged ring structures.

The term "heteroaryl", either alone or as part of other substituents, may be used interchangeably with the term "heteroaromatic ring" or "heteroaromatic group" and refers to a monocyclic or polycyclic aromatic ring system. In some embodiments, 1 to 3 atoms of the ring system are heteroatoms, i.e., elements other than carbon, including but not limited to N, O, S, or P. Examples of heteroaryl include furyl, imidazolyl, indolyl, pyrrolidinyl, pyrimidinyl, tetrazolyl, thienyl, pyridyl, pyrrolyl, N-methylpyrrolyl, quinolyl, and isoquinolyl. Heteroaryl may be optionally fused to a benzene ring, and may include monocyclic, bicyclic, tricyclic, spirocyclic, or bridged ring structures.

The term "5- to 10-membered heteroaryl", either alone or as part of other substituents, should be interpreted to include a monovalent monocyclic, bicyclic, or tricyclic aromatic ring group having 5 to 10 ring atoms and containing heteroatoms selected from N, O, and S. It should be interpreted as a monovalent monocyclic, bicyclic, or tricyclic aromatic ring group having 5, 6, 7, 8, 9, or 10 ring atoms-in particular 5, 6, 9, or 10 carbon atoms-and containing 1 to 5, preferably 1 to 3, heteroatoms independently selected from N, O, and S, and which may additionally in each case be benzo-fused. The term "5- to 8-membered heteroaryl" should be interpreted as a monovalent monocyclic, bicyclic, or tricyclic aromatic ring group having 5 to 8 ring atoms-in particular 5 or 6 carbon atoms-and containing 1 to 5, preferably 1 to 3, heteroatoms independently selected from N, O, and S, and which may additionally in each case be benzo-fused. Examples of heteroaryl include, but are not limited to: thienyl, furyl, pyrrolyl, oxazolyl, thiazolyl, imidazolyl, pyrazolyl, isoxazolyl, isothiazolyl, oxadiazolyl, triazolyl, thiadiazolyl, and the like, as well as their benzo derivatives, such as benzofuryl, benzothienyl, benzoxazolyl, benzisoxazolyl, benzimidazolyl, benzotriazolyl, indazolyl, indolyl, and isoindolyl; or pyridyl, pyridazinyl, pyrimidinyl, pyrazinyl, triazinyl, and the like, as well as their benzo derivatives, such as quinolinyl, quinazolinyl, and isoquinolinyl; or azocinyl, indolizinyl, purinyl, and the like, as well as their benzo derivatives; or cinnolinyl, phthalazinyl, quinazolinyl, quinoxalinyl, naphthyridinyl, pteridinyl, carbazolyl, acridinyl, phenazinyl, phenothiazinyl, phenoxazinyl, and the like.

The term "halogenated", either alone or as part of other substituents, may be used interchangeably with the term "halogen-substituted". "Halogenated alkyl" or "halogen-substituted alkyl" refers to a saturated aliphatic hydrocarbon group including branched and straight chains having a specified number of carbon atoms, and substituted with one or more halogens (e.g., -CᵥF_{w}, where v=1 to 3, and w=1 to (2v+1)). Examples of halogenated alkyl include, but are not limited to, trifluoromethyl, trichloromethyl, pentafluoroethyl, pentachloroethyl, 2,2,2-trifluoroethyl, heptafluoropropyl, and heptachloropropyl.

The compound provided in the present disclosure includes intermediates useful for preparing the compound provided in the present disclosure. The intermidiates contain reactive functional groups (such as, but not limited to, carboxyl, hydroxyl, and amino moieties), and also include protected derivatives thereof. "Protected derivatives" are those compounds in which one or more reactive sites are blocked by one or more protecting groups. Suitable protecting groups for carboxyl moieties include benzyl, tert-butyl, and the like, as well as isotopes and the like. Suitable protecting groups for amino and amido include acetyl, trifluoroacetyl, tert-butoxycarbonyl, benzyloxycarbonyl, and the like. Suitable protecting groups for hydroxyl moieties include benzyl and the like. Other suitable protecting groups are well-known to those skilled in the art.

In the present disclosure, the term "optional" or "optionally" means that the subsequently described event or situation may or may not occur, and such expression includes both occurrences and non-occurrences of the event or condition. For example, "optionally substituted aryl" means that the aryl is substituted or unsubstituted, and such expression includes both substituted and unsubstituted aryl.

In the present disclosure, the term "salt" or "pharmaceutically acceptable salt" includes pharmaceutically acceptable acid addition salts and pharmaceutically acceptable base addition salts. The term "pharmaceutically acceptable" means that the compounds, materials, compositions, and/or dosage forms are suitable for contact with human and animal tissues without excess toxicity, irritation, allergic reactions, or other problems or complications within the scope of reliable medical judgment, and are commensurate with a reasonable benefit/risk ratio.

As used herein, the term "pharmaceutically acceptable acid addition salt" refers to a salt formed with an inorganic acid or organic acid that retains the biological effectiveness of the free base without other adverse effects. "Pharmaceutically acceptable base addition salt" refers to a salt formed with an inorganic base or an organic base that retains the biological effectiveness of the free acid without other adverse effects. In addition to pHarmaceutically acceptable salts, other salts may be adopted in the present disclosure, and they can serve as intermediates in the purification of compounds or in the preparation of other pharmaceutically acceptable salts, or can be used for identifying, characterizing, or purifying the compounds of the present disclosure.

The term "stereoisomer" refers to isomers formed due to different spatial arrangements of atoms in a molecule, including a cis-trans isomer, an enantiomer, a diastereoisomer, and a conformational isomer.

According to selected starting materials and methods, the compounds of the present disclosure may be present in the form of a possible isomer or a mixture of isomers, for example, in the form of a pure optical isomer or a mixture of isomers such as a mixture of racemate and diastereoisomer, depending on the number of asymmetric carbon atoms. When an optically active compound is described, prefixes *D* and *L*, or *R* and *S* are used to represent an absolute configuration of a molecule with respect to a chiral center (or multiple chiral centers) in the molecule. Prefixes *D* and *L*, or (*+*) and (*-*) are symbols used for designating the rotation of plane polarized light caused by a compound, and (*-*) or *L* indicates that a compound is levorotatory. Compounds with the prefix (*+*) or *D* are dextrorotatory.

When a bond to a chiral carbon in the formula of the present disclosure is depicted as a straight line, it is to be understood that (*R*) and (*S*) configurations of the chiral carbon and produced enantiomerically pure compounds and mixtures are all included within the scope of the general formula. The racemate or enantiomerically pure compounds of the present disclosure are graphically represented with reference to Maehr, J. Chem. Ed. 1985, 62: 114-120. A wedge bond and a dashed bond are used to represent an absolute configuration of a stereocenter.

The term "tautomer" refers to functional group isomers formed by rapid movement of a certain atom in a molecule between two positions. The compounds of the present disclosure may have a tautomerism phenomenon. The tautomeric compound may be present in two or more interconvertible forms. A prototropic tautomer is formed by migration of a hydrogen atom covalently bonded between two atoms. Tautomer is generally present in an equilibrium form, and separation of a single tautomer usually yields a mixture whose physicochemical properties are consistent with those of a mixture of compounds. The position of equilibrium depends on intramolecular chemical properties. For example, in several aliphatic aldehydes and ketones such as acetaldehyde, ketonic configurations prevail, while in phenol, an enolic configuration prevails. The present disclosure covers all tautomer of the compound.

In the present disclosure, "pharmaceutical composition" refers to a formulation of the compound of the present disclosure and a medium commonly accepted in the art for delivery of a biologically active compound to a mammal (e.g., a human). The medium includes a pharmaceutically acceptable carrier. The purpose of pharmaceutical composition is to facilitate administration to an organism, facilitate the absorption of the active ingredients, and further facilitate the exertion of the biological activity.

In the present disclosure, "pharmaceutically acceptable carrier" or "pharmaceutically acceptable excipient" includes, but is not limited to, any adjuvant, carrier, excipient, glidant, sweetener, diluent, preservative, dye/colorant, flavoring agent, surfactant, wetting agent, dispersant, suspending agent, stabilizer, isotonic agent, solvent, or emulsifier approved by the relevant governmental regulatory authorities as acceptable for human or livestock use.

The term "prodrug" refers to a substance that can be transformed into the compound of the present disclosure having bioactivity under physiological conditions or by dissolving in a solvent. The prodrug of the present disclosure is prepared by modifying functional groups in the compound, and the modification can be removed by conventional operations or removed *in vivo* to obtain a parent compound. The prodrug includes a compound formed by linking one hydroxyl group or amino group in the compound of the present disclosure to any group, and when administered to an individual mammal, the prodrug of the compound of the present disclosure is cleaved to form a free hydroxyl group or free amino group.

The compound of the present disclosure may contain an unnatural proportion of atomic isotopes at one or more atoms forming the compound. For example, the compound can be labeled with radioisotopes such as deuterium (²H), tritium (³H), iodine-125 (¹²⁵I), and C-14 (¹⁴C). All changes made to the isotope composition of the compound of the present disclosure, regardless of whether they are radioactive or not, shall fall within the scope of the present disclosure.

In the present disclosure, the term "adjuvant" refers to medicinal inert ingredients. Examples of types of the term "excipient" include, but are not limited to, an adhesive, a disintegrant, a lubricant, a glidant, a stabilizer, a filler, a diluent, etc. Excipients can enhance the operating characteristics of pharmaceutical preparations, that is, improve the fluidity and/or adhesiveness to enable preparations to be more suitable for direct compression.

As used herein, the term "treatment" and other similar synonyms include the following meanings:
(i) preventing a disease or condition from occurring in a mammal, particularly where such mammal is susceptible to the disease or condition but has not yet been diagnosed as having the disease or condition;
(ii) inhibiting a disease or condition, i.e., suppressing development thereof;
(iii) alleviating a disease or condition, that is, enabling the state of the disease or condition to regress; or
(iv) alleviating the symptoms caused by the disease or condition.

For the reaction in each step, the reaction temperature may be suitably selected depending on the solvent, starting materials, reagents, etc., and the reaction time may also be suitably selected depending on the reaction temperature, solvent, starting materials, reagents, etc. After completion of the reaction in each step, the target compound may be isolated and purified from the reaction system by conventional methods, such as filtration, extraction, recrystallization, washing, and silica gel column chromatography. The target compound may also be directly used in the next step without isolation and purification, provided that it does not affect the subsequent reaction. Each step of the reaction in the present disclosure is preferably carried out in an inert solvent. The inert solvent includes, but is not limited to, toluene, benzene, water, methanol, ethanol, isopropanol, ethylene glycol, N-methylpyrrolidone, dimethyl sulfoxide, tetrahydrofuran, dichloromethane, trichloromethane, 1,2-dichloroethane, acetonitrile, N,N-dimethylformamide, N,N-dimethylacetamide, dioxane, or a combination thereof.

### DETAILED DESCRIPTION OF THE EMBODIMENTS

The present disclosure is further described below in conjunction with specific embodiments. It should be understood that the following description is only the most preferred embodiment of the present disclosure, and should not be regarded as limiting the protection scope of the present disclosure. On the basis of fully understanding the present disclosure, the experimental methods in the following embodiments for which specific conditions are not specified are generally carried out under conventional conditions or under conditions recommended by the manufacturer. Those skilled in the art can make non-essential changes to the technical solutions of the present disclosure, and such changes should fall within the protection scope of the present disclosure.

Reagents used herein:
**SEM**, i.e., (trimethylsilyl)ethoxymethyl, having the formula-CH₂OCH₂CH₂Si(CH₃)₃, is an amino-protecting group.
**Boc**, i.e., tert-butoxycarbonyl, is an amino-protecting group.
**PMB**, i.e., p-methoxybenzyl, is an amino-protecting group.

### Example 1: Preparation of Compound I-1

The synthetic route is as follows:

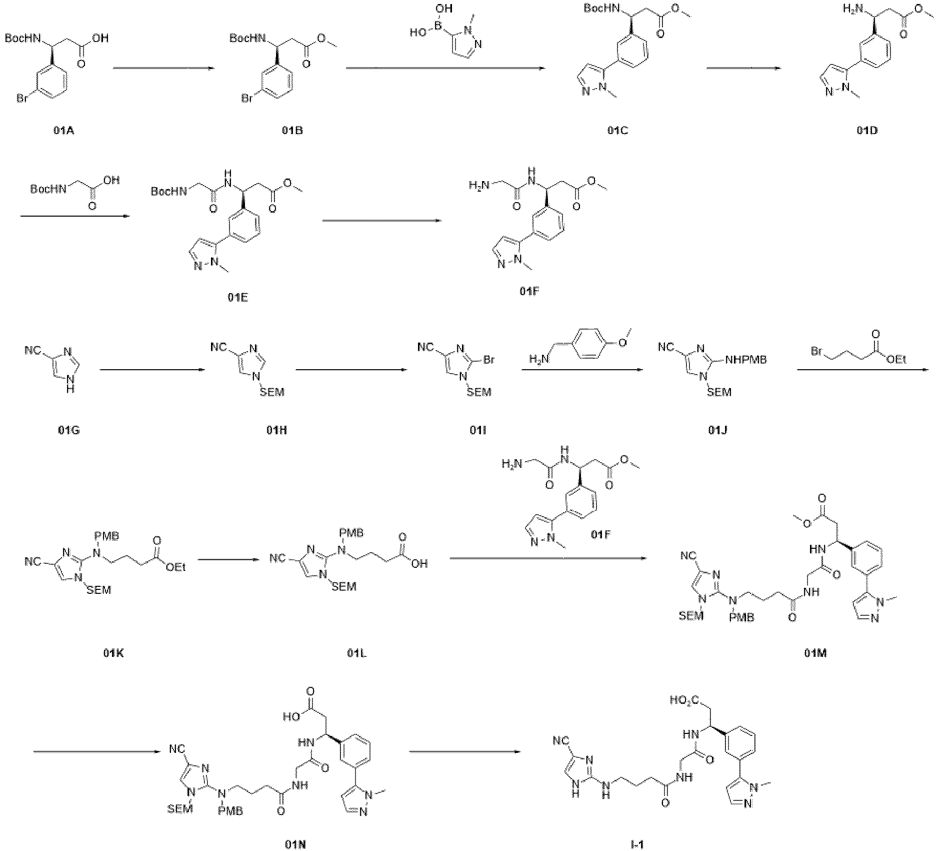

### Step 1: Synthesis of methyl (S)-3-(3-bromophenyl)-3-((tert-butoxycarbonyl)amino)propanoate (01B)

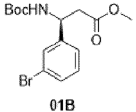

Compound (*S*)-3-(3-bromophenyl)-3-((tert-butoxycarbonyl)amino)propanoic acid (1.20 g, 3.49 mmol) was dissolved in anhydrous DMF (10 mL) at room temperature. Potassium carbonate (726 mg, 5.25 mmol) and methyl iodide (1.03 g, 7.23 mmol) were added sequentially at 0°C. After complete conversion of the starting material monitored by thin-layer chromatography (TLC), the mixture was diluted with 20 mL of water and extracted with tert-butyl methyl ether (10 mL×3). The combined organic phases were washed once with saturated NaHCO₃ solution (10 mL). The organic phase was separated, dried over anhydrous sodium sulfate, filtered, and concentrated under reduced pressure to obtain methyl (S)-3-(3-bromophenyl)-3-((tert-butoxycarbonyl)amino)propanoate (01B) (1.21 g, yield 97.2%).

LC-MS, M/Z (ESI): 358.0, 360.0 [M+H]⁺.

### Step 2: Synthesis of methyl (S)-3-((tert-butoxycarbonyl)amino)-3-(3-(1-methyl-1H-pyrazol-5-yl)phenyl)propanoate (01C)

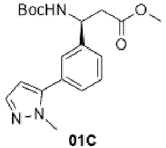

Compound methyl (S)-3-(3-bromophenyl)-3-((tert-butoxycarbonyl)amino)propanoate (1.21 g, 3.39 mmol), 1-methyl-1H-pyrazol-5-ylboronic acid (643 mg, 5.11 mmol), and Pd(PPh₃)₂Cl₂ (249 mg, 0.355 mmol), Na₂CO₃ (1.08 g, 10.2 mmol), DME (10 mL), and H₂O (2.5 mL) were added to a 100 mL round-bottom flask. The mixture was ultrasonicated and then purged with nitrogen three times and stirred at 100°C. After complete conversion of the starting material monitored by TLC, the reaction solution was filtered, and the filtrate was concentrated under reduced pressure to obtain a crude product. The crude product was purified by column chromatography (petroleum ether/ethyl acetate (V/V)=3:1) to obtain methyl (S)-3-((tert-butoxycarbonyl)amino)-3-(3-(1-methyl-1H-pyrazol-5-yl)phenyl)propanoate (01C) (360 mg, yield 29.7%).

LC-MS, M/Z (ESI): 360.2 [M+H]⁺.

### Step 3: Synthesis of methyl (S)-3-amino-3-(3-(1-methyl-1H-pyrazol-5-yl)phenyl)propanoate (01D)

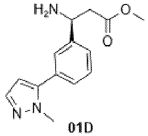

Methyl (*S*)-3-((tert-butoxycarbonyl)amino)-3-(3-(1-methyl-1H-pyrazol-5-yl)phenyl)propanoate (600 mg, 1.67 mmol), dichloromethane (20 mL), and trifluoroacetic acid (3.81 g, 33.4 mmol) were added to a 100 mL single-necked flask, and the mixture was stirred at room temperature for 4 hours. The reaction mixture was concentrated under reduced pressure to obtain a crude product. The crude product was dissolved in dichloromethane (20 mL), washed sequentially with saturated sodium bicarbonate solution (20 mL) and saturated brine (20 mL), dried over anhydrous sodium sulfate, filtered, and concentrated under reduced pressure to obtain methyl (*S*)-3-amino-3-(3-(1-methyl-1H-pyrazol-5-yl)phenyl)propanoate (01D) (430 mg, yield 99.3%).

LC-MS, M/Z (ESI): 260.13 [M+H]⁺.

### Step 4: Synthesis of methyl (S)-3-(2-((tert-butoxycarbonyl)amino)acetamido)-3-(3-(1-methyl-1H-pyrazol-5-yl)phenyl)propanoate (01E)

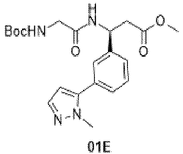

Methyl (*S*)-3-amino-3-(3-(1-methyl-1H-pyrazol-5-yl)phenyl)propanoate (400 mg, 1.54 mmol), DMF (10 mL), BOC-glycine (298 mg, 1.70 mmol), TBTU (740 mg, 2.30 mmol), and DIPEA (598 mg, 4.63 mmol) were added sequentially to a 100 mL single-necked flask. The mixture was stirred at room temperature for 16 hours under nitrogen protection. Water (10 mL) was added to the reaction solution, and the mixture was extracted with ethyl acetate (20 mL×3). The combined organic phases were washed sequentially with saturated sodium bicarbonate solution (20 mL) and saturated brine (20 mL), dried over anhydrous sodium sulfate, filtered, and concentrated to obtain a crude product. The crude product was purified by column chromatography (petroleum ether/ethyl acetate (V/V)=1:0 to 0:1) to obtain methyl (*S*)-3-(2-((tert-butoxycarbonyl)amino)acetamido)-3-(3-(1-methyl-1H-pyrazol-5-yl)phenyl)propanoate (01E) (600 mg, yield 93.4%).

LC-MS, M/Z (ESI): 417.27 [M+H]⁺.

### Step 5: Synthesis of methyl (S)-3-(2-aminoacetamido)-3-(3-(1-methyl-1H-pyrazol-5-yl)phenyl)propanoate (01F)

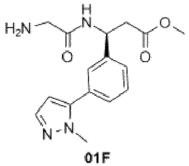

Methyl (*S*)-3-(2-((tert-butoxycarbonyl)amino)acetamido)-3-(3-(1-methyl-1H-pyrazol-5-yl)phenyl)propanoate (600 mg, 1.44 mmol), dichloromethane (10 mL), and trifluoroacetic acid (3.29 g, 28.85 mmol) were sequentially added to a 100 mL single-necked flask, and the mixture was stirred at room temperature for 4 hours. The reaction mixture was concentrated under reduced pressure to obtain a crude product. The crude product was dissolved in dichloromethane (10 mL), washed sequentially with saturated sodium bicarbonate solution (10 mL) and saturated brine (10 mL), dried over anhydrous sodium sulfate, filtered, and concentrated under reduced pressure to obtain methyl (S)-3-(2-aminoacetamido)-3-(3-(1-methyl-1H-pyrazol-5-yl)phenyl)propanoate (01F) (460 mg).

LC-MS, M/Z (ESI): 317.32 [M+H]⁺.

### Step 6: Synthesis of 1-((2-(trimethylsilyl)ethoxy)methyl)-1H-imidazole-4-nitrile (01H)

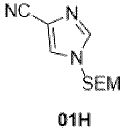

1H-imidazole-4-carbonitrile (6.00 g, 64.5 mmol), acetone (60 mL), and potassium carbonate (17.82 g, 128.9 mmol) were added sequentially to a 250 mL single-necked flask. The mixture was cooled to 0°C in an ice bath. SEMCl (11.82 g, 70.90 mmol) was added, and the mixture was stirred in an ice bath for 20 minutes and then stirred at room temperature for 16 hours. The reaction solution was diluted with ethyl acetate (200 mL) and washed with water (200 mL). The resulting organic phase was dried over anhydrous sodium sulfate, filtered, and concentrated to obtain a crude product. The crude product was purified by column chromatography (petroleum ether/ethyl acetate (V/V)=1:0 to 1:1) to obtain 1-((2-(trimethylsilyl)ethoxy)methyl)-1H-imidazole-4-nitrile (8.00 g, yield 55.6%).

LC-MS, M/Z (ESI): 224.11 [M+H]⁺.

### Step 7: Synthesis of 2-bromo-1-((2-(trimethylsilyl)ethoxy)methyl)-1H-imidazole-4-nitrile (01I)

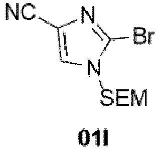

1-((2-(Trimethylsilyl)ethoxy)methyl)-1H-imidazole-4-nitrile (8.00 g, 35.8 mmol), carbon tetrachloride (100 mL), NBS (7.01 g, 39.4 mmol), and AIBN (590 mg, 3.59 mmol) were added sequentially to a 250 mL single-necked flask, and the mixture was stirred at 60°C for 6 hours. After cooling to room temperature, the reaction solution was diluted with ethyl acetate (200 mL), washed sequentially with saturated sodium bicarbonate solution (200 mL) and saturated brine (200 mL), dried over anhydrous sodium sulfate, filtered, and concentrated to obtain a crude product. The crude product was purified by column chromatography (petroleum ether/ethyl acetate (V/V)=1:0 to 1:1) to obtain 2-bromo-1-((2-(trimethylsilyl)ethoxy)methyl)-1H-imidazole-4-nitrile (01I) (4.50 g, yield 41.6%).

LC-MS, M/Z (ESI): 302.02 [M+H]⁺.

### Step 8: Synthesis of 2-((4-methoxybenzyl)amino)-1-((2-(trimethylsilyl)ethoxy)methyl)-1H-imidazole-4-nitrile (01J)

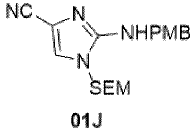

2-Bromo-1-((2-(trimethylsilyl)ethoxy)methyl)-1H-imidazole-4-nitrile (3.00 g, 9.93 mmol), 4-methoxybenzylamine (2.04 g, 14.9 mmol), Pd₂(dba)₃ (454 mg, 0.496 mmol), Xantphos (574 mg, 0.992 mmol), cesium carbonate (9.70 g, 29.8 mmol) and 1,4-dioxane (30 mL) were added sequentially to a 100 mL single-necked flask. The mixture was stirred at 100°C for 16 hours. The reaction solution was filtered and concentrated to obtain a crude product. The crude product was purified by column chromatography (petroleum ether/ethyl acetate (V/V)=1:0 to 1:1) to obtain 2-((4-methoxybenzyl)amino)-1-((2-(trimethylsilyl)ethoxy)methyl)-1H-imidazole-4-nitrile (01J) (850 mg, yield 23.9%).

¹H NMR (400 MHz, DMSO-*d₆*) δ 7.75 (s, 1H), 7.25 (d, *J*=8.6 Hz, 2H), 6.90-6.84 (m, 3H), 5.19 (s, 2H), 4.32 (d, *J*=5.9 Hz, 2H), 3.72 (s, 3H), 3.49-3.45 (m, 2H), 0.82 (d, *J*=8.0 Hz, 2H), -0.04 (s, 9H).

LC-MS, M/Z (ESI): 359.47 [M+H]⁺.

### Step 9: Synthesis of ethyl 4-((4-cyano-1-((2-(trimethylsilyl)ethoxy)methyl)-1H-imidazol-2-yl)(4-methoxybenzyl)amino)butanoate (01K)

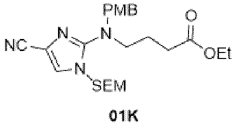

2-((4-Methoxybenzyl)amino)-1-((2-(trimethylsilyl)ethoxy)methyl)-1H-imidazole-4-nitrile (850 mg, 2.37 mmol) and DMF (10 mL) were sequentially added to a 100 mL single-necked flask. Under nitrogen protection, the mixture was cooled to -10°C, and NaH (60%, 142 mg, 3.55 mmol) was added. The mixture was stirred at this temperature for 0.5 hours. Ethyl 4-bromobutyrate (694 mg, 3.56 mmol) was added, and the mixture was stirred at -10°C for 0.5 hours, and then warmed to room temperature and stirred for 2 hours. Saturated ammonium chloride aqueous solution was added, followed by 10 mL of water. The mixture was extracted with ethyl acetate (20 mL×3). The combined organic phases were washed with saturated brine (20 mL), dried over anhydrous sodium sulfate, filtered, and concentrated to obtain a crude product. The crude product was purified by column chromatography (petroleum ether/ethyl acetate (V/V)=1:0 to 1:1) to obtain ethyl 4-((4-cyano-1-((2-(trimethylsilyl)ethoxy)methyl)-1H-imidazol-2-yl)(4-methoxybenzyl)amino)butanoate (200 mg, yield 17.8%).

¹H NMR (400 MHz, DMSO-*d₆*) δ 8.05 (s, 1H), 7.15 (d, *J*=8.6 Hz, 2H), *6.85* (d, *J*=8.7 Hz, 2H), 5.21 (s, 2H), 4.17 (s, 2H), 4.00-3.94 (m, 2H), 3.71 (s, 3H), 3.51-3.47 (m, 2H), 3.04-2.99 (m, 2H), 2.21 (t, *J*=7.4 Hz, 2H), 1.66-1.61 (m, 2H), 1.12 (t, *J*=7.1 Hz, 3H), 0.84-0.78 (m, 2H), -0.06 (s, 9H).

LC-MS, M/Z (ESI): 473.51 [M+H]⁺.

### Step 10: Synthesis of 4-((4-cyano-1-((2-(trimethylsilyl)ethoxy)methyl)-1H-imidazol-2-yl)(4-methoxybenzyl)amino)butanoic acid (01L)

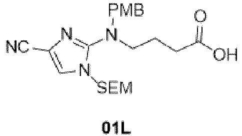

Ethyl 4-((4-cyano-1-((2-(trimethylsilyl)ethoxy)methyl)-1H-imidazol-2-yl)(4-methoxybenzyl)amino)butanoate (200 mg, 0.423 mmol) and THF (9 mL) were added sequentially to a 100 mL single-necked flask. The mixture was dissolved in an aqueous solution (3 mL) of lithium hydroxide monohydrate (23.1 mg, 0.550 mmol) and stirred at room temperature for 6 hours. The reaction solution was concentrated under reduced pressure to remove THF. Water (10 mL) was added, and pH was adjusted to 6 with 1 N hydrochloric acid. The mixture was extracted with ethyl acetate (10 mL×3). The combined organic phases were washed with saturated brine (10 mL), dried over anhydrous sodium sulfate, filtered, and concentrated to obtain 4-((4-cyano-1-((2-(trimethylsilyl)ethoxy)methyl)-1H-imidazol-2-yl)(4-methoxybenzyl)amino)butanoic acid (01L) (100 mg, yield 53.2%).

LC-MS, M/Z (ESI): 445.22 [M+H]⁺.

### Step 11: Synthesis of methyl (S)-3-(2-(4-((4-cyano-1-((2-(trimethylsilyl)ethoxy) methyl)-1H-imidazol-2-yl)(4-methoxybenzyl)amino)butanamido)acetamido)-3-(3-(1-methyl-1H-pyrazol-5-yl)phenyl)propanoate (01M)

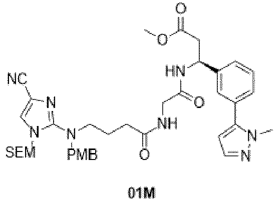

4-((4-Cyano-1-((2-(trimethylsilyl)ethoxy)methyl)-1H-imidazol-2-yl)(4-methoxybenzyl)amino)butanoic acid (100 mg, 0.225 mmol), DMF (10 mL), methyl (5)-3-(2-aminoacetamido)-3-(3-(1-methyl-1H-pyrazol-5-yl)phenyl)propanoate (71.2 mg, 0.225 mmol), TBTU (108 mg, 0.321 mmol), and DIPEA (87.2 mg, 0.675 mmol) were added sequentially to a 100 mL single-necked flask. The mixture was stirred at room temperature for 16 hours under nitrogen protection. Water (10 mL) was added to the reaction solution, and the mixture was extracted with ethyl acetate (20 mL×3). The combined organic phases were washed sequentially with saturated sodium bicarbonate solution (20 mL) and saturated brine (20 mL), dried over anhydrous sodium sulfate, filtered, and concentrated to obtain a crude product. The crude product was purified by column chromatography (petroleum ether/ethyl acetate (V/V)=1:0 to 0:1) to obtain methyl (5)-3-(2-(4-((4-cyano-1-((2-(trimethylsilyl)ethoxy)methyl)-1H-imidazol-2-yl)(4-methoxybenzyl)amino)butanamido)acetamido)-3-(3-(1-methyl-1H-pyrazol-5-yl)phenyl)propanoate (90.0 mg, yield 53.9%).

LC-MS, M/Z (ESI): 743.36 [M+H]⁺.

### Step 12: Synthesis of (S)-3-(2-(4-((4-cyano-1-((2-(trimethylsilyl)ethoxy)methyl) -1H-imidazol-2-yl)(4-methoxybenzyl)amino)butanamido)acetamido)-3-(3-(1-methyl-1H-pyra zol-5-yl)phenyl)propanoic acid (01N)

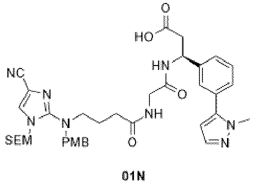

Methyl (*S*)-3-(2-(4-((4-cyano-1-((2-(trimethylsilyl)ethoxy)methyl)-1H-imidazol-2-yl)(4-methoxybenzyl)amino)butanamido)acetamido)-3-(3-(1-methyl-1H-pyrazol-5-yl)phenyl) propanoate (90.0 mg, 0.121 mmol), THF (6 mL), and an aqueous solution (2 mL) of lithium hydroxide monohydrate (6.6 mg, 0.16 mmol) were added sequentially to a 100 mL single-necked flask. The mixture was stirred at room temperature for 6 hours. The reaction solution was concentrated under reduced pressure to remove THF. Water (5 mL) was added, and pH was adjusted to 6 with 1 N hydrochloric acid. The mixture was extracted with ethyl acetate (5 mL×3). The combined organic phases were washed with saturated brine (5 mL), dried over anhydrous sodium sulfate, filtered, and concentrated to obtain (*S*)-3-(2-(4-((4-cyano-1-((2-(trimethylsilyl)ethoxy)methyl)-1H-imidazol-2-yl)(4-methoxybenzyl)amino)butanamido) acetamido)-3-(3-(1-methyl-1H-pyrazol-5-yl)phenyl)propanoic acid (70.0 mg, yield 79.3%).

LC-MS, M/Z (ESI): 729.35 [M+H]⁺.

### Step 13: Synthesis of (S)-3-(2-(4-((4-cyano-1H-imidazol-2-yl)amino)butanamido)acetamido)-3-(3-(1-methyl-1H-pyrazol-5-yl)phenyl)propanoic acid (I-1)

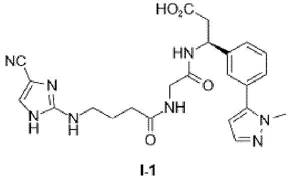

(*S*)-3-(2-(4-((4-cyano-1-((2-(trimethylsilyl)ethoxy)methyl)-1H-imidazol-2-yl)(4-methoxybenzyl)amino)butanamido)acetamido)-3-(3-(1-methyl-1H-pyrazol-5-yl)phenyl) propanoic acid (70.0 mg, 96.0 µmol), dichloromethane (8 mL), and trifluoroacetic acid (438 mg, 3.84 mmol) were sequentially added to a 100 mL single-necked flask. The mixture was stirred at room temperature for 6 hours. The reaction solution was concentrated under reduced pressure to obtain a crude product. The crude product was purified by preparative reversed-phase high-performance liquid chromatography (column: YMC-Triart Prep C18 (30 mm×40 cm, 7 µm); mobile phase: A=0.1% aqueous ammonia, B=acetonitrile; gradient: 30% to 100%) to obtain (*S*)-3-(2-(4-((4-cyano-1H-imidazol-2-yl)amino)butylamido)acetamido)-3-(3-(1-methyl-1H-pyrazol-5-yl)phenyl)propanoic acid (I-1) (11 mg, yield 24%).

¹H NMR (400 MHz, DMSO-*d₆*) δ 8.44 (d, J=8.3 Hz, 1H), 8.09 (t, J=5.8 Hz, 1H), 7.72 (s, 1H), 7.45-7.42 (m, 2H), 7.41 (d, J=7.4 Hz, 1H), 7.39-7.33 (m, 2H), 6.38 (d, J=1.8 Hz, 1H), 5.23 (q, J=7.4 Hz, 1H), 3.82 (s, 3H), 3.69 (d, J=5.8 Hz, 2H), 3.12 (t, J=7.0 Hz, 2H), 2.73 (d, *J=7.2* Hz, 2H), *2.16* (t, *J=7.4* Hz, 2H), 1.73-1.66 (m, 2H).

LC-MS, M/Z (ESI): 479.3 [M+H]⁺.

### Example 2: Preparation of Compound I-2

The synthetic route is as follows:

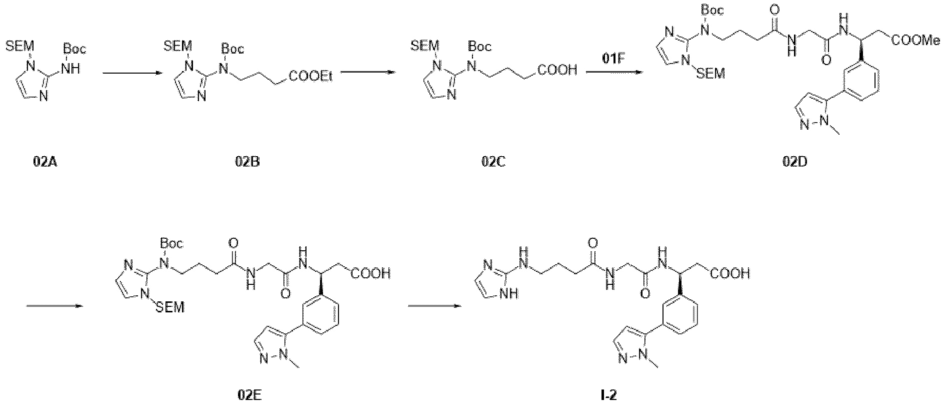

### Step 1 : Synthesis of ethyl 4-((tert-butoxycarbonyl)(1-((2-(trimethylsilyl)ethoxy)methyl)-1H-imidazol-2-yl)amino)butanoate (02B)

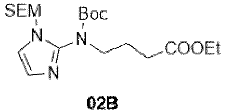

Tert-butyl (1-((2-(trimethylsilyl)ethoxy)methyl)-1H-imidazol-2-yl)carbamate (2.80 g, 15.3 mmol) and DMF (30 mL) were added to a 100 mL single-necked flask. Under nitrogen protection, the mixture was cooled to 0°C in an ice bath. NaH (1.07 g, 26.8 mmol, 60%) was added, and the mixture was stirred at 0°C for 0.5 hours. Then, ethyl 4-bromobutyrate (7.47 g, 38.3 mmol) and potassium iodide (2.54 g, 15.3 mmol) were added. The mixture was stirred at 0°C for 1 hour, and then warmed to room temperature and stirred for 16 hours. The reaction was quenched with saturated ammonium chloride, and water (30 mL) was added. The mixture was extracted with ethyl acetate (60 mL×3). The combined organic phases were washed with saturated brine (100 mL), dried over anhydrous sodium sulfate, filtered, and concentrated to obtain a crude product. The crude product was purified by column chromatography (petroleum ether/ethyl acetate (V/V)=1:0 to 2:3) to obtain ethyl 4-((tert-butoxycarbonyl)(1-((2-(trimethylsilyl)ethoxy)methyl)-1H-imidazol-2-yl)amino)butanoate (02B) as a colorless oil (2.80 g, yield 61.5%).

LC-MS, M/Z (ESI): 428.35 [M+H]⁺.

### Step 2: Synthesis of 4-((tert-butoxycarbonyl)(1-((2-(trimethylsilyl)ethoxy)methyl)-1H-imidazol-2-yl)amino)butanoic acid (02C)

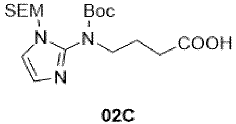

Ethyl 4-((tert-butoxycarbonyl)(1-((2-(trimethylsilyl)ethoxy)methyl)-1H-imidazol-2-yl)amino)butanoate (215 mg, 0.503 mmol), THF/H₂O (4 mL, v:v=1:1), and lithium hydroxide monohydrate (63 mg, 1.5 mmol) were added sequentially to a 100 mL single-necked flask. The mixture was add stirred at room temperature for 3 hours. After the reaction was completed, 1 N HCl (2 mL) was added, and the mixture was extracted with ethyl acetate (20 mL). The combined organic phases were dried over anhydrous sodium sulfate, filtered, and concentrated to obtain 4-((tert-butoxycarbonyl)(1-((2-(trimethylsilyl)ethoxy)methyl)-1H-imidazol-2-yl)amino)butanoic acid (02C) (200 mg, yield 99.6%).

LC-MS, M/Z (ESI): 400.35 [M+H]⁺.

### Step 3: Synthesis of methyl (3S)-3-((N-(4-((tert-butoxycarbonyl)(1-((2-(trimethylsilyl)ethoxy)methyl)-1H-imidazol-2-yl)amino)butanoyl)glycyl)amino)-3-(3-(1-methyl-1H-pyrazol-5-yl)phenyl)propanoate (02D)

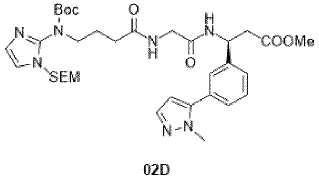

4-((Tert-butoxycarbonyl)(1-((2-(trimethylsilyl)ethoxy)methyl)-1H-imidazol-2-yl)amino)butanoic acid (02C) (200 mg, 0.501 mmol), DMF (10 mL), methyl (S)-3-(2-aminoacetamido)-3-(3-(1-methyl-1H-pyrazol-5-yl)phenyl)propanoate (01F) (158 mg, 0.499 mmol), TBTU (176 mg, 0.548 mmol) and DIPEA (194 mg, 1.50 mmol) were added sequentially to a 100 mL single-necked flask. The mixture was stirred at room temperature for 16 hours. Water (10 mL) was added, and the mixture was extracted with ethyl acetate (20 mL×3). The combined organic phases were washed sequentially with saturated sodium bicarbonate solution (20 mL) and saturated brine (20 mL), dried over anhydrous sodium sulfate, filtered, and concentrated to obtain a crude product. The crude product was purified by column chromatography (petroleum ether/ethyl acetate (V/V)=1:0 to 0:1) to obtain methyl (3S)-3-((N-(4-((tert-butoxycarbonyl)(1-((2-(trimethylsilyl)ethoxy)methyl)-1H-imidazol-2-yl)amino) butanoyl)glycyl)amino)-3-(3-(1-methyl-1H-pyrazol-5-yl)phenyl)propanoate (02D) (230 mg, yield 66.0%).

LC-MS, M/Z (ESI): 698.44 [M+H]⁺.

### Step 4: Synthesis of (3S)-3-((N-(4-((tert-butoxycarbonyl)(1-((2-(trimethylsilyl)ethoxy)methyl)-1H-imidazol-2-yl)amino)butanoyl)glycyl)amino)-3-(3-(1-methyl-1H-pyrazol-5-yl)phenyl)propanoic acid (02E)

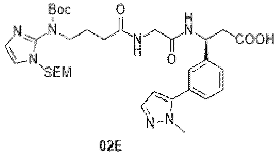

Methyl (3*S*)-3-((*N*-(4-((tert-butoxycarbonyl)(1-((2-(trimethylsilyl)ethoxy)methyl)-1H-imidazol-2-yl)amino)butanoyl)glycyl)amino)-3-(3-(1-methyl-1H-pyrazol-5-yl)phenyl) propanoate (230 mg, 0.330 mmol), THF/H₂O (4 mL, v:v=1:1), and lithium hydroxide monohydrate (42 mg, 1.0 mmol) were added sequentially to a 100 mL single-necked flask. The mixture was stirred at room temperature for 3 hours. 1 N HCl (1 mL) was added, and the mixture was extracted with ethyl acetate (20 mL). The combined organic phases were dried over anhydrous sodium sulfate, filtered, and concentrated to obtain (3*S*)-3-((*N*-(4-((tert-butoxycarbonyl)(1-((2-(trimethylsilyl)ethoxy)methyl)-1H-imidazol-2-yl)amino)butanoyl)glycyl)amino)-3-(3-(1-methyl-1H-pyrazol-5-yl)phenyl)propanoic acid (02E) (225 mg, yield 99.8%).

LC-MS, M/Z (ESI): 684.45 [M+H]⁺.

### Step 5: Synthesis of (3S)-3-((N-(4-((1H-imidazol-2-yl)amino)butanoyl)glycyl)amino)-3-(3-(1-methyl-1H-pyrazol-5-yl)phenyl)propanoic acid (I-2)

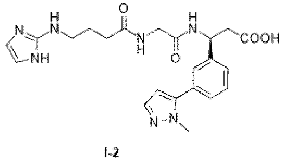

(3*S*)-3-((*N*-(4-((tert-butoxycarbonyl)(1-((2-(trimethylsilyl)ethoxy)methyl)-1H-imidazol-2-yl)amino)butanoyl)glycyl)amino)-3-(3-(1-methyl-1H-pyrazol-5-yl)phenyl) propanoic acid (225 mg, 0.329 mmol), DCM (5 mL) and TFA (2 mL) were added sequentially to a 100 mL single-necked flask. The mixture was stirred at room temperature for 2 days. The reaction solution was concentrated under reduced pressure to obtain a crude product. The crude product was purified by preparative reversed-phase high-performance liquid chromatography (column: YMC-Triart Prep C18 (30 mm×40 cm, 7 µm); mobile phase: A=0.1% aqueous ammonia, B=acetonitrile; gradient: 30% to 100%) to obtain (3*S*)-3-((*N*-(4-((1H-imidazol-2-yl)amino)butanoyl)glycyl)amino)-3-(3-(1-methyl-1H-pyrazol-5-yl)phenyl)propanoic acid (I-2) (73 mg, yield 49%).

¹H NMR (400 MHz, DMSO-*d₆*) δ 9.47 (d, *J*=8.0 Hz, 1H), 8.69 (s, 1H), 8.46 (t, *J*=6.0 Hz, 1H), 7.45-7.39 (m, 2H), 7.39-7.30 (m, 3H), 6.74 (s, 2H), 6.36 (d, *J*=2.0 Hz, 1H), 5.04 (m, 1H), 3.80 (m, 5H), 3.55 (m, 2H), 2.65-2.52 (m, 2H), 2.37 (t, *J*=6.8 Hz, 2H), 1.85 (m, 2H).

LC-MS, M/Z (ESI): 454.31 [M+H]⁺.

### Example 3: Preparation of Compound I-3

The synthetic route is as follows:

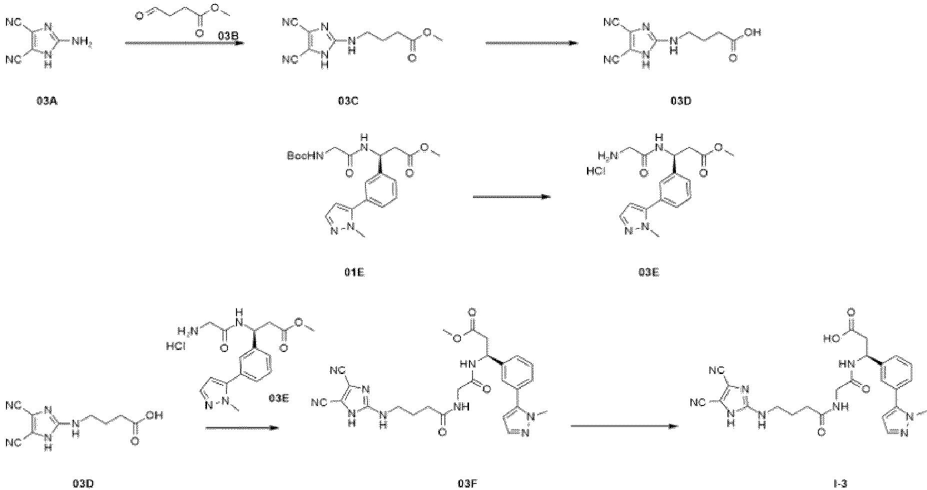

### Step 1: Synthesis of methyl 4-((4,5-dicyano-1H-imidazol-2-yl)amino)butanoate (03C)

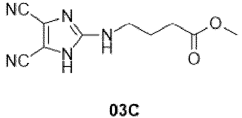

2-Amino-4,5-imidazole dinitrile (150 mg, 1.13 mmol), methanol (5 mL), methyl 4-oxobutyrate (131 mg, 1.13 mmol), and acetic acid (135 mg, 2.25 mmol) were added sequentially to a 100 mL single-necked flask. The mixture was stirred at room temperature for 16 hours under nitrogen protection. Sodium cyanoborohydride (212 mg, 3.37 mmol) was added, and the mixture was stirred at room temperature for 3 hours. The reaction was quenched with saturated ammonium chloride solution. Water (5 mL) was added, and the reaction solution was concentrated under reduced pressure to remove the organic solvent. The mixture was extracted with ethyl acetate (10 mL×3). The combined organic phases were washed with saturated brine (10 mL), dried over anhydrous sodium sulfate, filtered, and concentrated to obtain a crude product. The crude product was purified by column chromatography (petroleum ether/ethyl acetate (V/V)=1:0 to 0:1) to obtain methyl 4-((4,5-dicyano-1H-imidazol-2-yl)amino)butanoate (03B) (100 g, yield 38.0%).

¹H NMR (400 MHz, DMSO-*d₆*) δ 12.47 (s, 1H), 7.29 (t, *J*=5.5 Hz, 1H), 3.55 (s, 3H), 3.12 (q, *J*=6.6 Hz, 2H), 2.32 (t, *J*=7.4 Hz, 2H), 1.75-1.68 (m, 2H).

LC-MS, M/Z (ESI): 234.23[M+H]⁺.

### Step 2: Synthesis of 4-((4,5-dicyano-1H-imidazol-2-yl)amino)butanoic acid (03D)

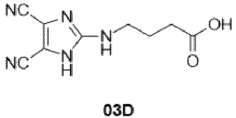

Methyl 4-((4,5-dicyano-1H-imidazol-2-yl)amino)butanoate (50 mg, 0.21 mmol), tetrahydrofuran (5 mL), and a solution of lithium hydroxide monohydrate (11.7 mg, 0.279 mmol) in water (0.5 mL) were added sequentially to a 100 mL single-necked flask. The mixture was stirred at room temperature for 16 hours. The reaction solution was concentrated under reduced pressure to remove the organic solvent. The pH was adjusted to 7 with 1 N hydrochloric acid, and the mixture was concentrated under reduced pressure to obtain the product 4-((4,5-dicyano-1H-imidazol-2-yl)amino)butanoic acid (03D), which was directly used in the next step of reaction.

LC-MS, M/Z (ESI): 220.21[M+H]⁺.

### Step 3: Synthesis of methyl (S)-3-(2-aminoacetamido)-3-(3-(1-methyl-1H-pyrazol-5-yl)phenyl)propanoate hydrochloride (03E)

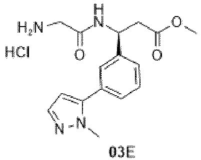

Methyl (S)-3-(2-((tert-butoxycarbonyl)amino)acetamido)-3-(3-(1-methyl-1H-pyrazol-5-yl)phenyl)propanoate (3.2 g, 7.7 mmol), methanol (20 mL), and a solution of hydrogen chloride in 1,4-dioxane (4 M, 9.61 mL) were added sequentially to a 100 mL single-necked flask. The mixture was stirred at room temperature for 2 hours. The reaction solution was concentrated under reduced pressure to obtain methyl (S)-3-(2-aminoacetamido)-3-(3-(1-methyl-1H-pyrazol-5-yl)phenyl)propanoate hydrochloride (03E) (2.6 g, yield 96%).

LC-MS, M/Z (ESI): 317.32 [M+H]⁺.

### Step 4: Synthesis of methyl (S)-3-(2-(4-((4,5-dicyano-1H-imidazol-2-yl)amino)butanamido)acetamido)-3-(3-(1-methyl-1H-pyrazol-5-yl)phenyl)propanoate (03F)

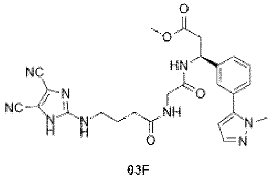

4-((4,5-Dicyano-1H-imidazol-2-yl)amino)butanoic acid obtained in step 2, DMF (10 mL), methyl (S)-3-(2-aminoacetamido)-3-(3-(1-methyl-1H-pyrazol-5-yl)phenyl)propanoate hydrochloride (91 mg, 0.26 mmol), TBTU (103 mg, 0.321 mmol), and DIPEA (111 mg, 0.859 mmol) were added sequentially to a 100 mL single-necked flask. The mixture was stirred at room temperature for 16 hours under nitrogen protection. Water (10 mL) was added, and the mixture was extracted with ethyl acetate (20 mL×3). The combined organic phases were washed with saturated brine (20 mL), dried over anhydrous sodium sulfate, filtered, and concentrated to obtain a crude product. The crude product was purified by column chromatography (dichloromethane/methanol (V/V)=1:0 to 9:1) to obtain methyl (S)-3-(2-(4-((4,5-dicyano-1H-imidazol-2-yl)amino)butanamido)acetamido)-3-(3-(1-methyl-1H-pyrazol-5-yl)phenyl)propanoate (03F) (35 mg, yield 32% over two steps).

LC-MS, M/Z (ESI): 518.26 [M+H]⁺.

### Step 5: Synthesis of (S)-3-(2-(4-((4,5-dicyano-1H-imidazol-2-yl)amino)butanamido)acetamido)-3-(3-(1-methyl-1H-pyrazol-5-yl)phenyl)propanoic acid (I-3)

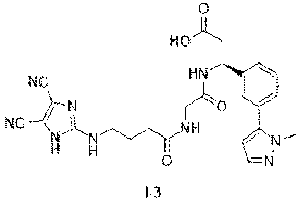

Methyl (S)-3-(2-(4-((4,5-dicyano-1H-imidazol-2-yl)amino)butanamido)acetamido)-3-(3-(1-methyl-1H-pyrazol-5-yl)phenyl)propanoate (35 mg, 0.068 mmol), tetrahydrofuran (5 mL), and a solution of lithium hydroxide monohydrate (3.7 mg, 0.088 mmol) in water (0.5 mL) were added sequentially to a 100 mL single-necked flask. The mixture was stirred at room temperature for 3 hours. The reaction solution was concentrated under reduced pressure to remove the organic solvent. The pH was adjusted to 6 with 1 N hydrochloric acid, and the mixture was concentrated under reduced pressure to obtain a crude product. The crude product was purified by reversed-phase high-performance liquid chromatography (column: YMC-Triart Prep C18 (30 mm×40 cm, 7 µm); solvent: A=0.1% formic acid, B=acetonitrile; gradient: 42% to 90%) to obtain (*S*)-3-(2-(4-((4,5-dicyano-1H-imidazol-2-yl)amino)butylamido)acetamido)-3-(3-(1-methyl-1H-pyrazole-5)-yl)phenyl) propanoic acid (I-3) (3.2 mg, yield 9.4%).

¹H NMR (400 MHz, DMSO-*d₆*) δ 8.54 (d, *J*=8.3 Hz, 1H), 8.14-8.11 (m, 1H), 7.44-7.32 (m, 5H), 6.37 (d, *J*=1.7 Hz, 1H), 5.18 (q, *J*=7.2 Hz, 1H), 3.81 (s, 3H), 3.67-3.63 (m, 2H), 3.08-3.05 (m, 2H), 2.69 (d, *J*=6.8 Hz, 2H), 2.16 (t, *J*=7.4 Hz, 2H), 1.71-1.64 (m, 2H).

LC-MS, M/Z (ESI): 504.41 [M+H]⁺.

### Example 4: Preparation of Compound I-4

The synthetic route is as follows:

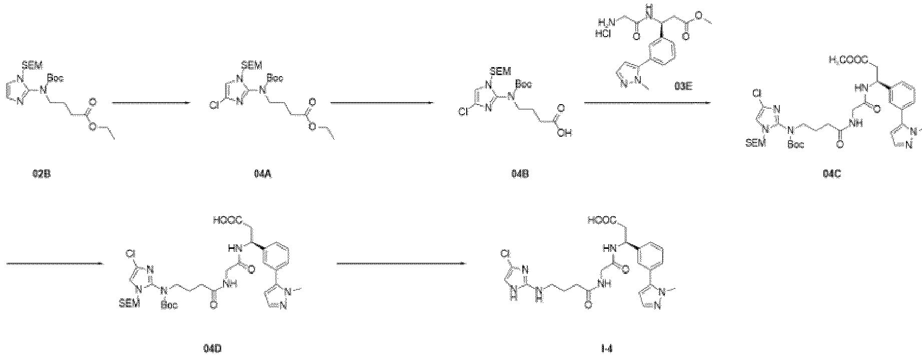

### Step 1: Synthesis of ethyl 4-((tert-butoxycarbonyl)(4-chloro-1-((2-(trimethylsilyl)ethoxy)methyl)-1H-imidazol-2-yl)amino)butanoate (04A)

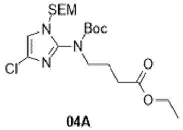

Ethyl 4-((tert-butoxycarbonyl)(1-((2-(trimethylsilyl)ethoxy)methyl)-1H-imidazol-2-yl)amino)butanoate (400 mg, 0.935 mmol), DMF (10 mL), and *N*-chlorosuccinimide (125 mg, 0.936 mmol) were added sequentially to a 100 mL single-necked flask. The mixture was stirred at room temperature for 2 hours. The reaction solution was heated to 40°C and stirred for 16 hours. Water (10 mL) was added, and the mixture was extracted with ethyl acetate (20 mL×3). The combined organic phases were washed with saturated brine (20 mL), dried over anhydrous sodium sulfate, filtered, and concentrated to obtain a crude product. The crude product was purified by column chromatography (petroleum ether/ethyl acetate (V/V)=1:0 to 1:1) to obtain ethyl 4-((tert-butoxycarbonyl)(4-chloro-1-((2-(trimethylsilyl)ethoxy)methyl)-1H-imidazol-2-yl)amino)butanoate (04A) (280 mg, yield 64.8%).

LC-MS, M/Z (ESI): 462.3, 464.3 [M+H]⁺.

### Step 2: Synthesis of 4-((tert-butoxycarbonyl)(4-chloro-1-((2-(trimethylsilyl)ethoxy)methyl)-1H-imidazol-2-yl)amino)butanoic acid (04B)

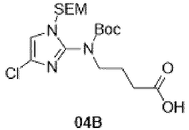

Ethyl 4-((tert-butoxycarbonyl)(4-chloro-1-((2-(trimethylsilyl)ethoxy)methyl)-1H-imidazol-2-yl)amino)butanoate (70 mg, 0.15 mmol), THF (5 mL), and a solution of lithium hydroxide monohydrate (19 mg, 0.45 mmol)) in water (0.5 mL) were added sequentially to a 100 mL single-necked flask. The mixture was stirred at room temperature for 16 hours. The reaction solution was concentrated under reduced pressure to remove the organic solvent. Water (5 mL) was added, and the pH was adjusted to 6 with 1 N hydrochloric acid. The mixture was extracted with ethyl acetate (10 mL×3). The combined organic phases were washed with saturated brine (10 mL), dried over anhydrous sodium sulfate, filtered, and concentrated to obtain 4-((tert-butoxycarbonyl)(4-chloro-1-((2-(trimethylsilyl)ethoxy)methyl)-1H-imidazol-2-yl)amino)butanoic acid (04B) (66 mg, yield 100%).

LC-MS, M/Z (ESI): 434.3, 436.3 [M+H]⁺.

### Step 3: Synthesis of methyl (S)-5-(4-chloro-1-((2-(trimethylsilyl)ethoxy)methyl)-1H-imidazol-2-yl)-2,2-dimethyl-14-(3-(1-methyl)-1H-pyrazol-5-yl)phenyl)-4,9,12-trioxo-3-oxa-5,10,13-triazahexadecan-16-oleate (04C)

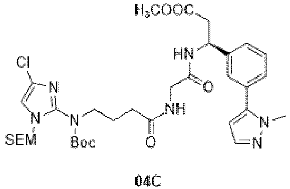

4-((Tert-butoxycarbonyl)(4-chloro-1-((2-(trimethylsilyl)ethoxy)methyl)-1H-imidazol-2-yl)amino)butanoic acid (66 mg, 0.15 mmol), DMF (10 mL), methyl (*S*)-3-(2-aminoacetamido)-3-(3-(1-methyl-1H-pyrazol-5-yl)phenyl)propanoate hydrochloride (54 mg, 0.15 mmol), TBTU (73 mg, 0.23 mmol), and DIPEA (79 mg, 0.61 mmol) were added sequentially to a 100 mL single-necked flask. The mixture was stirred at room temperature for 16 hours under nitrogen protection. Water (10 mL) was added, and the mixture was extracted with ethyl acetate (20 mL×3). The combined organic phases were washed sequentially with saturated sodium bicarbonate solution (20 mL) and saturated brine (20 mL), dried over anhydrous sodium sulfate, filtered, and concentrated to obtain a crude product. The crude product was purified by column chromatography (dichloromethane/methanol (V/V)=1:0 to 9:1) to obtain methyl (*S*)-5-(4-chloro-1-((2-(trimethylsilyl)ethoxy)methyl)-1H-imidazol-2-yl)-2,2-dimethyl-14-(3-(1-methyl)-1H-pyrazol-5-yl)phenyl)-4,9,12-trioxo-3-oxa-5,10,13-triazahexadecan-16-oleate (04C) (60 mg, yield 54%).

LC-MS, M/Z (ESI): 732.3, 734.3 [M+H]⁺.

### Step 4: Synthesis of (S)-5-(4-chloro-1-((2-(trimethylsilyl)ethoxy)methyl)-1H-imidazol-2-yl)-2,2-dimethyl-14-(3-(1-methyl-1H-pyrazol-5-yl)phenyl)-4,9,12-trioxo-3-oxa-5,10,13-triazahexadecan-16-oleic acid (04D)

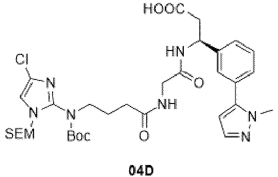

Methyl (*S*)-5-(4-chloro-1-((2-(trimethylsilyl)ethoxy)methyl)-1H-imidazol-2-yl)-2,2-dimethyl-14-(3-(1-methyl)-1H-pyrazol-5-yl)phenyl)-4,9,12-trioxo-3-oxa-5,10,13-triazahexadecan-16-oleate (60 mg, 0.082 mmol), THF (6 mL), and a solution of lithium hydroxide monohydrate (10 mg, 0.24 mmol) in water (1 mL) were added sequentially to a 100 mL single-necked flask. The mixture was stirred at room temperature for 6 hours. The reaction solution was concentrated under reduced pressure to remove the organic solvent. Water (5 mL) was added, and pH was adjusted to 6 with 1 N hydrochloric acid. The mixture was extracted with ethyl acetate (5 mL×3). The combined organic phases were washed with saturated brine (5 mL), dried over anhydrous sodium sulfate, filtered, and concentrated to obtain (*S*)-5-(4-chloro-1-((2-(trimethylsilyl)ethoxy)methyl)-1H-imidazol-2-yl)-2,2-dimethyl-14-(3-(1-methyl-1H-pyrazol-5-yl)phenyl)-4,9,12-trioxo-3-oxa-5,10,13-triazahexadecan-16-oleic acid (04D) (45 mg, yield 76%).

LC-MS, M/Z (ESI): 718.4, 720.4 [M+H]⁺.

### Step 5: Synthesis of (S)-3-(2-(4-((4-chloro-1H-imidazol-2-yl)amino)butylamido)acetamido)-3-(3-(1-methyl-1H-pyrazol-5-yl))phenyl)propanoic acid (I-4)

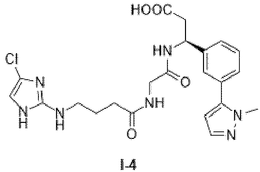

(*S*)-5-(4-Chloro-1-((2-(trimethylsilyl)ethoxy)methyl)-1H-imidazol-2-yl)-2,2-dimethyl-14-(3-(1-methyl-1H-pyrazol-5-yl)phenyl)-4,9,12-trioxo-3-oxa-5,10,13-triazahexadecan-16-oleic acid (45 mg, 0.063 mmol), dichloromethane (4 mL), and trifluoroacetic acid (1 mL) were added sequentially to a 100 mL single-necked flask. The mixture was stirred at room temperature for 16 hours. The reaction solution was concentrated under reduced pressure to obtain a crude product. The crude product was purified by preparative reversed-phase high-performance liquid chromatography (column: YMC-Triart Prep C18 (30 mm×40 cm, 7 µm); solvent: A=0.1% formic acid, B=acetonitrile; gradient: 42% to 98%) to obtain (*S*)-3-(2-(4-((4-chloro-1H-imidazol-2-yl)amino)butylamido)acetamido)-3-(3-(1-methyl-1H-pyrazol-5-yl))phenyl)propanoic acid (I-4) (3.4 mg, yield 11%).

¹H NMR (400 MHz, DMSO-*d₆*) δ 10.05 (s, *J*=8.3 Hz, 1H) , 8.44 (d, *J*=8.3 Hz, 1H), 8.11 (t, *J*=5.7 Hz, 1H), 7.50-7.34 (m, 5H), 6.54 (s, 1H), 6.44-6.37 (m, 1H), 5.97 (s, 1H), 5.25 (q, *J*=7.5 Hz, 1H), 3.84 (s, 3H), 3.70 (d, *J*=5.7 Hz, 2H), 3.04 (q, *J*=6.3 Hz, 2H), 2.75 (d, *J*=7.2 Hz, 2H), 2.17 (t, *J*=7.4 Hz, 2H), 1.72-1.65 (m, 2H).

LC-MS, M/Z (ESI): 488.2, 490.2 [M+H]⁺.

### Example 5: Preparation of Compound I-5

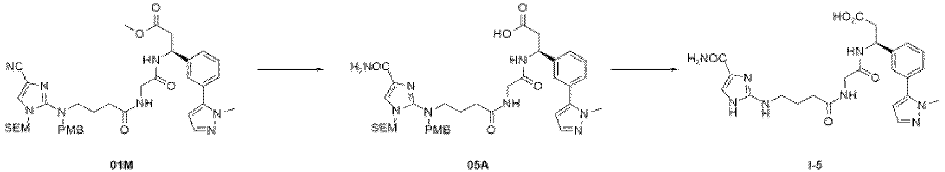

### Step 1: Synthesis of (S)-3-(2-(4-((4-carbamoyl-1-((2-(trimethylsilyl)ethoxy)met hyl)-1H-imidazol-2-yl)(4-methoxybenzyl)amino)butanamido)acetamido)-3-(3-(1-methyl-1H-pyrazol-5-yl)phenyl)propanoic acid (05A)

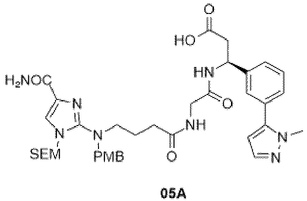

Methyl (*S*)-3-(2-(4-((4-cyano-1-((2-(trimethylsilyl)ethoxy)methyl)-1H-imidazol-2-yl)(4-methoxybenzyl)amino)butanamido)acetamido)-3-(3-(1-methyl-1H-pyrazol-5-yl)phenyl) propanoate (300 mg, 0.404 mmol), THF (3 mL), and an aqueous solution (3 mL) of lithium hydroxide monohydrate (84.8 mg, 2.02 mmol) were added sequentially to a 100 mL single-necked flask. The mixture was stirred at room temperature for 6 hours. The reaction solution was concentrated under reduced pressure to remove THF. Water (5 mL) was added, and pH was adjusted to 6 with 1 N hydrochloric acid. The mixture was extracted with ethyl acetate (5 mL×3). The combined organic phases were washed with saturated brine (5 mL), dried over anhydrous sodium sulfate, filtered, and concentrated to obtain (*S*)-3-(2-(4-((4-carbamoyl-1-((2-(trimethylsilyl)ethoxy)methyl)-1H-imidazol-2-yl)(4-methoxybenzyl)amino)butanamido) acetamido)-3-(3-(1-methyl-1H-pyrazol-5-yl)phenyl)propanoic acid (180 mg, yield 59.7%).

LC-MS, M/Z (ESI): 747.36 [M+H]⁺.

### Step 2: Synthesis of (S)-3-(2-(4-((4-carbamoyl-1H-imidazol-2-yl)amino)butanamido)acetamido)-3-(3-(1-methyl-1H-pyrazol-5-yl)phenyl)propanoic acid (I-5)

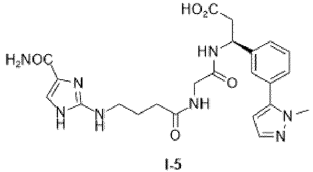

(*S*)-3-(2-(4-((4-Carbamoyl-1-((2-(trimethylsilyl)ethoxy)methyl)-1H-imidazol-2-yl)(4-methoxybenzyl)amino)butanamido)acetamido)-3-(3-(1-methyl-1H-pyrazol-5-yl)phenyl) propanoic acid (180 mg, 0.241 mol), dichloromethane (2 mL), trifluoroacetic acid (2 mL), anisole (199 mg, 1.84 mmol), and trifluoromethanesulfonic acid (170 mg, 1.13 mmol) were added sequentially to a 100 mL single-necked flask. The mixture was stirred at room temperature for 30 minutes. The reaction solution was concentrated under reduced pressure to obtain a crude product. The crude product was prepared by preparative reversed-phase high-performance liquid chromatography (column: YMC-Triart Prep C18 (30 mm×40 cm, 7 µm); mobile phase: A=0.1% aqueous ammonia, B=acetonitrile; gradient: 30% to 100%) to obtain (*S*)-3-(2-(4-((4-carbamoyl-1H-imidazol-2-yl)amino)butanamido)acetamido)-3-(3-(1-methyl-1H-pyrazol-5-yl)phenyl)propanoic acid (I-5) (65 mg, yield 54%).

¹H NMR (400 MHz, DMSO-*d₆*) δ 8.62 (d, *J*=8.4 Hz, 1H), 8.19 (q, *J*=_{6.4} Hz, 1H), 7.47-7.43 (m, 2H), 7.41 (t, *J*=6.8 Hz, 1H), 7.39-7.35 (m, 2H), 7.13 (s, 1H), 6.39 (d, *J*=2.0 Hz, 1H), 6.25 (s, 1H), 5.21 (q, *J*=7.2 Hz, 1H), 3.83 (s, 3H), 3.76-3.62 (m, 2H), 3.13 (d, *J*=6.0 Hz, 2H), 2.72 (d, *J*=7.2 Hz, 2H), 2.21 (t, *J*=7.2 Hz, 2H), 1.76-1.68 (m, 2H).

LC-MS, M/Z (ESI): 497.22 [M+H]⁺.

### Example 6: Preparation of Compounds II-1A & II-1B

The synthetic route is as follows:

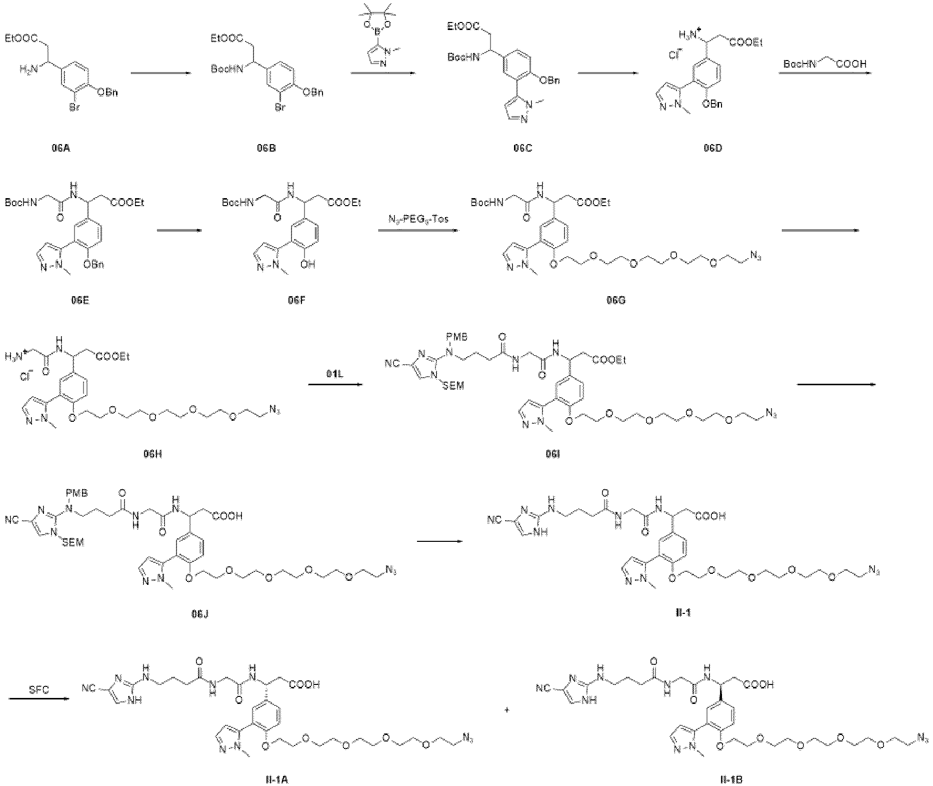

### Step 1: Synthesis of ethyl 3-(4-(benzyloxy)-3-bromophenyl)-3-((tert-butoxycarbonyl)amino)propanoate (06B)

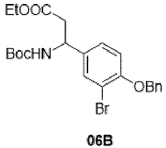

Ethyl 3-amino-3-(4-(benzyloxy)-3-bromophenyl)propanoate (14.00 g, 37.01 mmol), dichloromethane (150 mL), and di-tert-butyl dicarbonate (12.12 g, 55.53 mmol) were added sequentially to a 500 mL single-necked flask. The mixture was stirred at room temperature for 3 hours. The reaction solution was washed with water (150 mL). The combined organic phases were dried over anhydrous sodium sulfate, filtered, and concentrated to obtain a crude product. The crude product was purified by column chromatography (petroleum ether/ethyl acetate (V/V)=1:0 to 1:1) to obtain ethyl 3-(4-(benzyloxy)-3-bromophenyl)-3-((tert-butoxycarbonyl)amino)propanoate (06B) (14.00 g, yield 79.07%).

LC-MS, M/Z (ESI): 478.12, 480.11 [M+H]⁺.

### Step 2: Synthesis of ethyl 3-(4-(benzyloxy)-3-(1-methyl-1H-pyrazol-5-yl)phenyl)-3-((tert-butoxycarbonyl)amino)propanoate (06C)

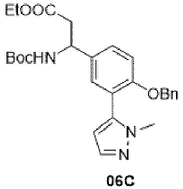

Ethyl 3-(4-(benzyloxy)-3-bromophenyl)-3-((tert-butoxycarbonyl)amino)propanoate (14.00 g, 29.27 mmol), 1-methyl-1H-pyrazole-5-boronic acid pinacol ester (12.18 g, 58.54 mmol), Pd₂(dba)₃ (1.07 g, 1.17 mmol), tricyclohexylphosphine (657 mg, 2.34 mmol), dipotassium hydrogen phosphate (10.20 g, 58.56 mmol), 1,4-dioxane (100 mL), and water (30 mL) were added sequentially to a 250 mL single-necked flask. The mixture was stirred at 100°C for 16 hours. After cooling to room temperature, the mixture was filtered and the filtrate was concentrated to obtain a crude product. The crude product was purified by column chromatography (petroleum ether/ethyl acetate (V/V)=1:0 to 1:1) to obtain ethyl 3-(4-(benzyloxy)-3-(1-methyl-1H-pyrazol-5-yl)phenyl)-3-((tert-butoxycarbonyl)amino)propanoate (06C) (14.00 g, yield 99.75%).

LC-MS, M/Z (ESI): 480.24 [M+H]⁺.

### Step 3: Synthesis of ethyl 3-amino-3-(4-(benzyloxy)-3-(1-methyl-1H-pyrazol-5-yl)phenyl)propanoate hydrochloride (06D)

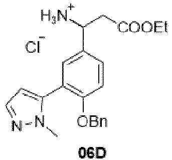

Ethyl 3-(4-(benzyloxy)-3-(1-methyl-1H-pyrazol-5-yl)phenyl)-3-((tert-butoxycarbonyl)amino)propanoate (8.00 g, 16.7 mmol) and a solution of hydrogen chloride in 1,4-dioxane (4 M, 41.7 mL) were added sequentially to a 250 mL single-necked flask. The mixture was stirred at room temperature for 2 hours. The reaction solution was concentrated under reduced pressure to obtain the product ethyl 3-amino-3-(4-(benzyloxy)-3-(1-methyl-1H-pyrazol-5-yl)phenyl)propanoate hydrochloride (06D) (6.00 g, yield 86.5%).

LC-MS, M/Z (ESI): 380.19 [M+H]⁺.

### Step 4: Synthesis of ethyl 3-(4-(benzyloxy)-3-(1-methyl-1H-pyrazol-5-yl)phenyl)-3-((N-(tert-butoxycarbonyl)glycyl)amino)propanoate (06E)

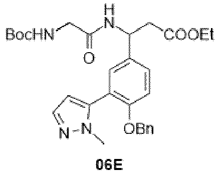

At room temperature, compound ethyl 3-amino-3-(4-(benzyloxy)-3-(1-methyl-1H-pyrazol-5-yl)phenyl)propanoate hydrochloride (1.0 g, 2.4 mmol) was dissolved in anhydrous DMF (10 mL), and then TBTU (0.93 g, 2.9 mmol) was added. DIPEA (1.02 g, 7.89 mmol) was added at 0°C, and the mixture was warmed to room temperature and stirred for half an hour. Then, N-(tert-butoxycarbonyl)glycine (0.46 g, 2.6 mmol) was added, and the mixture was stirred. After completion of the reaction monitored by TLC, saturated NH₄Cl solution (50 mL) was added, and the mixture was extracted with ethyl acetate (50 mL×3). The combined organic phases were washed once with saturated NaHCO₃ solution (50 mL), dried over anhydrous sodium sulfate, suction filtered, and concentrated under reduced pressure. The resulting residue was purified by column chromatography (petroleum ether/ethyl acetate (V/V)=1:1 to 1:0) to obtain compound ethyl 3-(4-(benzyloxy)-3-(1-methyl-1H-pyrazol-5-yl)phenyl)-3-((N-(tert-butoxycarbonyl)glycyl)amino)propanoate (06E) (0.80 g, yield 62%).

LC-MS, M/Z (ESI): 537.6 [M+H]⁺.

### Step 5: Synthesis of ethyl 3-((N-(tert-butoxycarbonyl)glycyl)amino)-3-(4-hydroxy-3-(1-methyl-1H-pyrazol-5-yl)phenyl)propanoate (06F)

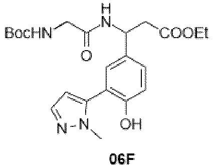

At room temperature, compound ethyl 3-(4-(benzyloxy)-3-(1-methyl-1H-pyrazol-5-yl)phenyl)-3-((N-(tert-butoxycarbonyl)glycyl)amino)propanoate (0.80 g, 1.5 mmol) was dissolved in methanol (10 mL), and 10% wet palladium on carbon (100 mg) was added. The mixture was purged with hydrogen three times and stirred under a hydrogen atmosphere at room temperature. After complete conversion of the starting material monitored by TLC, the reaction solution was suction filtered. The filtrate was concentrated under reduced pressure to obtain compound ethyl 3-((N-(tert-butoxycarbonyl)glycyl)amino)-3-(4-hydroxy-3-(1-methyl-1H-pyrazol-5-yl)phenyl)propanoate (06F) (0.66 g, yield 99%).

LC-MS, M/Z (ESI): 447.5 [M+H]⁺.

### Step 6: Synthesis of ethyl 3-(4-((14-azido-3,6,9,12-tetraoxatetradecan-1-yl)oxy)-3-(1-methyl-1H-pyrazol-5-yl)phenyl)-3-((N-(tert-butoxycarbonyl)glycyl)amino)propanoate (06G)

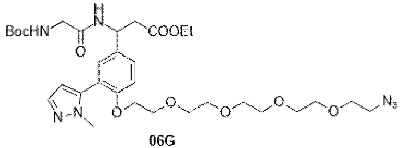

At room temperature, compound ethyl 3-((N-(tert-butoxycarbonyl)glycyl)amino)-3-(4-hydroxy-3-(1-methyl-1H-pyrazol-5-yl)phenyl)propanoate (0.66 g, 1.5 mmol) was dissolved in anhydrous DMF (5 mL), and potassium carbonate (0.51 g, 3.7 mmol) and azide-pentaethylene glycol-p-toluenesulfonate (0.80 g, 1.9 mmol) were added. The mixture was stirred at 80°C for 6 hours. After complete conversion of the starting material monitored by TLC, the mixture was cooled to room temperature. Saturated NaHCO₃ solution (10 mL) was added, and the mixture was extracted with ethyl acetate (10 mL×3). The combined organic phases were washed once with saturated NaCl solution (20 mL), dried over anhydrous sodium sulfate, suction filtered, and concentrated under reduced pressure to obtain compound ethyl 3-(4-((14-azido-3,6,9,12-tetraoxatetradecan-1-yl)oxy)-3-(1-methyl-1H-pyrazol-5-yl)phenyl)-3-((N-(tert-butoxycarbonyl)glycyl)amino)propanoate (06G) (0.97 g, yield 95%).

LC-MS, M/Z (ESI): 692.8 [M+H]⁺.

### Step 7: Synthesis of ethyl 3-(4-((14-azido-3,6,9,12-tetraoxatetradecan-1-yl)oxy)-3-(1-methyl-1H-pyrazol-5-yl)phenyl)-3-(glycylamino)propanoate hydrochloride (06H)

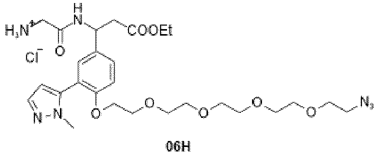

At room temperature, compound ethyl 3-(4-((14-azido-3,6,9,12- tetraoxatetradecan-1-yl)oxy)-3-(1-methyl-1H-pyrazol-5-yl)phenyl)-3-((*N*-(tert-butoxycarbonyl)glycyl)amino) propanoate (0.97 g, 1.4 mmol) was dissolved in a solution of hydrogen chloride in 1,4-dioxane (4 M, 7 mL), and the mixture was stirred for half an hour. After complete conversion of the starting material monitored by TLC, the mixture was concentrated under reduced pressure to obtain a crude product. Ethyl acetate was added, and the mixture was stirred and suction filtered. The filter cake was collected and dried under vacuum to obtain compound ethyl 3-(4-((14-azido-3,6,9,12-tetraoxatetradecan-1-yl)oxy)-3-(1-methyl-1H-pyrazol-5-yl)phenyl)-3-(glycylamino)propanoate hydrochloride (06H) (0.88 g, yield 100%).

LC-MS, M/Z (ESI): 592.6 [M+H]⁺.

### Step 8: Synthesis of ethyl 3-(4-((14-azido-3,6,9,12-tetraoxatetradecan-1-yl)oxy)-3-(1-methyl-1H-pyrazol-5-yl)phenyl)-3-((N-(4-((4-cyano-1-((2-(trimethylsilyl)ethoxy)methyl)-1H-imidazol-2-yl)((4-methoxyphenyl)methyl)amino)butanoyl)glycyl)amino)propanoate (06I)

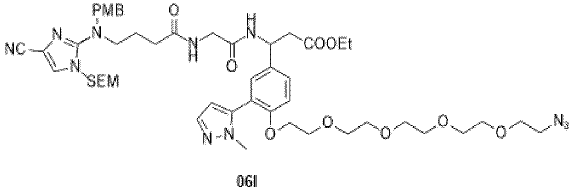

4-((4-Cyano-1-((2-(trimethylsilyl)ethoxy)methyl)-1H-imidazol-2-yl)(4-methoxybenzyl)amino)butanoic acid (01L) (0.88 g, 2.0 mmol), DMF (20 mL), ethyl 3-(4-((14-azido-3,6,9,12-tetraoxotetradecan-1-yl)oxy)-3-(1-methyl-1H-pyrazol-5-yl)phenyl)-3-(glycylamido)propanoate hydrochloride (1.5 g, 2.4 mmol), TBTU (0.77 g, 2.4 mmol), and DIPEA (0.77 g, 6.0 mmol) were added sequentially to a 100 mL single-necked flask. The mixture was stirred at room temperature for 16 hours under nitrogen protection. Water (10 mL) was added to the reaction solution, and the mixture was extracted with ethyl acetate (20 mL×3). The combined organic phases were washed sequentially with saturated sodium bicarbonate solution (20 mL) and saturated brine (20 mL), dried over anhydrous sodium sulfate, filtered, and concentrated to obtain a crude product. The crude product was purified by column chromatography (dichloromethane/methanol (V/V)=95:5) to obtain ethyl 3-(4-((14-azido-3,6,9,12-tetraoxatetradecan-1-yl)oxy)-3-(1-methyl-1H-pyrazol-5-yl)phenyl)-3-((*N*-(4-((4-cyano-1-((2-(trimethylsilyl)ethoxy)methyl)-1H-imidazol-2-yl)((4-methoxyphenyl)methyl) amino)butanoyl)glycyl)amino)propanoate (06I) (1.5 g, yield 74%).

LC-MS, M/Z (ESI): 1018.5 [M+H]⁺.

### Step 9: Synthesis of 3-(4-((14-azido-3,6,9,12-tetraoxatetradecan-1-yl)oxy)-3-(1-methyl-1H-pyrazol-5-yl)phenyl)-3-((N-(4-((4-cyano-1-((2-(trimethylsilyl)ethoxy)methyl)-1H-imidazol-2-yl)((4-methoxyphenyl)methyl)amino)butanoyl)glycyl)amino)propanoic acid (06J)

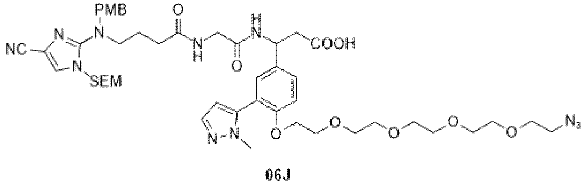

Ethyl 3-(4-((14-azido-3,6,9,12-tetraoxatetradecan-1-yl)oxy)-3-(1-methyl-1H-pyrazol-5-yl)phenyl)-3-((N-(4-((4-cyano-1-((2-(trimethylsilyl)ethoxy)methyl)-1 H-imidazol-2-yl)((4-methoxyphenyl)methyl)amino)butanoyl)glycyl)amino)propanoate (1.0 g, 0.98 mmol), THF/H₂O (v:v=1:1, 5 mL), and lithium hydroxide monohydrate (50 mg, 1.2 mmol) were added sequentially to a 100 mL single-necked flask. The mixture was stirred at room temperature for 3 hours. The reaction solution was concentrated under reduced pressure to remove THF. The pH of the residue was adjusted to 6 with 1 N hydrochloric acid, and the mixture was extracted with ethyl acetate (5 mL×3). The combined organic phases were washed with saturated brine (5 mL), dried over anhydrous sodium sulfate, filtered, and concentrated to obtain 3-(4-((14-azido-3,6,9,12-tetraoxatetradecan-1-yl)oxy)-3-(1-methyl-1H-pyrazol-5-yl)phenyl)-3-((N-(4-((4-cyano-1-((2-(trimethylsilyl)ethoxy)methyl)-1H-imidazol-2-yl)((4-methoxyphenyl)methyl) amino)butanoyl)glycyl)amino)propanoic acid (06J) (734 mg, yield 75%).

LC-MS, M/Z (ESI): 990.5 [M+H]⁺.

### Step 10: Synthesis of 3-(4-((14-azido-3,6,9,12-tetraoxtetracan-1-yl)oxy)-3-(1-methyl-1H-pyrazol-5-yl)phenyl)-3-((N-(4-((4-cyano-1H-imidazol-2-yl)amino)butanoyl)glycyl)amino)propanoic acid (II-1)

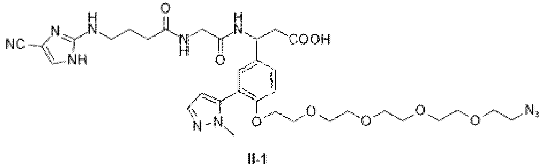

3-(4-((14-Azido-3,6,9,12-tetraoxatetradecan-1-yl)oxy)-3-(1-methyl-1H-pyrazol-5-yl)phenyl)-3-((*N*-(4-((4-cyano-1-((2-(trimethylsilyl)ethoxy)methyl)-1H-imidazol-2-yl)((4-methoxyphenyl)methyl)amino)butanoyl)glycyl)amino)propanoic acid (634 mg, 0.640 mmol), dichloromethane (20 mL), trifluoroacetic acid (6 mL, 80 mmol), anisole (697 mg, 6.44 mmol), and trifluoromethanesulfonic acid (510 mg, 3.40 mmol) were added sequentially to a 100 mL single-necked flask. The mixture was stirred at room temperature for 3 hours. The reaction solution was concentrated under reduced pressure to obtain a crude product. The crude product was purified by preparative reversed-phase high-performance liquid chromatography (column: YMC-Triart Prep C18 (30 mm×40 cm, 7 µm); mobile phase: A=0.1% formic acid, B=acetonitrile; gradient: 10% to 95%) to obtain 3-(4-((14-azido-3,6,9,12-tetraoxtetracan-1-yl)oxy)-3-(1-methyl-1H-pyrazol-5-yl)phenyl)-3-((*N*-(4-((4-cyano-1H-imidazol-2-yl)amino)butanoyl)glycyl)amino)propanoic acid (II-1) (450 mg, yield 95.0%).

¹H NMR (600 MHz, DMSO-*d₆*) δ 11.25 (s, 1H), 8.44 (s, 1H), 8.07 (s, 1H), 7.49 (s, 1H), 7.42 (d, *J*=6.0 Hz, 1H), 7.37-7.31 (m, 1H), 7.20 (d, *J*=2.8 Hz, 1H), 7.08 (d, *J*=8.4 Hz, 1H), 6.24 (s, 1H), 5.17 (t, *J*=8.0 Hz, 1H), 4.20-4.02 (m, 2H), 3.71-3.58 (m, 7H), 3.61-3.56 (m, 2H), 3.56-3.45 (m, 12H), 3.37 (d, *J*=4.4 Hz, 2H), 3.12-3.02 (m, 2H), 2.68 (d, *J*=7.2 Hz, 2H)*,* 2.17 (t, *J*=7.2 Hz, 2H), 1.72-1.63 (m, 2H).

LC-MS, M/Z (ESI): 740.3 [M+H]⁺.

### Step 11: Synthesis of (R)-3-(4-((14-azido-3,6,9,12-tetraoxatetradecan-1-yl)oxy)-3-(1-methyl-1H-pyrazol-5-yl)phenyl)-3-((N-(4-((4-cyano-1H-imidazol-2-yl)amino)butanoyl) glycyl)amino)propanoic acid (II-1A) and (S)-3-(4-((14-azido-3,6,9,12-tetraoxatetradecan-1-yl)oxy)-3-(1-methyl-1H-pyrazol-5-yl)phenyl)-3-((N-(4-((4-cyano-1H-imidazol-2-yl)amino) butanoyl)glycyl)amino)propanoic acid (II-1B)

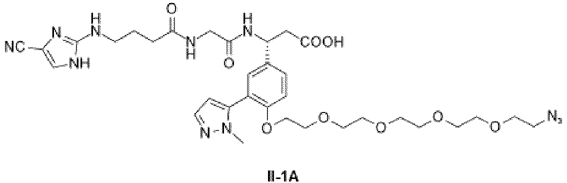

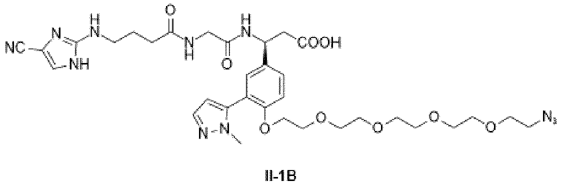

3-(4-((14-Azido-3,6,9,12-tetraoxatetradecan-1-yl)oxy)-3-(1-methyl-1H-pyrazol-5-yl)phenyl)-3-((*N*-(4-((4-cyano-1H-imidazol-2-yl)amino)butanoyl)glycyl)amino)propanoic acid was subjected to supercritical fluid chromatography (SFC) resolution (column: (*S,S*) WHELK-O1 (250 mm×30 mm, 3.5 µm, mobile phase: A=CO₂, B=methanol (0.05% ethylenediamine); gradient: 30% to 60%) to obtain compound (*R*)-3-(4-((14-azido-3,6,9,12-tetraoxatetradecan-1-yl)oxy)-3-(1-methyl-1H-pyrazol-5-yl)phenyl)-3-((*N*-(4-((4-cyano-1H-imidazol-2-yl)amino)butanoyl)glycyl)amino)propanoic acid (**II-1A**; column: (S,S)Whelk-O1 50×4.6 mm I.D., 3.5 µm; mobile phase: A=CO₂, B=methanol (0.05% ethylenediamine); gradient: 30% to 60%; flow rate: 3 mL/min; retention time: 1.471 min) and compound (*S*)-3-(4-((14-azido-3,6,9,12-tetraoxatetradecan-1-yl)oxy)-3-(1-methyl-1H-pyrazol-5-yl)phenyl)-3-((*N*-(4-((4-cyano-1H-imidazol-2-yl)amino)butanoyl)glycyl)amino)propanoic acid (**II-1B**; column: (S,S)Whelk-O1 50×4.6 mm I.D., 3.5 µm; mobile phase: A=CO₂, B=methanol (0.05% ethylenediamine); gradient: 30% to 60%; flow rate: 3 mL/min; retention time: 2.159 min).

**II-1A**: ¹H NMR (400 MHz, DMSO-*d₆*) δ 12.50-11.15 (m, 1H), 8.50 (s, 1H), 8.15-8.05 (m, 1H), 7.49 (s, 1H), 7.42 (s, 1H), 7.37-7.31 (m, 1H), 7.20 (d, J=2.8 Hz, 1H), 7.08 (d, *J*=8.4 Hz, 1H), 6.25 (s, 1H), 5.20-5.08 (m, 1H), 4.20-4.02 (m, 2H), 3.71-3.58 (m, 7H), 3.60-3.45 (m, 14H), 3.40-3.30 (m, 2H), 3.12-3.02 (m, 2H), 2.68 (d, *J*=7.2 Hz, 2H), 2.17 (t, *J*=7.2 Hz, 2H), 1.80-1.60 (m, 2H).

**II-1B**: ¹H NMR (400 MHz, DMSO-*d₆*) δ 12.4-11.4 (m, 1H), 8.44 (s, 1H), 8.15-8.05 (m, 1H), 7.49 (s, 1H), 7.42 (s, 1H), 7.37-7.31 (m, 1H), 7.20 (d, *J*=2.8 Hz, 1H), 7.10-7.08 (m, 1H), 6.24 (s, 1H), 5.20-5.10 (m, 1H), 4.20-4.02 (m, 2H), 3.71-3.58 (m, 7H), 3.61-3.56 (m, 2H), 3.56-3.45 (m, 12H), 3.40-3.30 (m, 2H), 3.12-3.02 (m, 2H), 2.68 (d, *J*=7.2 Hz, 2H), 2.17 (t, *J*=7.2 Hz, 2H), 1.80-1.60 (m, 2H).

### Example 7: Preparation of Compound III-1

The synthetic route is as follows:

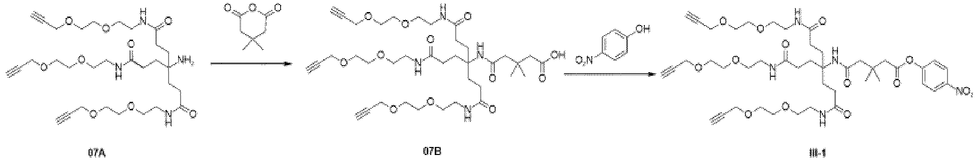

### Step 1: Synthesis of 18,18-dimethyl-11,16-dioxo-14,14-bis(3-oxo-3-((2-(2-((prop-2-yn-1-yl)oxy)ethoxy)ethyl)amino)propyl)-4,7-dioxo-10,15-diaza-1-yl-20-acid (07B)

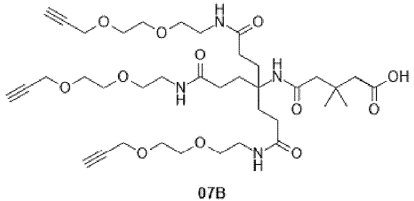

4-Amino-4-(3-oxo-3-((2-(2-((prop-2-yn-1-yl)oxy)ethoxy)ethyl)amino)propyl)-N1,N7-bis(2-(2-((prop-2-yn-1-yl)oxy)methoxy)ethyl ester)heptanediamide (500 mg, 0.803 mmol), DCM (5 mL), triethylamine (0.5 mL), and dihydro-4,4-dimethyl-2H-pyrano-2,6(3H)-dione (137 mg, 0.964 mmol) were added sequentially to a 100 mL single-necked flask. The mixture was stirred at room temperature for 3 hours. The reaction solution was concentrated under reduced pressure to obtain a crude product. The crude product was purified by column chromatography (dichloromethane/methanol (V/V)=92:8) to obtain 18,18-dimethyl-11,16-dioxo-14,14-bis(3-oxo-3-((2-(2-((prop-2-yn-1-yl)oxy)ethoxy)ethyl)amino)propyl)-4,7-dioxo-10,15-diaza-1-yl-20-acid (500 mg, yield 81.4%).

### Step 2: Synthesis of 18,18-dimethyl-11,16-dioxo-14,14-bis(3-oxo-3-((2-(2-((prop-2-yn-1-yl)oxy)ethoxy)ethyl)amino)propyl)-4,7-dioxo-10,15-diaza-1-yl-20-methanoic acid, 4-nitrophenyl ester (III-1)

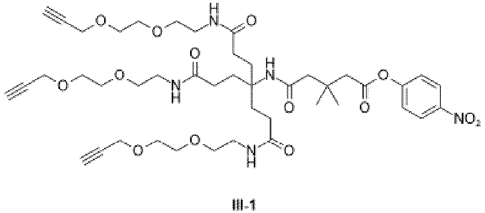

18,18-Dimethyl-11,16-dioxo-14,14-bis(3-oxo-3-((2-(2-((prop-2-yn-1-yl)oxy)ethoxy)ethyl)amino)propyl)-4,7-dioxo-10,15-diaza-1-yl-20-acid (500 mg, 0.654 mmol), DCM (5 mL), 4-nitrophenol (109 mg, 0.784 mmol), and EDCI (150 mg, 0.782 mmol) were added sequentially to a 100 mL single-necked flask. The mixture was stirred at room temperature for 12 hours. The reaction solution was concentrated under reduced pressure to obtain a crude product. The crude product was purified by column chromatography (dichloromethane/methanol (V/V)=92:8) to obtain the product 18,18-dimethyl-11,16-dioxo-14,14-bis(3-oxo-3-((2-(2-((prop-2-yn-1-yl)oxy)ethoxy)ethyl)amino)propyl)-4,7-dioxo-10,15-diaza-1-yl-20-methanoic acid, 4-nitrophenyl ester (**III-1**) (268 mg, yield 46.3%).

¹H NMR (400 MHz, DMSO-*d₆*) δ 8.27 (d, 2H), 7.81 (t, 3H), 7.41 (d, 2H), 7.21 (s, 1H), 4.10 (d, 6H), 3.51-3.49 (m, 12H), 3.38-3.36 (m, 9H), 3.16-3.13 (m, 6H), 2.73 (s, 2H), 2.17 (s, 2H), 2.01-1.97 (m, 6H), 1.80-1.76 (d, 6H), 1.09 (s, 6H).

LC-MS, M/Z (ESI): 887.3 [M+H]⁺.

### Example 8: Preparation of Compound III-2

The synthetic route is as follows:

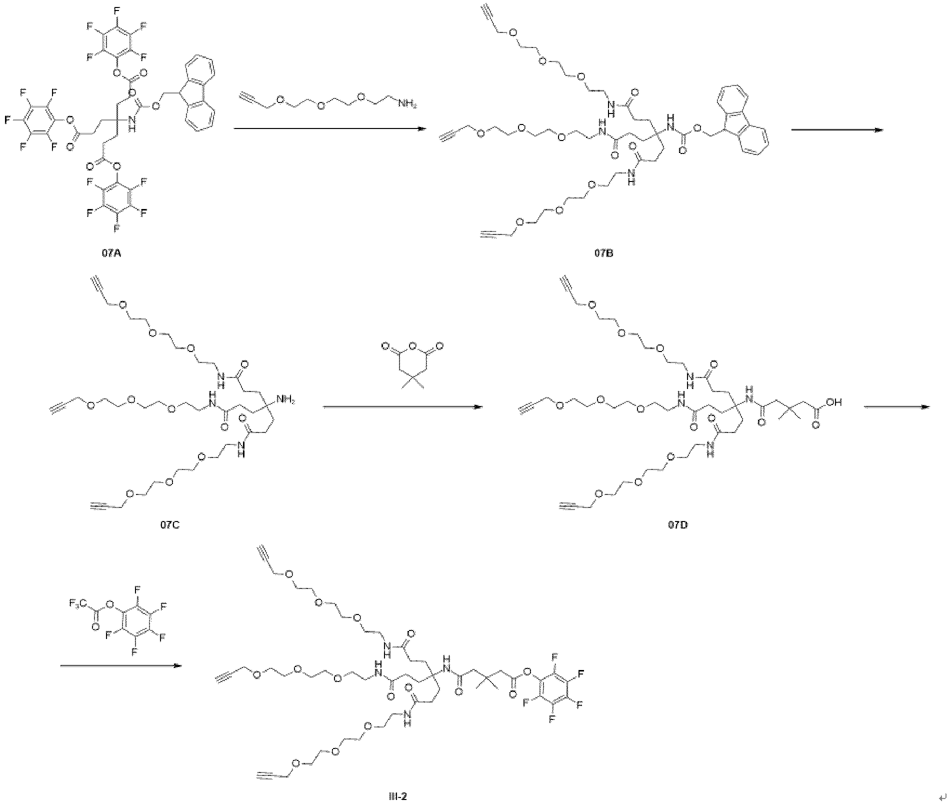

### Step 1: Synthesis of (9H-fluoren-9-yl)methyl(14,20-dioxo-17-(3-oxo-7,10,13-trioxa-4-azahexadec-15-yn-1-yl)-4,7,10,24,27,30-hexaoxa-13,21-diazadotriacont-1,32-diyn-17-yl)carbamate (07B)

Bis(perfluorophenyl) 4-((((9H-fluorene-9-yl)methoxy)carbonyl)amino)-4-(3-oxo-3-(perfluorophenoxy)propyl) heptanedioate (3.0 g, 3.1 mmol), DCM (30 mL), and 2-(2-(2-(prop-2-yn-1-yloxy)ethoxy)ethoxy)ethan-1-amine (2.1 g, 11 mmol) were added sequentially to a 100 mL single-necked flask. The mixture was stirred at room temperature for 3 hours. The reaction solution was concentrated under reduced pressure to obtain a crude product. The crude product was purified by column chromatography (dichloromethane/methanol (V/V)=95:5) to obtain (9H-fluoren-9-yl)methyl(14,20-dioxo-17-(3-oxo-7,10,13-trioxa-4-azahexadec-15-yn-1-yl)-4,7,10,24,27,30-hexaoxa-13,21-diazadotriacont-1,32-diyn-17-yl)carbamate (3.0 g, yield 99%).

### Step 2: Synthesis of 4-amino-4-(3-oxo-7,10,13-trioxa-4-azahexadec-15-yn-1-yl)-N1,N7-bis(2-(2-(2-(prop-2-yn-1-yloxy)ethoxy)ethoxy)ethyl)heptanediamide (07C)

(9H-Fluoren-9-yl)methyl(14,20-dioxo-17-(3-oxo-7,10,13-trioxa-4-azahexadec-15-yn-1-yl)-4,7,10,24,27,30-hexaoxa-13,21-diazadotriacont-1,32-diyn-17-yl)carbamate (3.0 g, 3.1 mmol), DMF (30 mL), and triethylamine (20 mL) were added sequentially to a 100 mL single-necked flask. The mixture was stirred at room temperature for 36 hours. The reaction solution was concentrated under reduced pressure to obtain a crude product. The crude product was purified by column chromatography (dichloromethane/methanol (V/V)=95:5) to obtain 4-amino-4-(3-oxo-7,10,13-trioxa-4-azahexadec-15-yn-1-yl)-*N*1,*N*7-bis(2-(2-(2-(prop-2-yn-1-yloxy)ethoxy)ethoxy)ethyl)heptanediamide (2.0 g, yield 86%).

### Step 3: Synthesis of 21,21-dimethyl-14,19-dioxo-17,17-bis(3-oxo-7,10,13-trioxa-4-azahexadec-15-yn-1-yl)-4,7,10-trioxa-13,18-diaza-1-yn-23-oleic acid (07D)

4-Amino-4-(3-oxo-7,10,13-trioxa-4-azahexadec-15-yn-1-yl)-*N*1,*N*7-bis(2-(2-(2-(prop-2-yn-1-yloxy)ethoxy)ethoxy)ethyl)heptanediamide (2.0 g, 2.6 mmol), DCM (20 mL), triethylamine (2 mL) and 4,4-dimethyldihydro-2H-pyran-2,6(3*H*)-dione (753 mg, 5.30 mmol) were added sequentially to a 100 mL single-necked flask. The mixture was stirred at room temperature for 3 hours. The reaction solution was concentrated under reduced pressure to obtain a crude product. The crude product was purified by column chromatography (dichloromethane/methanol (V/V)=92:8) to obtain 21,21-dimethyl-14,19-dioxo-17,17-bis(3-oxo-7,10,13-trioxa-4-azahexadec-15-yn-1-yl)-4,7,10-trioxa-13,18-diaza-1-yn-23-oleic acid (2.0 g, yield 84%).

### Step 4: Synthesis of perfluorophenyl 21,21-dimethyl-14,19-dioxo-17,17-bis(3-oxo-7,10,13-trioxa-4-azahexadec-15-yn-1-yl)-4,7,10-trioxa-13,18-diaza-1-yn-23-oleate (III-2)

21,21-Dimethyl-14,19-dioxo-17,17-bis(3-oxo-7,10,13-trioxa-4-azahexadec-15-yn-1-yl)-4,7,10-trioxa-13,18-diaza-1-yn-23-oleic acid (500 mg, 0.557 mmol), DCM (5 mL), and pentafluorophenyl trifluoroacetate (312 mg, 1.11 mmol) were added sequentially to a 100 mL single-necked flask. The mixture was stirred at room temperature for 12 hours. The reaction solution was concentrated under reduced pressure to obtain a crude product. The crude product was purified by column chromatography (ethyl acetate/acetonitrile (V/V)=1:1) to obtain perfluorophenyl 21,21-dimethyl-14,19-dioxo-17,17-bis(3-oxo-7,10,13-trioxa-4-azahexadec-15-yn-1-yl)-4,7,10-trioxa-13,18-diaza-1-yn-23-oleate (**III-2**) (110 mg, yield 18.6%).

¹H NMR (400 MHz, CDCl₃) δ 7.39 (s, 1H), 6.59 (t, 3H), 4.2 (d, 6H), 3.51-3.49 (m, 12H), 3.70-3.69 (m, 12H), 3.64-3.62 (m, 12H), 3.58-3.54 (m, 6H), 3.38-3.36 (m, 6H), 3.0 (q, 3H), 2.83 (s, 2H), 2.48 (s, 2H), 2.25-2.22 (m, 6H), 2.06-2.04 (d, 6H), 1.18 (s, 6H).

### Preparation Example 1: Design and Synthesis of siRNA Conjugates

The synthesis steps of an siRNA conjugate with a conjugation group linked to the 5' end of the siRNA sense strand is illustrated below, taking C1 in Table 1 as an example.
1) Synthesis of single-stranded oligoribonucleotides: Oligoribonucleotides were synthesized according to phosphoramidite solid-phase synthesis technology. Synthesis was performed on universal controlled pore glass (CPG, 500 Å). All 2'-modified nucleotide phosphoramidites and auxiliary reagents were commercially available reagents (Shanghai Hongene Biotech Corporation). All phosphoramidites were dissolved in anhydrous acetonitrile and molecular sieves (3 Å) were added. The coupling time using 5-ethylthio-1H-tetrazole (ETT, Suzhou Kelema Biotechnology Co., Ltd.) as an activator was 5 minutes. Phosphorothioate linkages were generated using a solution of 50 mM 3-((dimethylamino-methylene)amino)-3H-1,2,4-dithiazole-3-thione (DDTT, Shanghai Hongene Biotech Corporation) in anhydrous acetonitrile/pyridine (v/v = 1/1), with a reaction time of 3 minutes. Synthesis of all sequences was completed after final removal of the DMT protecting group.
2) Cleavage and deprotection of oligoribonucleotides bound on CPG: After completion of the solid-phase synthesis, protecting groups were removed by treatment with 20% diethylamine in acetonitrile for 30 minutes. After centrifugation, the supernatant was transferred to a new tube, and the CPG was washed with aqueous ammonia. The combined solutions were concentrated to obtain a solid mixture.
3) Purification of single-stranded oligoribonucleotides: Oligomers were purified by HPLC using NanoQ anion exchange chromatography. Buffer A was a solution containing 10 mM sodium perchlorate, 20 mM Tris, and 1 mM EDTA in 20% acetonitrile, pH 7.4; and Buffer B was a solution containing 500 mM sodium perchlorate, 20 mM Tris, and 1 mM EDTA in 20% acetonitrile, pH 7.4. The target products were isolated, and some products were desalted using a reverse-phase C18 column.
4) Coupling of linker to single-stranded oligoribonucleotide: 12 mg of TA14 was dissolved in DMSO and added to a nucleic acid amino intermediate (10 mg, dissolved in DMSO). After ultrasonic vibration and mixing, carbonate buffer solution (pH = 9) was additionally added. The reaction was carried out at 25°C for 16 h, followed by purification.
5) Coupling of II-1A to single-stranded oligoribonucleotide-TA14 conjugate: 7 mg of nucleic acid-TA14 was dissolved in a buffer salt solution, and ligand II-1A (equivalent: 15, dissolved in DMF) was added to the above solution followed by vortex mixing. THPTA and CuSO₄·5H₂O were mixed at a molar ratio of 5:1 (equivalents: 15 and 3, respectively) and shaken at 40 °C for 5 min. The nucleic acid-ligand mixture was then added to the above reaction solution followed by vortex mixing. Sodium ascorbate (equivalent: 25) was rapidly added to the above solution followed by vortex mixing, and the reaction was carried out at 40°C for 1 h. After completion of the reaction, purification was performed to obtain the complete single-stranded oligoribonucleotide-TA14-II-1A conjugate.
6) Annealing to generate siRNA: The single-stranded oligoribonucleotides to be annealed were prepared into 200 µM solutions using sterile RNase-free water. The annealing reaction system was set as follows. A mixed solution with a total volume of 100 µL containing 10 nmol was placed in a 95°C water bath for 10 minutes (for ≥100 nmol, high-temperature treatment for 20 minutes was required). The mixture was immediately transferred to a 60°C water bath and allowed to cool naturally. The annealed solution should not be stored at high temperature after completion of annealing. Complementary strands were formed by combining equimolar single-stranded oligoribonucleotide solutions, thereby obtaining various unmodified siRNAs. The nucleotide sequences of the sense strand and antisense strand of the siRNAs obtained in the present disclosure are shown in Table 1. In addition, the molecular weights of the siRNAs were analyzed by liquid chromatography-mass spectrometry (LC-MS). Comparison between the measured molecular weights and the theoretical molecular weights showed that the measured values were approximately equal to the theoretical values, thereby confirming the siRNAs shown in Table 1. The specific structure of ligand (II-1A) in Table 1 is shown in the Summary section of the present specification (i.e., II-1A in the compound, or the stereoisomer, tautomer, or pharmaceutically acceptable salt thereof according to the third aspect). II-1A was derived from compound II-1A of Example 2, and III-1 and III-2 were derived from compounds III-1 and III-2 of Example 7 and Example 8, respectively.

**[Table 1]**

| Group | Nucleotide sequence of sense strand (5'-3') | Nucleotide sequence of antisense strand (5'-3') |
|---|---|---|
| AD-07475 | (TriSM6.1)(TA14)(NH₂C₆)csggaauggAfA fAfcugaacacaas(invAb) | cPrpusUfsgsUfgUfuCfaGfuUfuCfcAfuUfcCfsg |
| C1 | (II-1A)(TA14)(NH₂C₆) csggaauggAfAfAfcugaacacaas(invAb) | cPrpusUfsgsUfgUfuCfaGfuUfuCfcAfuUfcCfsg |
| C2 | (II-1B)(TA14)(NH₂C₆) csggaauggAfAfAfcugaacacaas(invAb) | cPrpusUfsgsUfgUfuCfaGfuUfuCfcAfuUfcCfsg |
| C3 | (II-1B)(III-1)(NH₂C₆) csggaauggAfAfAfcugaacacaas(invAb) | cPrpusUfsgsUfgUfuCfaGfuUfuCfcAfuUfcCfsg |
| C4 | (II-1B)(III-2)(NH₂C₆) csggaauggAfAfAfcugaacacaas(invAb) | cPrpusUfsgsUfgUfuCfaGfuUfuCfcAfuUfcCfsg |

AD-07475 was prepared according to Patent WO2022/216920A1. In Table 1, lowercase letters c, g, u, a, and t each represent a 2'-methoxy-modified nucleotide (i.e., the ribosyl 2'-OH group of the nucleotide represented by the corresponding uppercase letter is substituted with a methoxy group); "f" indicates that the nucleotide represented by the letter immediately preceding "f" is a 2'-fluoro-modified nucleotide (i.e., the 2'-OH group of the nucleotide represented by the letter immediately preceding "f" is substituted with a fluorine atom); and "s" indicates that the two adjacent nucleotide residues flanking "s" are linked through a phosphorothioate linkage (i.e., the 5'-phosphate group of the nucleotide represented by the letter immediately preceding "s" is substituted with a 5'-phosphorothioate group).

(TriSM6.1)(TA14)(NH₂C₆) is: 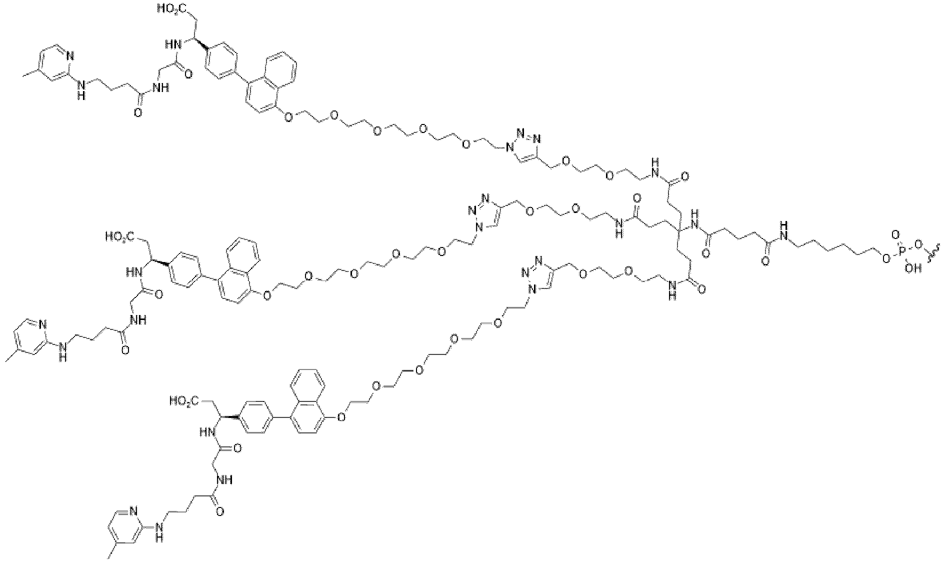 For its preparation method, reference can be made to WO2022/216920A1.

(II-1A)(TA14)(NH₂C₆) is:

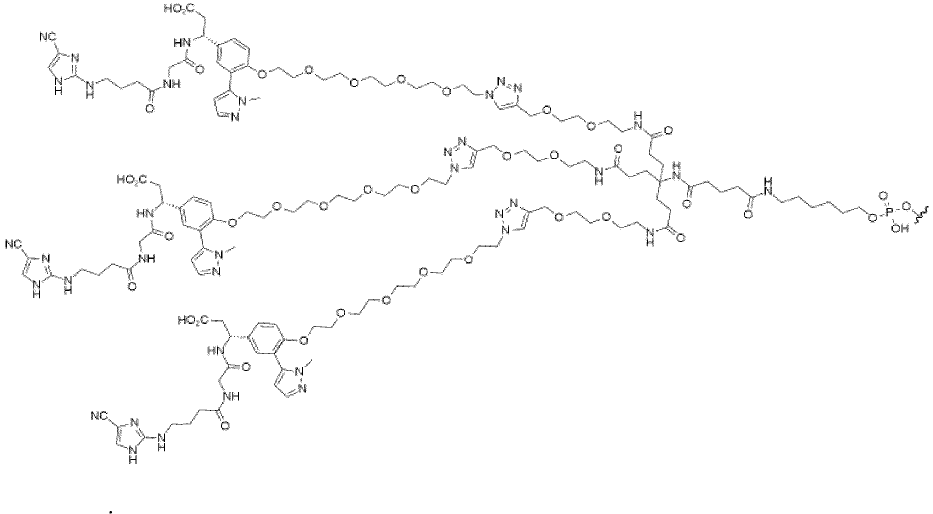

(II-1B)(TA14)(NH₂C₆) is:

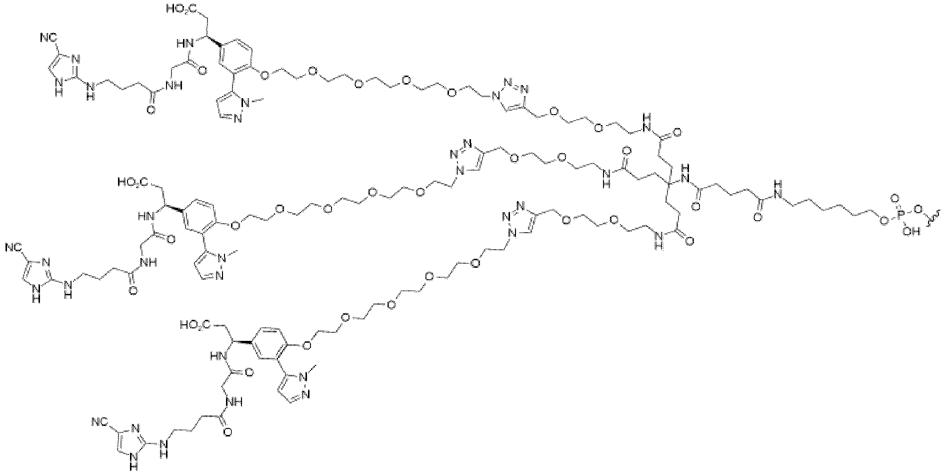

(II-1B)(III-1)(NH₂C₆) is:

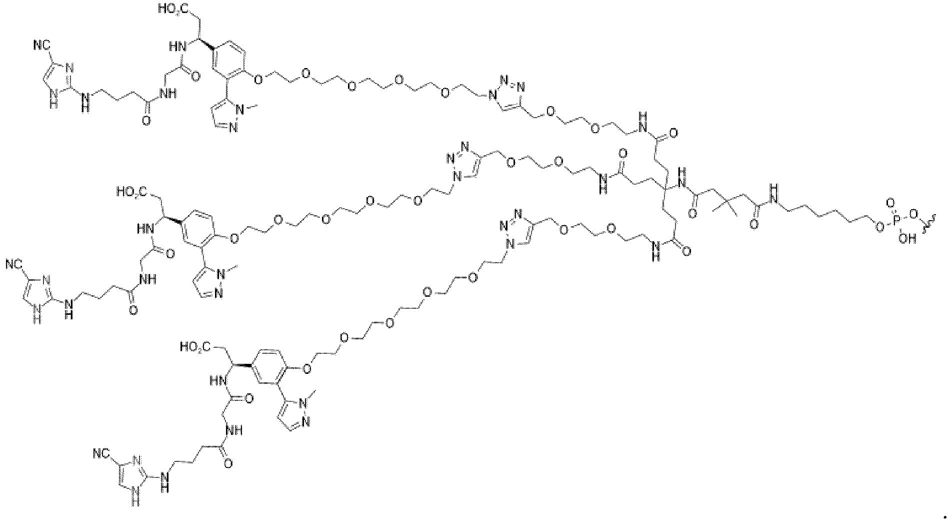

(II-1B)(III-2)(NH₂C₆) is:

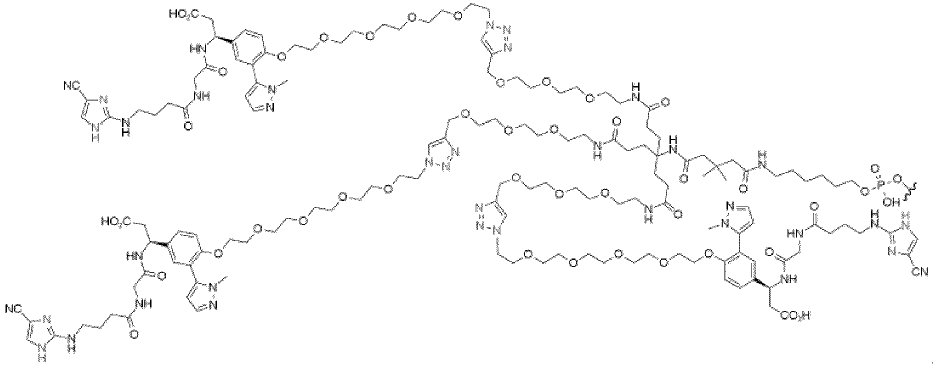

The structures of cPrpus and cPrpas are as follows:

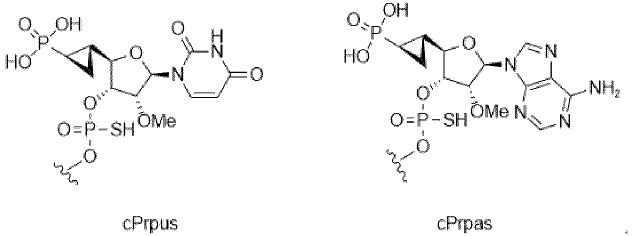

### Test Example 1: ELISA Binding Assay of αvβ6 Ligand

The integrin αvβ6 protein (source: Acro Biosystems) was diluted to 5 µg/mL with coating solution, and 25 µL of the protein was added to each well of a 384-well plate. The plate was centrifuged at 1,000 rpm for 1 minute and then left to stand overnight at 4°C. 100 µL of washing solution was added to each well and left to stand for 5 minutes. Then the plate was flicked dry and washed 3 times. 100 µL of blocking solution was added to each well. The plate was centrifuged at 1,000 rpm for 1 minute and then incubated at 25°C for 90 minutes. 100 µL of washing solution was added to each well and left to stand for 5 minutes. Then the plate was flicked dry and washed 3 times. 10 µL of the above compounds at different dilution concentrations was added to each well. The plate was centrifuged at 1,000 rpm for 1 minute and then incubated at 25°C for 10 minutes. 10 µL of fibronectin (source: R&D) was added to each well. The plate was centrifuged at 1,000 rpm for 1 minute and then incubated at 25°C for 60 minutes. 100 µL washing solution was added to each well and left to stand for 5 minutes. Then the plate was flicked dry and washed 3 times. 25 µL of biotinylated anti-fibronectin antibody (source: R&D) was added to each well. The plate was centrifuged at 1,000 rpm for 1 minute and then incubated at 25°C for 60 minutes. 100 µL washing solution was added to each well and left to stand for 5 minutes. Then the plate was flicked dry and washed 3 times. 25 µL of horseradish peroxidase-labeled streptavidin was added to each well. The plate was centrifuged at 1,000 rpm for 1 minute and then incubated at 25°C for 30 minutes. 100 µL of washing solution was added to each well and left to stand for 5 minutes. Then the plate was flicked dry and washed 4 times. 25 µL of ultrasensitive luminescent substrate solution was added to each well. The plate was centrifuged at 1000 rpm for 1 minute and then incubated at 25°C for 5 minutes. The luminescence values were read on a microplate reader.

**[Table 2]: Binding Activity of Integrin αvβ6 Ligands**

| **Compound No.** | **IC₅₀ (nM)** |
|---|---|
| I-1 | 4.42 |
| I-2 | 2.11 |
| II-1B | 4.53 |

The results show that the compounds of the present disclosure exhibited high binding activity to integrin αvβ6.

### Test Example 2: Silencing Efficacy of siRNA Conjugates Administered Intratracheally in Rats on Target Expression

Referring to Patent WO2022/216920A1, siRNA conjugates targeting rat RAGE protein were designed and synthesized. The list of the compounds is shown in Table 1.

### Experimental method:

### 1. Administration to Rats via Aerosol Needle and Collection of Serum and Lung Tissue Samples

On day 0, whole blood was collected from the jugular vein of rats, and serum was separated. On day 1, rats were anesthetized with isoflurane, and 200 µL of PBS or drug buffer was administered via an aerosol needle for inhalation at a dose of 0.1 mg/kg. Before the experimental endpoint, rats were anesthetized with 10% chloral hydrate. Blood was collected from the abdominal aorta using a negative-pressure blood collection tube, and serum was separated. Left and right lung tissues were then harvested and snap-frozen in liquid nitrogen. All treated serum and lung tissue samples were stored at -80°C.

### 2. Homogenization of Rat Lung Tissues and Probe Quantitative PCR

Lung tissues were thawed on ice and homogenized using a tissue homogenizer. Total RNA in lung tissues was extracted using Tissue RNA Extraction Kit 2.0 Plus (Vazyme, R411-C3). After reverse transcription (Vazyme, R333-01), the mRNA expression level of RAGE in rat lung tissues was detected by probe quantitative PCR (Vazyme, QN211-02), normalized against the expression level of rat GAPDH, and compared using the expression level of the PBS control group as 100%.

### 3. Detection of RAGE Protein Content in Rat Serum using ELISA Kit

Serum samples were thawed on ice and assayed according to the instructions of the Rat RAGE DuoSet ELISA kit (R&D Systems, DY1616). Based on fitting conversion according to the standard curve obtained from the assay, the expression level of RAGE protein in rat serum was calculated and compared using the expression level of the PBS control group as 100%.

**[Table 3]: Expression Levels of Rat Serum RAGE Protein and Lung RAGE mRNA**

| Group | Residual Serum sRAGE protein (%) | | | | Residual Lung RAGE mRNA (%) |
|---|---|---|---|---|---|
| | Day 8 | Day 15 | Day 22 | Day 29 | Experimental Endpoint (Day 36) |
| AD-07475 | 79.9 | 57.4 | 45.7 | 34.5 | 45.8 |
| C2 | 76.1 | 59.9 | 29.8 | 16.5 | 34.9 |
| C4 | 66.0 | 55.8 | 28.5 | 18.1 | 35.1 |

The results show that the siRNA conjugate significantly reduced the expression of RAGE mRNA and protein in rat lungs and serum. The compounds of the present disclosure can exhibit a stronger effect in silencing lung targets and corresponding secreted protein expression by siRNA, indicating their superior capability for targeted delivery of siRNA molecules to lung cells.

Although embodiments of the present disclosure have been shown and described, it would be appreciated that the above embodiments are exemplary and cannot be construed to limit the present disclosure, and changes, modifications, alternatives, and alterations can be made in the embodiments by those skilled in the art without departing from the scope of the present disclosure.

## Claims

1. A compound represented by Formula I, or a tautomer or stereoisomer thereof: 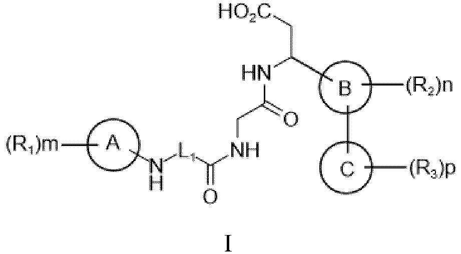 wherein:
ring A is 5-membered heteroaromatic ring;
ring B is 6- to 10-membered aromatic ring or 5- to 6-membered heteroaromatic ring;
ring C is 6- to 10-membered aromatic ring, 5- to 10-membered heteroaromatic ring, or 5-to 10-membered heterocycloalkyl;
L₁ is C₁ to C₆ alkylene or halogenated C₁ to C₆ alkylene;
R₁ is selected from H, halogen, -NH₂, -CN, -OH, -SF₅, -COOH, C₁₋₆ alkyl, halogenated C₁₋₆ alkyl, C₁₋₆ alkoxy, -CONH₂, -CONR₁₁R₁₂, -C(O)-C₁₋₆ alkyl, -S(O)₂-C₁₋₆ alkyl, -COOR₁₃, - NH-C₁₋₆ alkyl, and -NR₁₄R₁₅;
R₂ and R₃ are each independently selected from H, halogen, -NH₂, -CN, -OH, -SF₅, - COOH, C₁₋₆ alkyl, C₁₋₆ alkoxy, -CONH₂, -CONR₁₁R₁₂, -C(O)-C₁₋₆ alkyl, -S(O)₂-C₁₋₆ alkyl, - COOR₁₃, -NH-C₁₋₆ alkyl, and -NR₁₄R₁₅,
wherein R₁₁, R₁₂, R₁₃, R₁₄, and R₁₅ are each independently C₁₋₆ alkyl;
R₁, R₂, and R₃ are optionally substituted with one, two, or three identical or different substituents selected from halogen, -NH₂, -CN, -OH, -SF₅, -COOH, C₁₋₆ alkyl, and C₁₋₆ alkoxy;
ring B or ring C is optionally linked to a linker T, wherein the linker T is configured to directly or indirectly link an active molecule;
when a plurality of R₁ substituents are present, the plurality of R₁ substituents are identical or different;
when a plurality of R₂ substituents are present, the plurality of R₂ substituents are identical or different;
when a plurality of R₃ substituents are present, the plurality of R₃ substituents are identical or different;
m is 1, 2, or 3;
n is 1 or 2; and
p is 1 or 2.

2. The compound represented by Formula I, or the tautomer or stereoisomer thereof according to claim 1, wherein the compound satisfies one or more of the following conditions:
a) 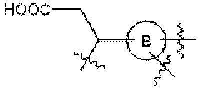 has a structure of 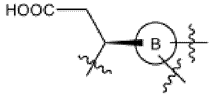 or 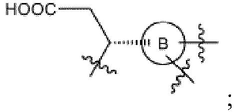
b) 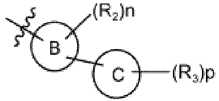 is 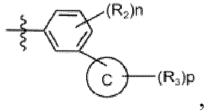 where R₂, R₃, ring C, n, and p are as defined in claim 1;
c) R₂ is H;
d) ring C is 5- to 8-membered heteroaromatic ring;
e) R₃ is halogen, -NH₂, -CN, -OH, C₁₋₆ alkyl, C₁₋₆ alkoxy, or halogenated C₁₋₆ alkyl, or R₃ is methyl; or
f) Li is C₁ to C₆ alkylene, or L₁ is -CH₂CH₂CH₂-;
preferably, ring C is 5- or 6-membered heteroaromatic ring, wherein heteroatom is selected from N, O, and S, and wherein when a plurality of heteroatoms are present, the plurality of heteroatoms are identical or different;
preferably, ring C is 5-membered N-containing heteroaromatic ring;
preferably, ring C is selected from furan, thiophene, pyrrole, thiazole, imidazole, pyrazole, oxazole, triazole, tetrazole, pyridine, pyridazine, pyrimidine, and pyrazine;
preferably, ring C is pyrazole; and
preferably, ring C is

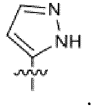

3. The compound represented by Formula I, or the tautomer or stereoisomer thereof according to claim 1, wherein the compound has a structure selected from one or more of: 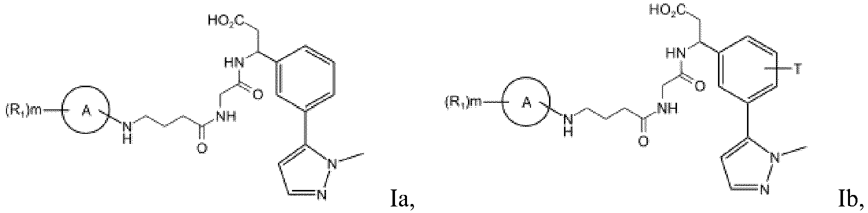 or 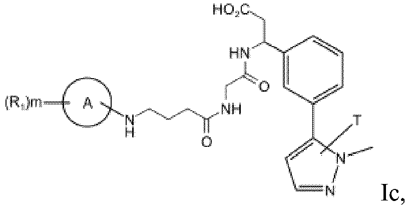 where ring A, R₁, m, and T are as defined in claim 1; and/or
the compound, when linked to the linker T, has a structure selected from one or more of: 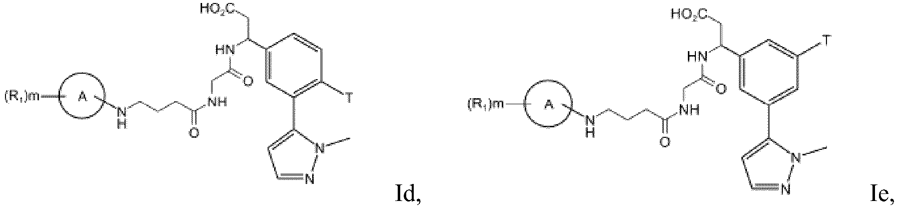 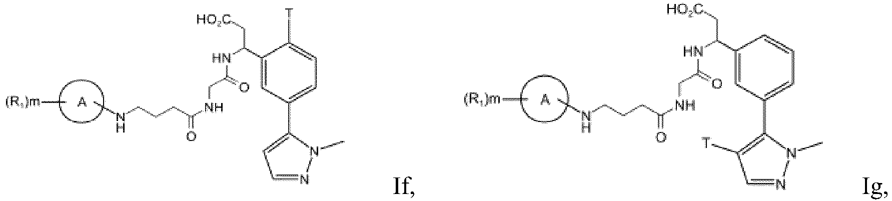 or 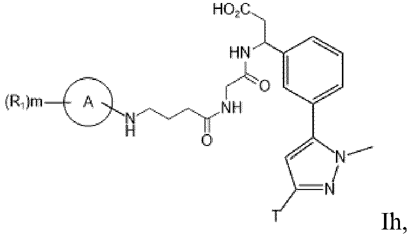 where ring A, R₁, m, and T are as defined in claim 1,
preferably, ring A is 5-membered heteroaryl; and/or
m is 0, 1, or 2.

4. The compound represented by Formula I, or the tautomer or stereoisomer thereof according to claim 1 or 3, wherein:
the linker T comprises a polyethylene glycol unit; or
the linker T comprises 2 to 20 polyethylene glycol units; or
the linker T is 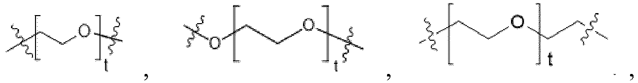 or 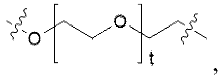 where t ranges from 1 to 10, and preferably, t is 1, 2, 3, 4, 5, or 6; and/or
the linker T is 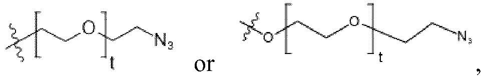 where t ranges from 1 to 10, and preferably, t is 1, 2, 3, 4, 5, or 6; or
the linker T is 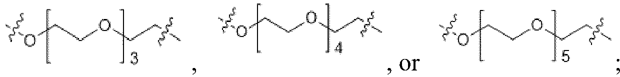 and/or
the linker T is

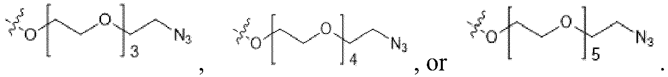

5. The compound represented by Formula I, or the tautomer or stereoisomer thereof according to claim 1, wherein the compound, when linked to the linker T, has a structure selected from one or more of: 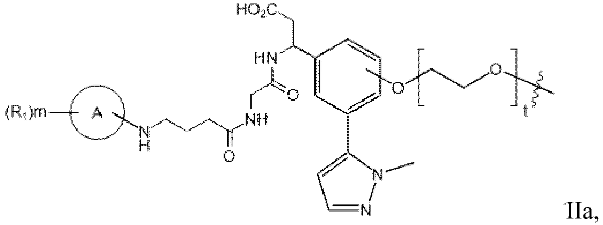 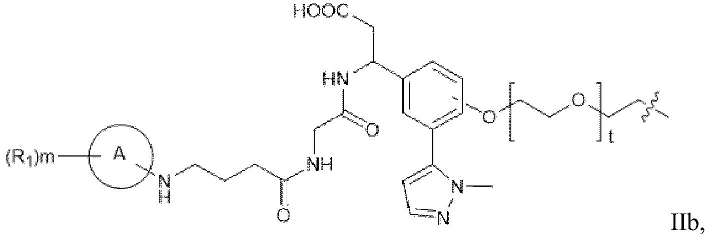 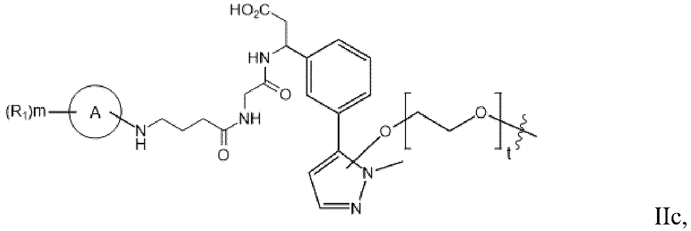 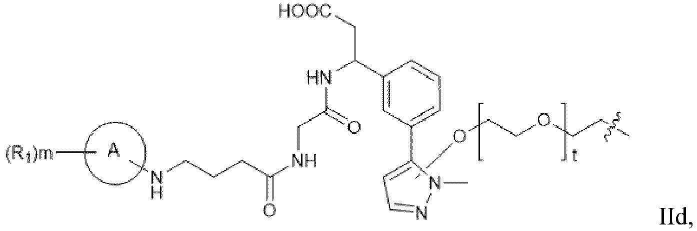 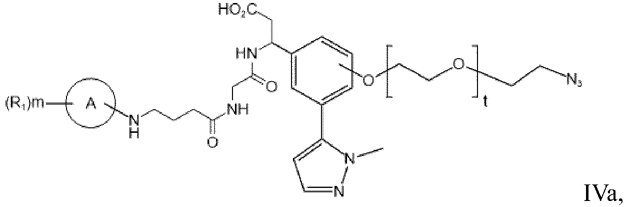 or 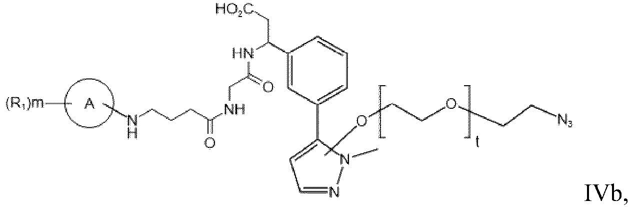 where t ranges from 1 to 10, or t is 1, 2, 3, 4, 5, or 6.

6. The compound represented by Formula I, or the tautomer or stereoisomer thereof according to any one of claims 1, 3, or 5, wherein the compound satisfies one or more of the following conditions:
g) ring A is 5-membered heteroaromatic ring, wherein heteroatom is selected from N, O, and S, and wherein when a plurality of heteroatoms are present, the plurality of heteroatoms are identical or different;
h) ring A is selected from furan, thiophene, pyrrole, thiazole, imidazole, pyrazole, oxazole, triazole, and tetrazole;
i) ring A is imidazole or triazole;
j) 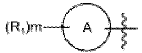 is 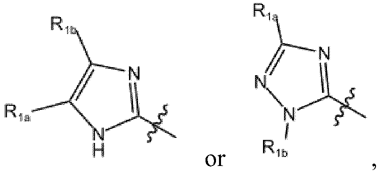 where R₁ₐ and R_{1b} are each independently R₁;
k) R₁ is halogen, -NH₂, -CN, -OH, -SF₅, -COOH, C₁₋₆ alkyl, -CONH₂, -C(O)-C₁₋₆ alkyl, or -S(O)₂-C₁₋₆ alkyl, wherein the C₁₋₆ alkyl, the -C(O)-C₁₋₆ alkyl, or the -S(O)₂-C₁₋₆ alkyl is optionally substituted with one, two, or three identical or different substituents selected from halogen, -NH₂, -CN, -OH, -SF₅, C₁₋₃ alkyl, and C₁₋₃ alkoxy;
m) ring A is 5-membered N-containing heteroaromatic ring, or ring A is 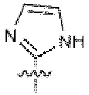 or 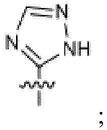
n) R₁ is H, -F, -Cl, -CN, -SF₅, -COOH, -CF₃, -CHF₂, -COCH₃, -CONH₂, or -S(O)₂CH₃; or R₁ is H, -F, -Cl, or -CN; or
p) 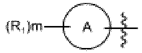 is selected from

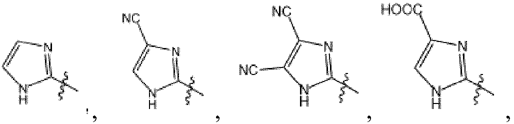

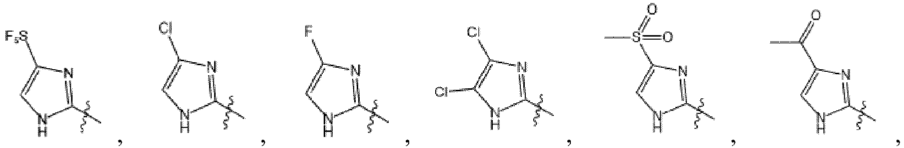

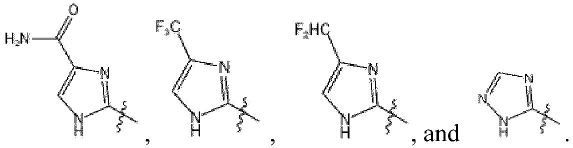

7. The compound represented by Formula I, or the tautomer or stereoisomer thereof according to claim 1, wherein the compound has a structure selected from one or more of: 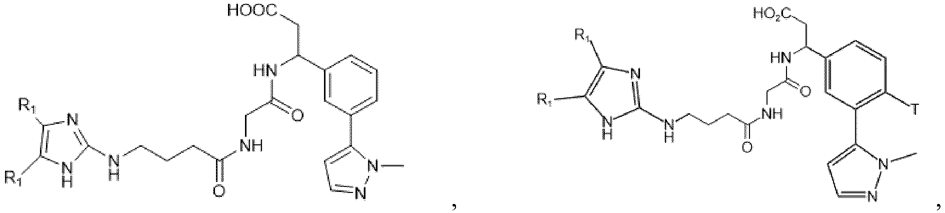 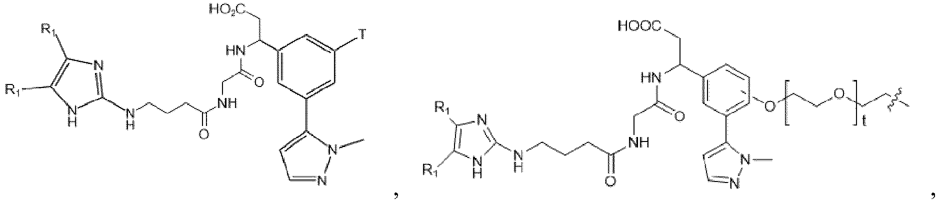 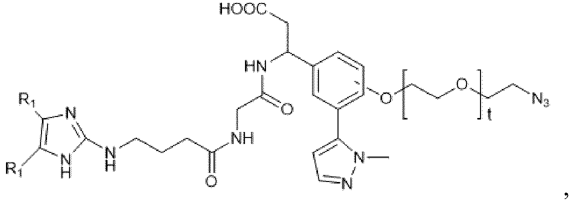 where: 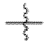
represents an attachment point; and
each R₁ is independently defined as in claim 1, 6, or 7, and preferably, each R₁ is independently H, -F, -Cl, or -CN.

8. The compound represented by Formula I, or the tautomer or stereoisomer thereof according to claim 1, wherein the compound has a structure selected from one or more of: 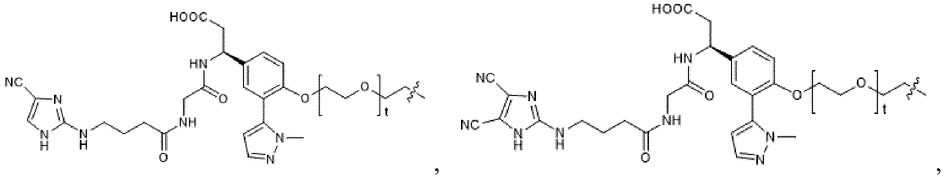 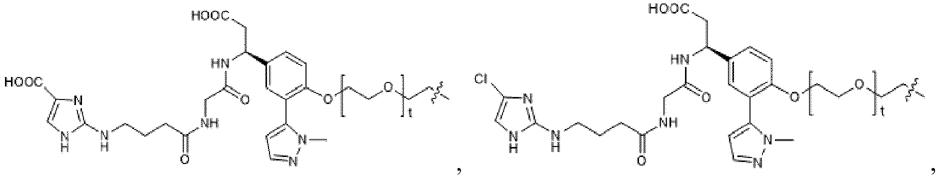 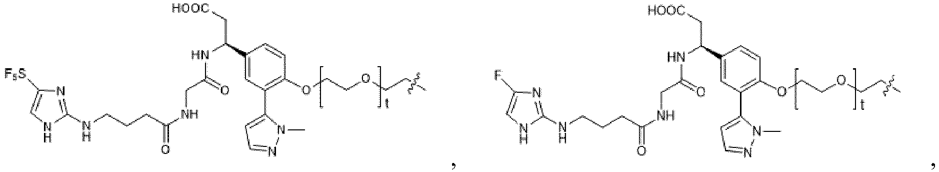 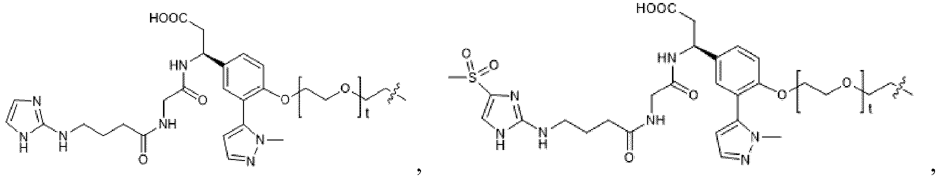 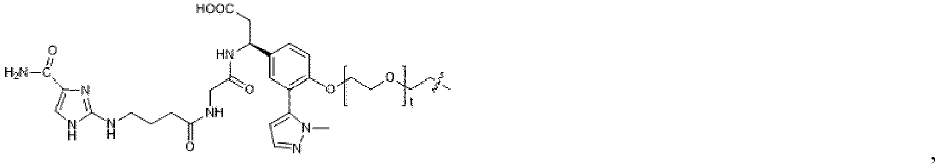 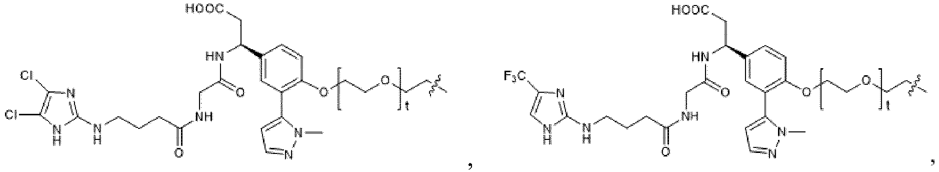 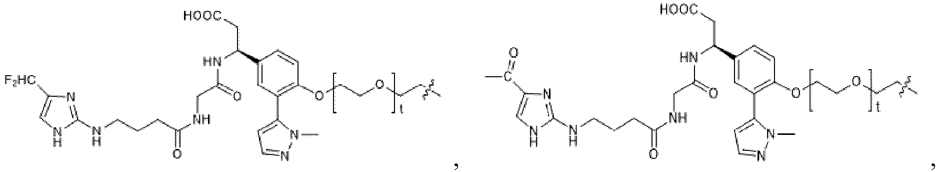 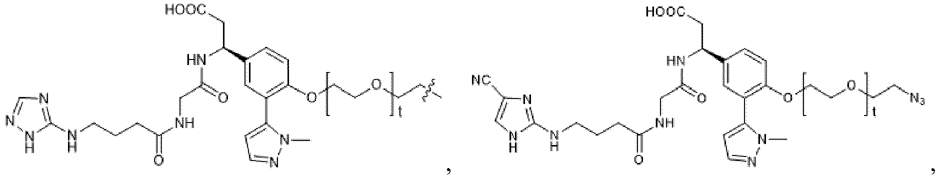 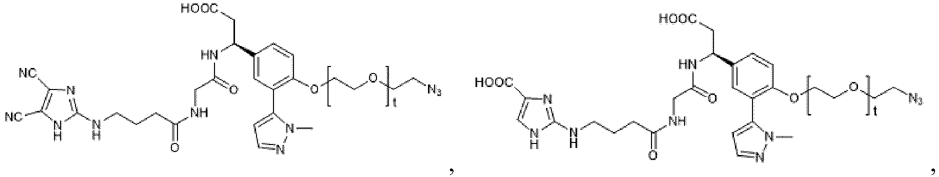 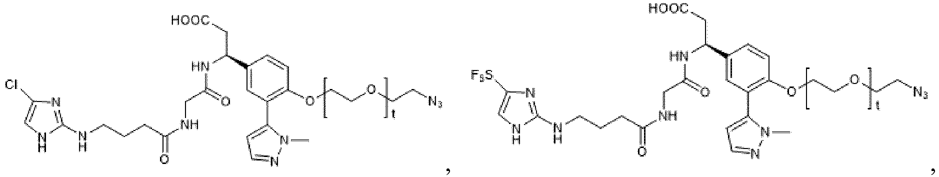 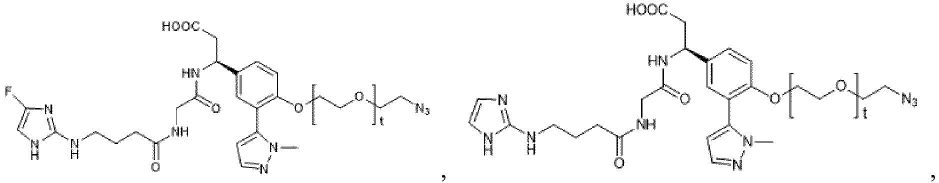 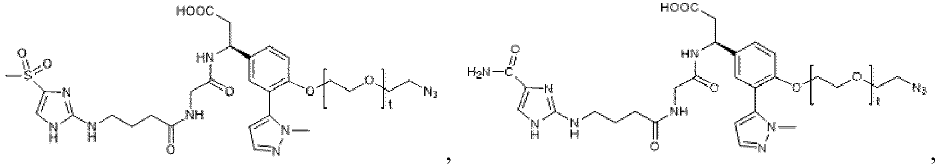 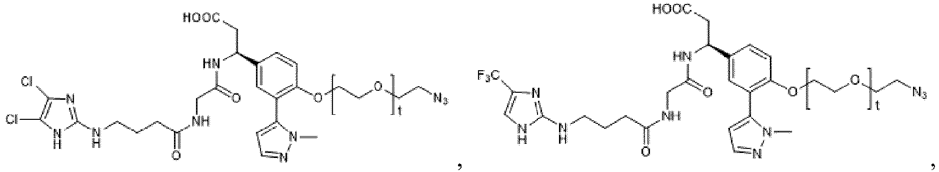 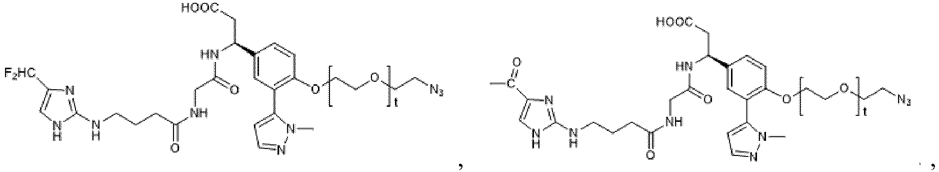 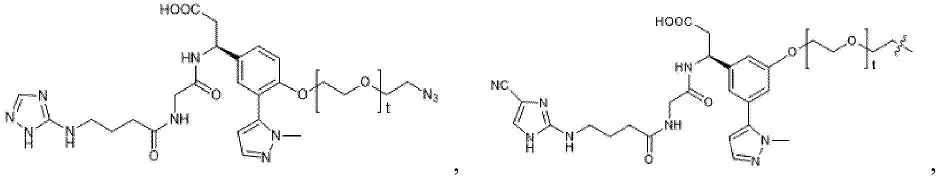 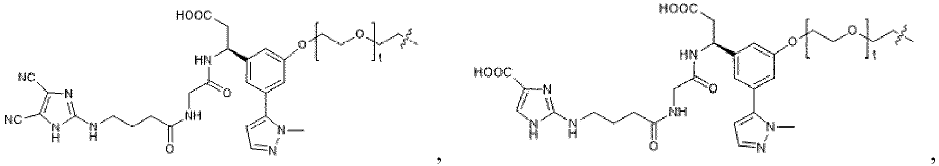 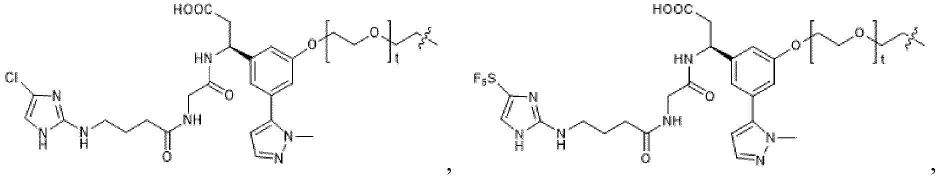 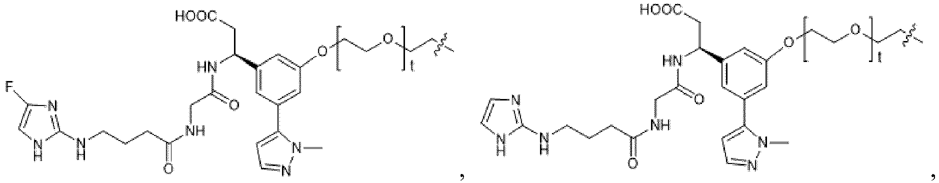 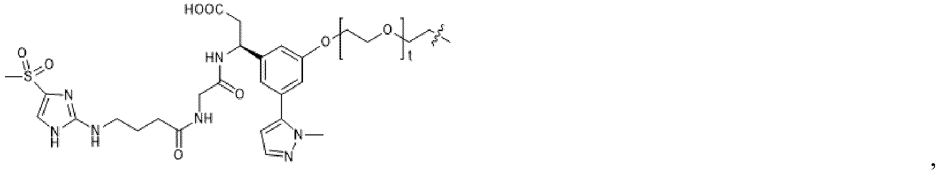 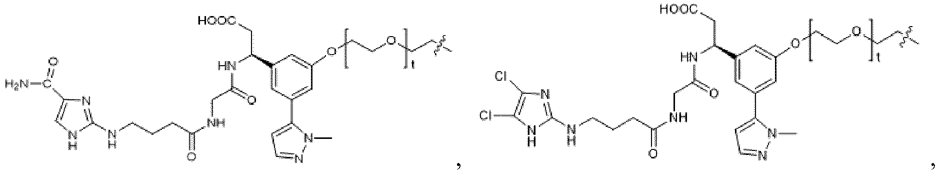 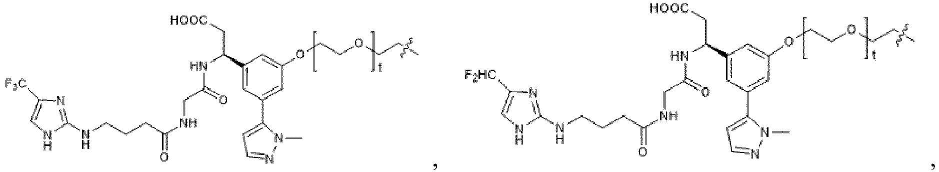 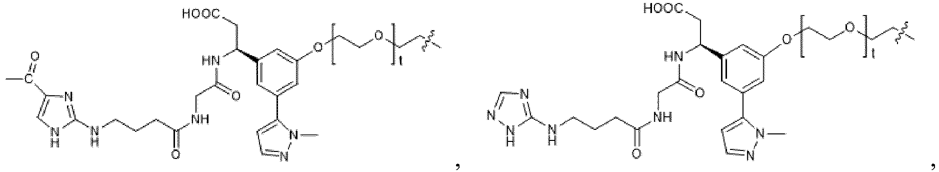 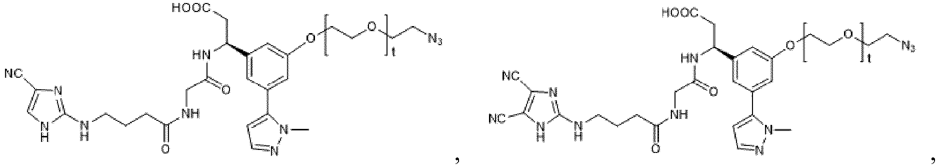 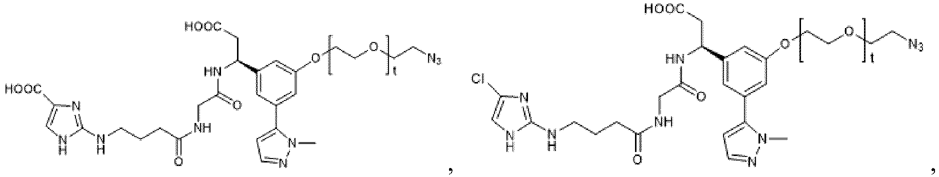 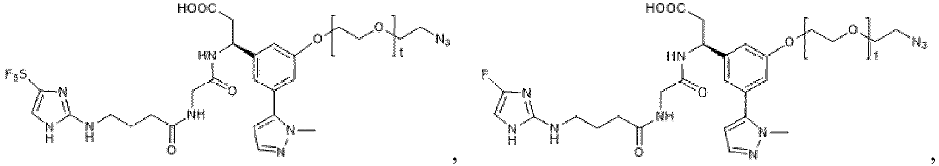 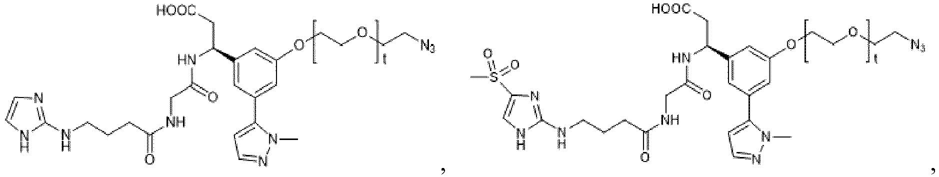 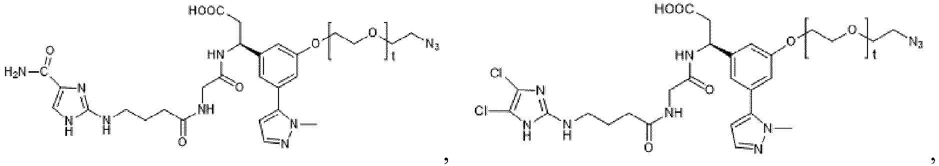 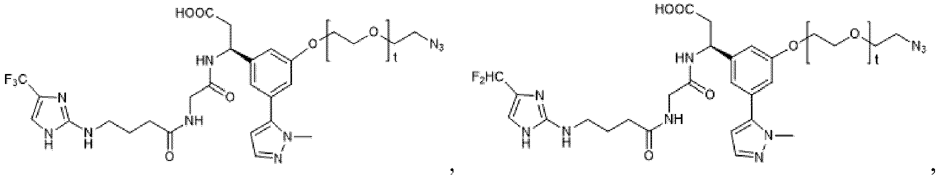 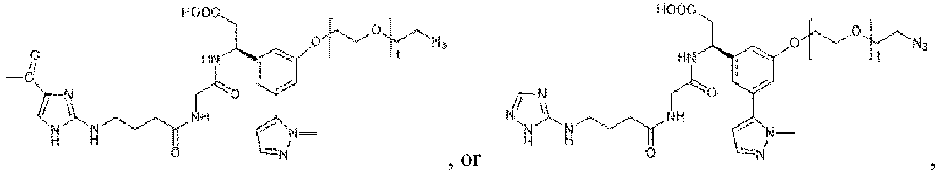 where:
t is 3, 4, 5, or 6; and 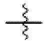
represents an attachment point.

9. The compound represented by Formula I, or the tautomer or stereoisomer according to claim 1, wherein the compound comprises:

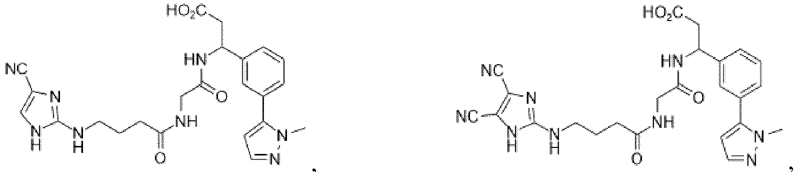

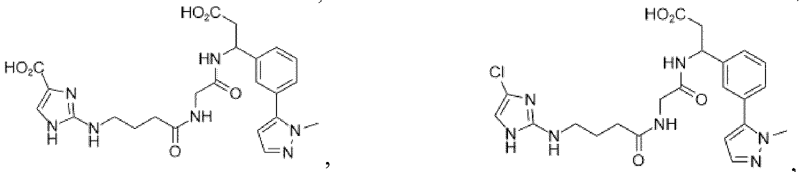

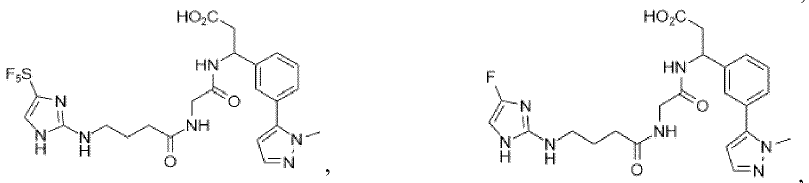

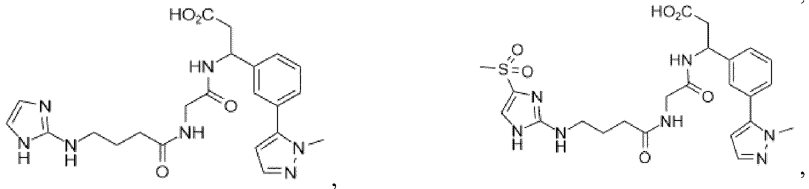

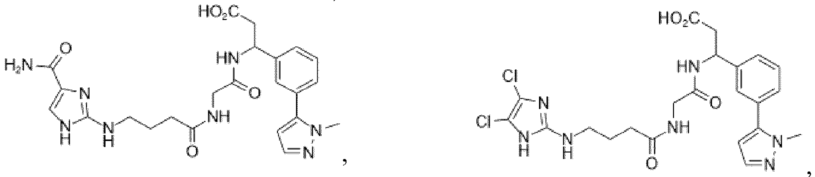

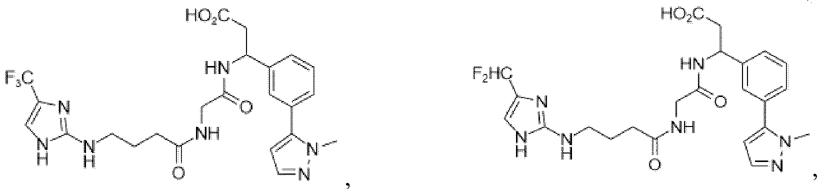

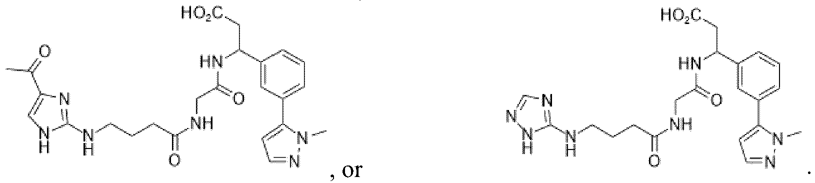

10. The compound represented by Formula I, or the tautomer or stereoisomer thereof according to claim 1, wherein the compound comprises: 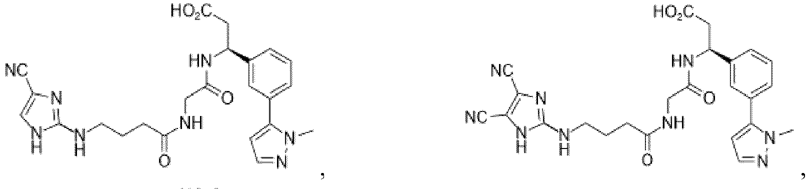 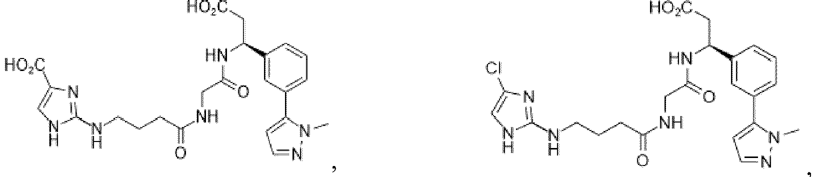 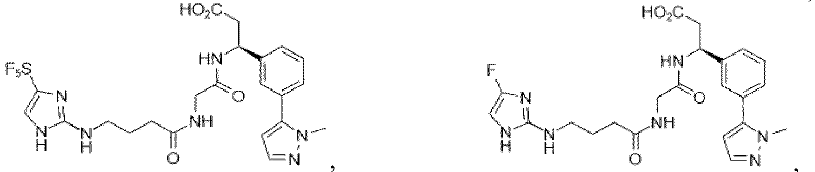 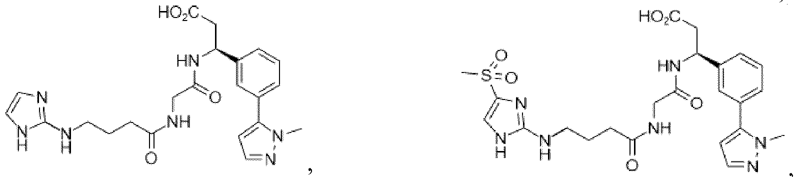 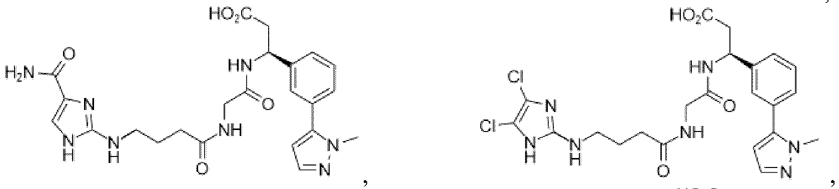 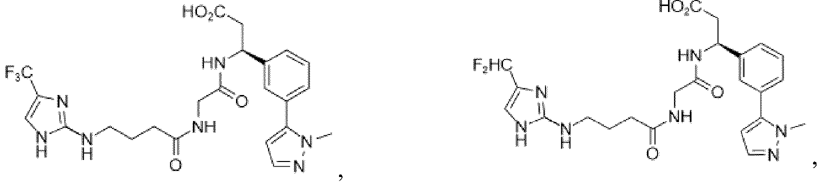 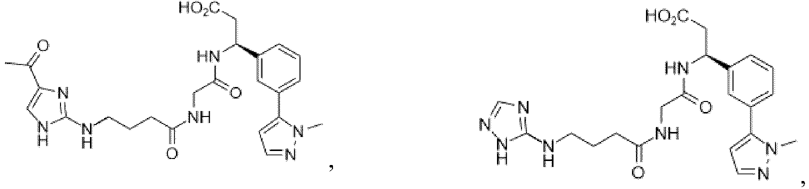 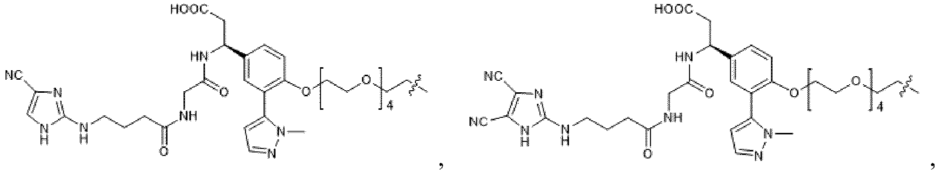 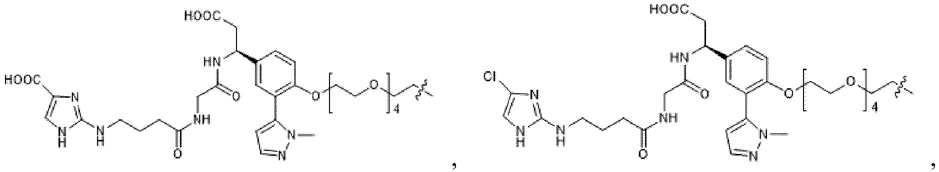 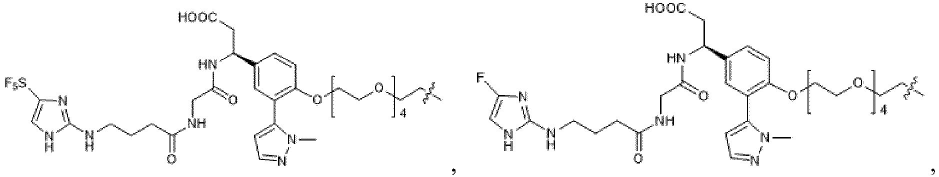 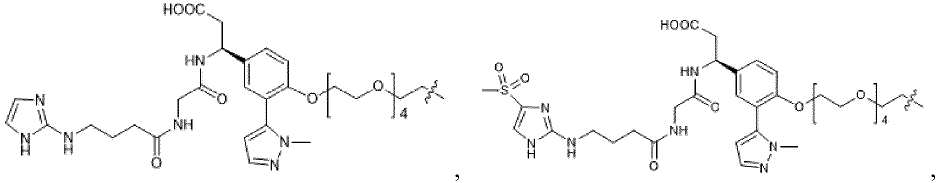 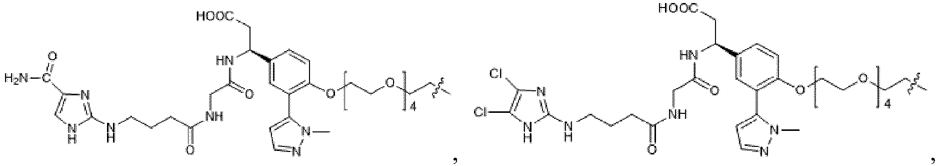 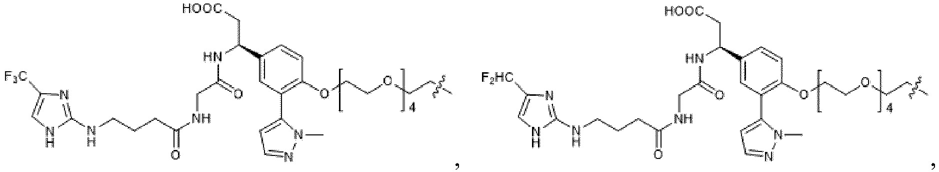 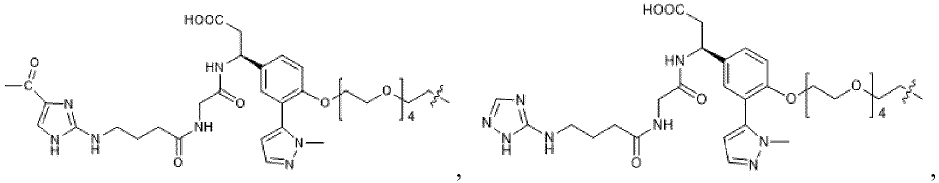 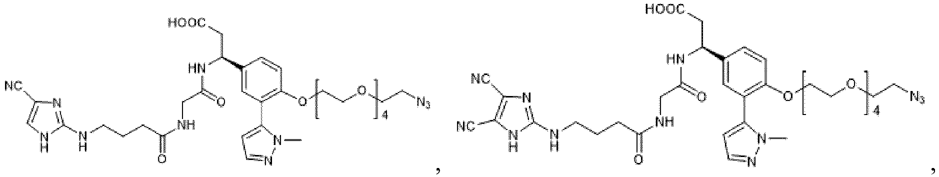 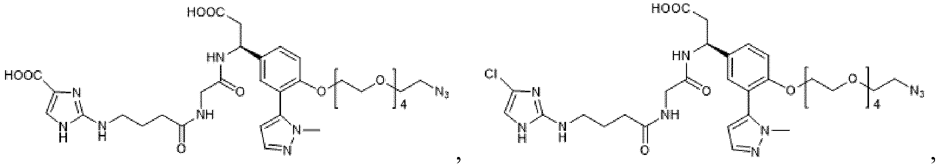 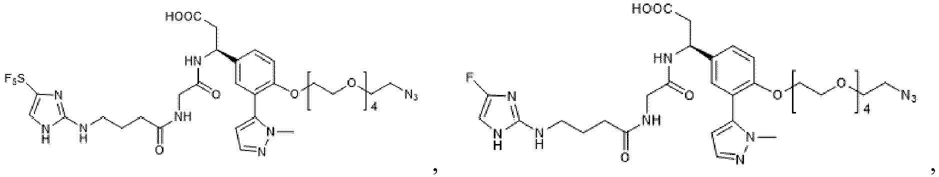 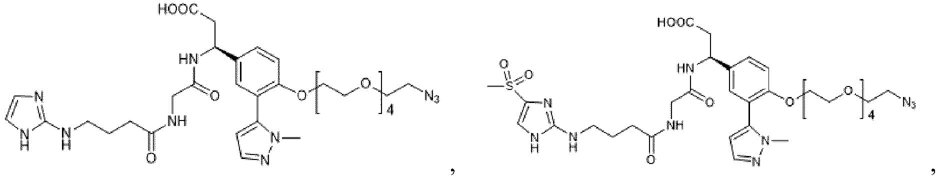 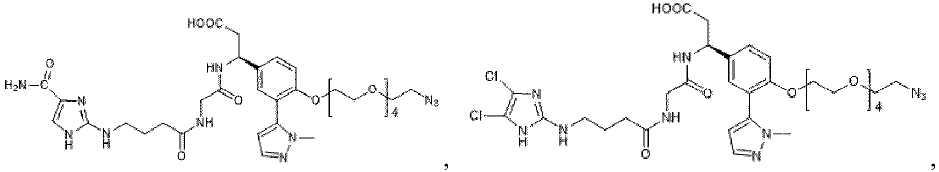 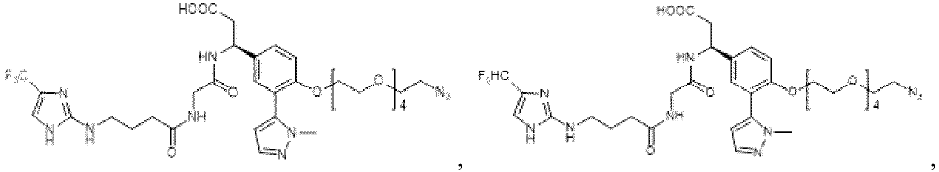 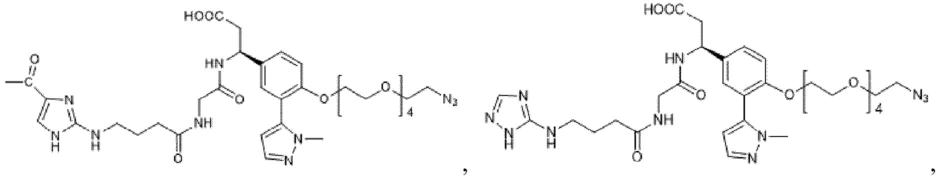 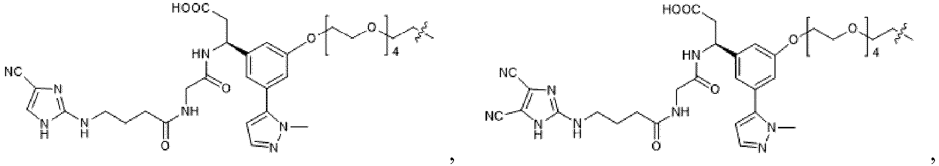 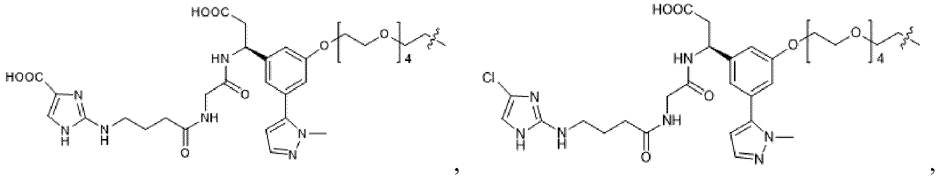 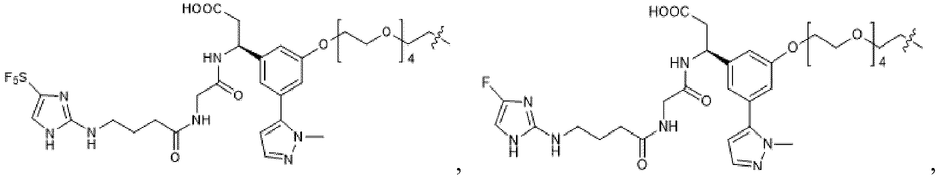 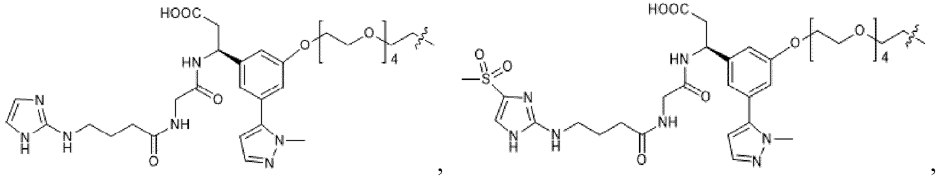 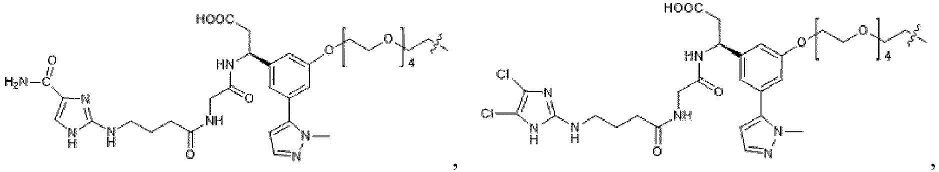 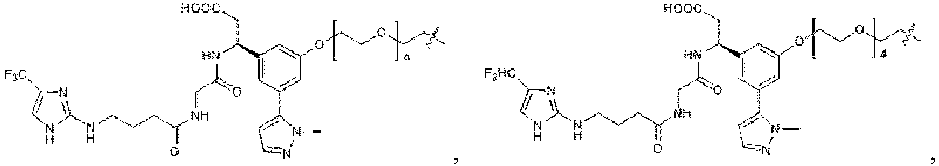 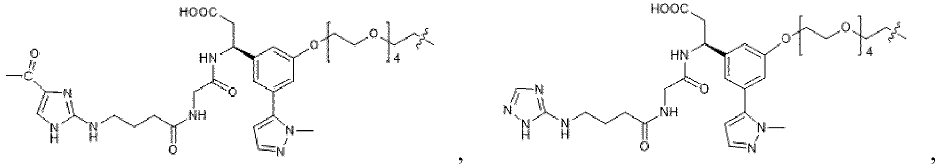 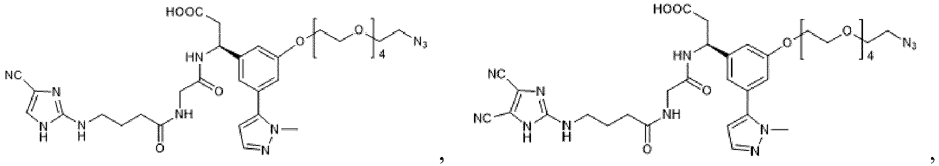 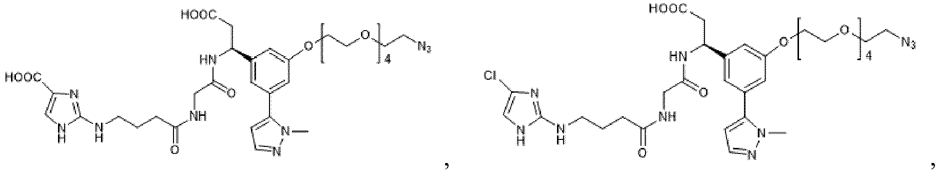 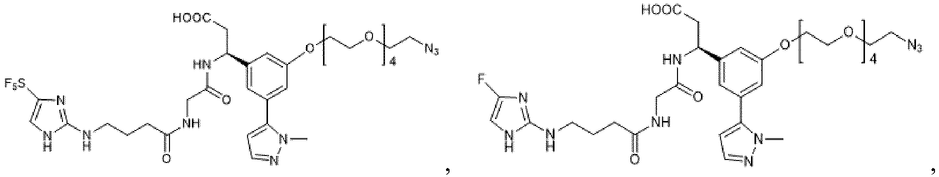 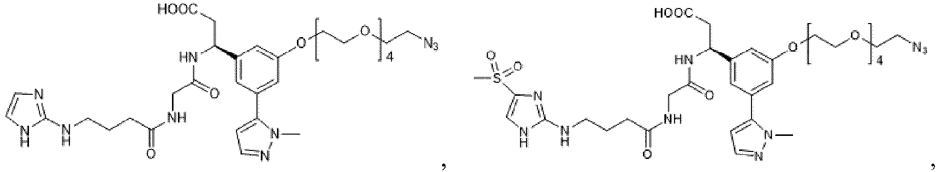 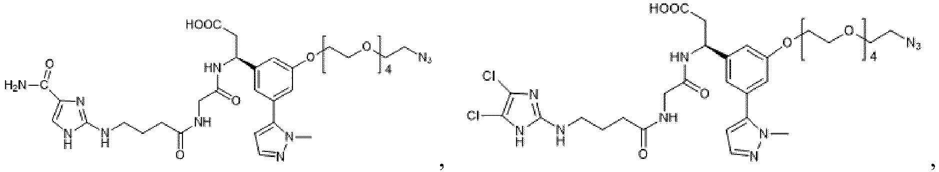 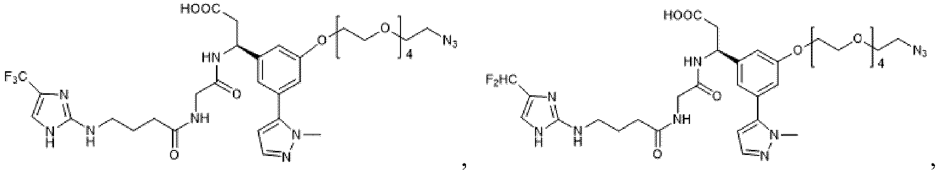 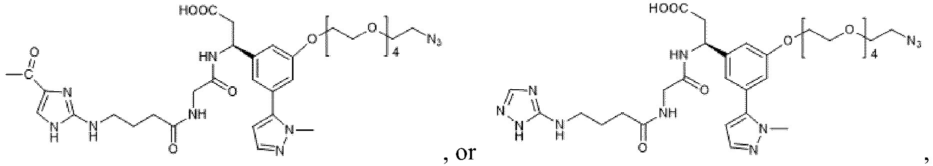 where 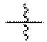 represents an attachment point.

11. A compound, comprising any one of the following structures: 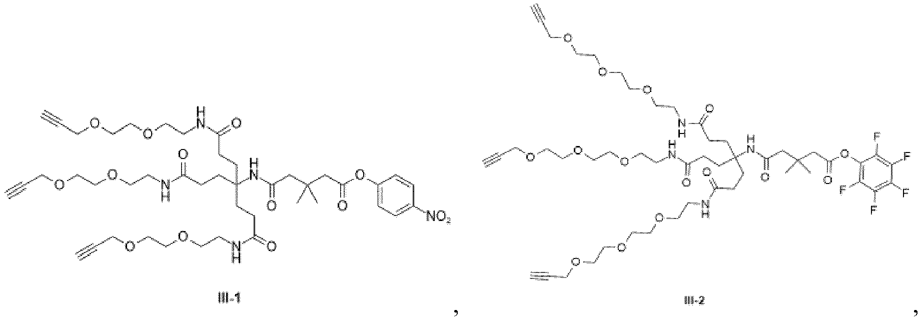 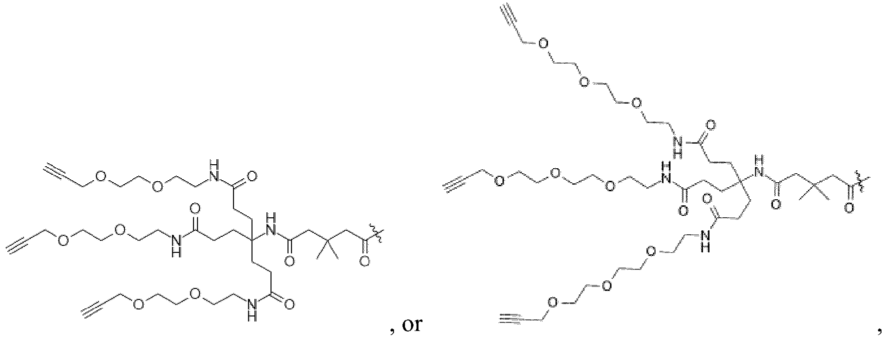 where 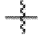 represents an attachment point.

12. A conjugate, comprising:
the compound represented by Formula I, or the tautomer or stereoisomer thereof according to any one of claims 1 to 10, wherein the compound represented by Formula I comprises a linker T; and
an active molecule G, wherein the active molecule G is linked to the linker T of the compound represented by Formula I.

13. The conjugate according to claim 12, further comprising a scaffold,
wherein the active molecule G is linked to the linker T of the compound represented by Formula I via the scaffold.

14. The conjugate according to claim 12 or 13, satisfying one or more of the following conditions:
aa) the active molecule G is an active pharmaceutical ingredient or a prodrug thereof;
bb) the active molecule G is a small molecule, an antibody, an antibody fragment, an immunoglobulin, a monoclonal antibody, a label or marker, a lipid, a natural or modified nucleic acid, a natural or modified oligonucleotide, a natural or modified polynucleotide, a peptide, a nucleic acid aptamer, a polymer, a polyamine, a protein, a toxin, a vitamin, polyethylene glycol, a hapten, digoxin, biotin, a radioactive atom or molecule, or a fluorophore;
cc) the active molecule G is a natural or modified oligonucleotide;
dd) the active molecule G is ASO, siRNA, or miRNA;
ee) the scaffold has a monodentate, bidentate, tridentate, or tetradentate structure;
ff) the active molecule G is linked to one, two, three, or four compounds represented by Formula I via the scaffold; or
gg) the scaffold is the compound according to claim 11.

15. The conjugate according to any one of claims 12 to 14, wherein the conjugate comprises the following structures: 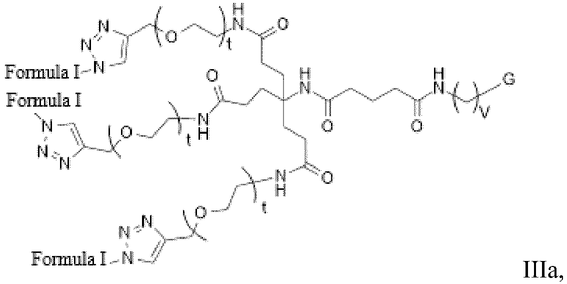 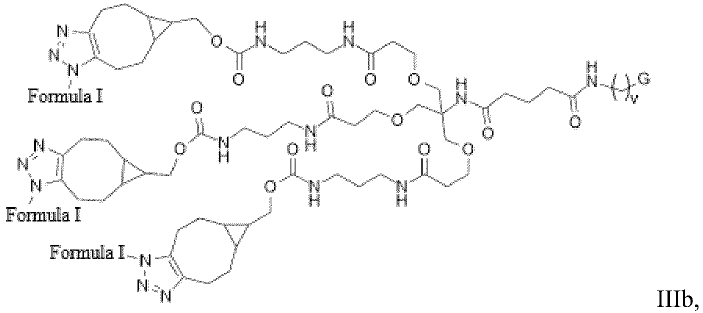 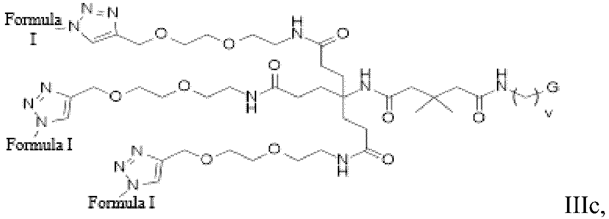 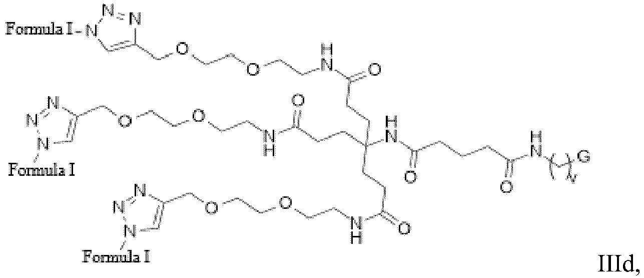 or 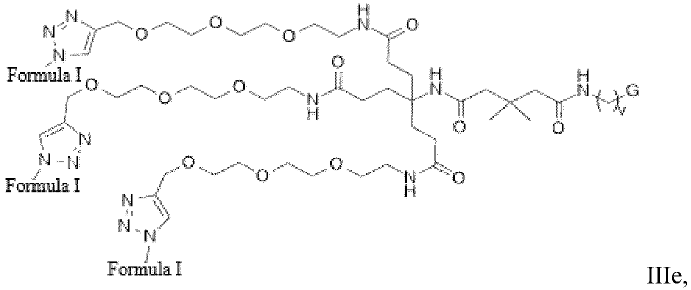 where:
Formula I is the compound represented by Formula I, or the tautomer or stereoisomer thereof,
G is the active molecule G; and
v ranges from 1 to 10, and preferably, v is 4, 5, 6, 7, or 8;
preferably, the active molecule G is a natural or modified oligonucleotide; and
preferably, the active molecule G is ASO, siRNA, or miRNA.

16. The conjugate according to claim 12, wherein the conjugate comprises the following structures: 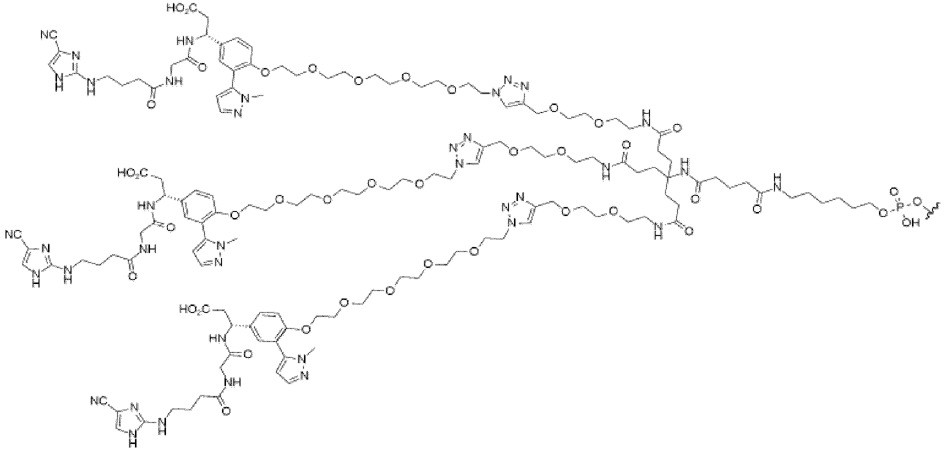 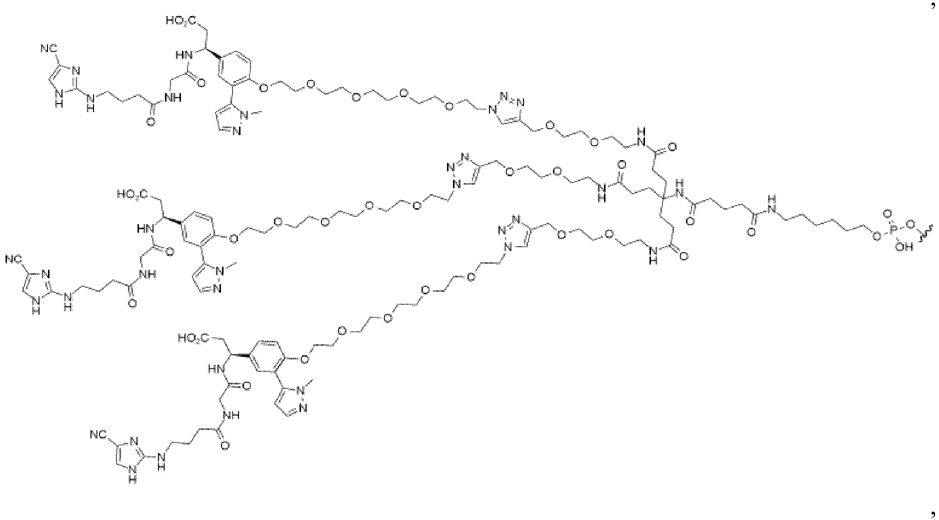 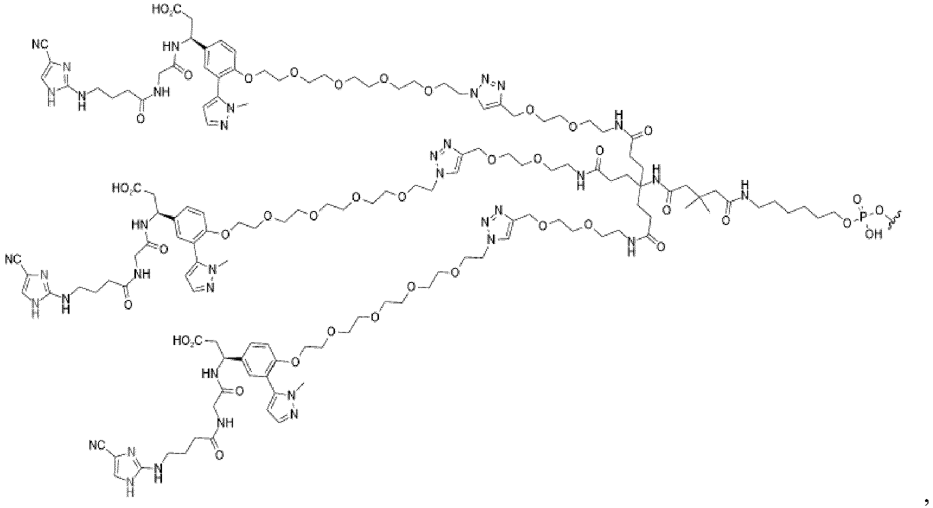 or 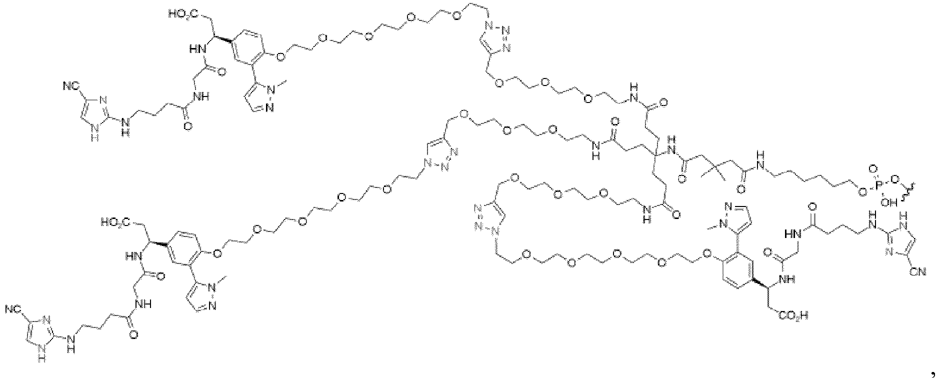 where 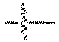 represents an attachment point to an oligonucleotide.

17. A composition, comprising:
the compound represented by Formula I, or the tautomer or stereoisomer thereof according to any one of claims 1 to 10, or the conjugate according to any one of claims 12 to 16; and
optionally, a pharmaceutically acceptable excipient.

18. Use of the compound represented by Formula I, or the tautomer or stereoisomer thereof according to any one of claims 1 to 10, the conjugate according to any one of claims 12 to 16, or the composition according to claim 17, in:
the delivery of an active molecule G to a cell; and/or
the inhibition of expression of a target gene in a cell; and/or
the treatment of a pulmonary disease; and/or
the manufacture of a medicament for inhibiting expression of a target gene in a cell; and/or
the manufacture of a medicament for treating a pulmonary disease.

19. The compound represented by Formula I, or the tautomer or stereoisomer thereof according to any one of claims 1 to 10, the conjugate according to any one of claims 12 to 16, or the composition according to claim 17, for use in:
delivering an active molecule G to a cell; and/or
inhibiting expression of a target gene in a cell; and/or
treating a pulmonary disease.

20. The use according to claim 18 or 19, wherein:
the cell is a cell expressing integrin αvβ6 or an epithelial cell; and/or
the cell is a type I or type II alveolar epithelial cell, a goblet cell, a secretory epithelial cell, a ciliated epithelial cell, a corneal or conjunctival epithelial cell, a dermal epithelial cell, a bile duct epithelial cell, an intestinal epithelial cell, a ductal epithelial cell, a glandular epithelial cell, or an epithelial tumor (carcinoma).

21. A method for delivering an active molecule to a cell, inhibiting expression of a target gene in a cell, or treating a pulmonary disease, the method comprising:
administering to a patient the compound represented by Formula I, or the tautomer or stereoisomer thereof according to any one of claims 1 to 10, the conjugate according to any one of claims 12 to 16, or the composition of claim 17.
